(19) Europäisches Patentamt
European Patent Office
Office européen des brevets

(11) Publication number : **0 462 800 A2**

# (12) EUROPEAN PATENT APPLICATION

(21) Application number : **91305502.6**

(22) Date of filing : **18.06.91**

(51) Int. Cl.$^5$ : **C07D 213/64,** C07D 213/76, C07D 401/06, C07D 401/12, C07D 407/06, C07D 407/12, C07D 409/06, C07D 409/12, C07D 413/06, C07D 413/12, C07D 417/06, // C07D417/12, A61K31/44

(30) Priority : **18.06.90 US 539643**
**25.01.91 US 646131**
**18.06.90 US 539681**
**07.06.91 US 712217**

(43) Date of publication of application :
**27.12.91 Bulletin 91/52**

(84) Designated Contracting States :
**AT BE CH DE DK ES FR GB GR IT LI LU NL SE**

(71) Applicant : **MERCK & CO. INC.**
**126, East Lincoln Avenue P.O. Box 2000**
**Rahway New Jersey 07065-0900 (US)**

(72) Inventor : **Hoffman, Jacob M., Jr.**
**Pennbrooke Gardens, Apt. 17A**
**North Wales, PA 19454 (US)**
Inventor : **Rooney, Clarence S.**
**2902 Hickory Hill Drive**
**Worcester, PA 19490 (US)**
Inventor : **Saari, Walfred S.**
**1740 Wagon Wheel Lane**
**Lansdale, PA 19446 (US)**
Inventor : **Wai, John S.**
**208 Tanglewood Way**
**Harleysville, PA 19438 (US)**
Inventor : **Williams, Theresa M.**
**260 Shadynook Road**
**Harleysville, PA 19438 (US)**
Inventor : **Bamberger, Dona L.**
**19B Brookside Drive**
**Lansdale, PA 19446 (US)**
Inventor : **Jones, James H.**
**6036 Cannon Hill Road**
**Fort Washington, PA 19034 (US)**

(74) Representative : **Barrett-Major, Julie Diane et al**
**Merck & Co., Inc. European Patent Department Terlings Park Eastwick Road**
**Harlow Essex CM20 2QR (GB)**

## (54) Inhibitors of HIV reverse transcriptase.

(57) Novel pyridones inhibit HIV reverse transcriptase, and are useful in the prevention or treatment of infection by HIV and the treatment of AIDS, either as compounds, pharmaceutically acceptable salts, pharmaceutical composition ingredients, whether or not in combination with other antivirals, anti-infectives, immunomodulators, antibiotics or vaccines. Methods of treating AIDS and methods of preventing or treating infection by IV are also described.

EP 0 462 800 A2

The present invention is concerned with compounds which inhibit the reverse transcriptase encoded by human immunodeficiency virus (HIV) or pharmaceutically acceptable salts thereof and are of value in the prevention of infection by HIV, the treatment of infection by HIV and the treatment of the resulting acquired immune deficiency syndrome (AIDS). It also relates to pharmaceutical compositions containing the compounds.

## BACKGROUND OF THE INVENTION

A retrovirus designated human immunodeficiency virus (HIV) is the etiological agent of the complex disease that includes progressive destruction of the immune system (acquired immune deficiency syndrome; AIDS) and degeneration of the central and peripheral nervous system. This virus was previously known as LAV, HTLV-III, or ARV. A common feature of retrovirus replication is reverse transcription of the RNA genome by a virally encoded reverse transcriptase to generate DNA copies of HIV sequences, a required step in viral replication. It is known that some compounds are reverse transcriptase inhibitors and are effective agents in the treatment of AIDS and similar diseases, e.g., azidothymidine or AZT.

Nucleotide sequencing of HIV shows the presence of a pol gene in one open reading frame [Ratner, L. et al., Nature, 313, 277(1985)]. Amino acid sequence homology provides evidence that the pol, sequence encodes reverse transcriptase, an endonuclease and an HIV protease [Toh, H. et al., EMBO J. 4, 1267 (1985); Power, M.D. et al., Science, 231, 1567 (1986); Pearl, L.H. et al., Nature 329, 351 (1987)].

Applicants demonstrate that the compounds of this invention are inhibitors of HIV reverse transcriptase. The inhibition is very specific, since there is little or no inhibition of the reverse transcriptases of AMV, MMLV or SIV. Further, the compounds of the present invention do not require bio=activation to be effective. They are also generally more potent than AZT.

## BRIEF DESCRIPTION OF THE INVENTION

Compounds of formula I, as herein defined, are disclosed. These compounds are useful in the inhibition of HIV reverse transcriptase, the prevention of infection by HIV, the treatment of infection by HIV and in the treatment of AIDS and/or ARC, either as compounds, pharmaceutically acceptable salts (when appropriate), pharmaceutical composition ingredients, whether or not in combination with other antivirals, anti-infectives, immunomodulators, antibiotics or vaccines. Methods of treating AIDS, methods of preventing infection by HIV, and methods of treating infection by HIV are also disclosed.

## DETAILED DESCRIPTION OF THE INVENTION AND PREFERRED EMBODIMENTS

This invention is concerned with the use of compounds of formula I, combinations thereof, or pharmaceutically acceptable salts thereof, in the inhibition of HIV reverse transcriptase, the prevention or treatment of infection by HIV and in the treatment of the resulting acquired immune deficiency syndrome (AIDS). Compounds of formula I are defined as follows:

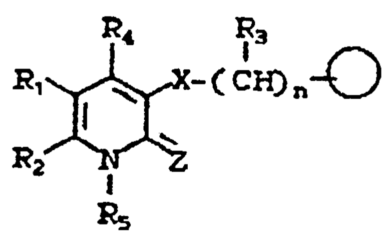

I

wherein

X is

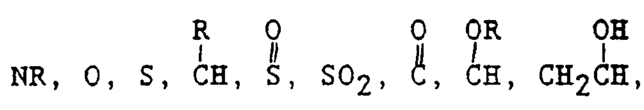

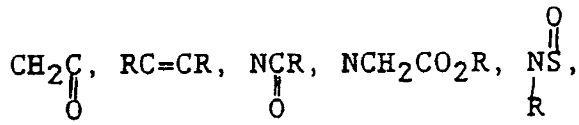

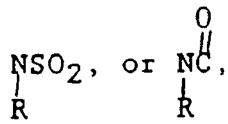

where R is H, $C_{1-8}$ alkyl, $C_{1-8}$ alkenyl or $C_{3-8}$ cycloalkyl;

Z is 0, S or $NR_x$ when $R_x$ is H or $C_{1-8}$ alkyl n is 0-4;

$R_1$, $R_2$ and $R_4$ are the same or different and are independently

(i) H;

(ii) $C_{1-8}$ alkyl, $C_{1-8}$ alkenyl, $C_{3-8}$ cycloalkyl, any of which is unsubstituted or substituted with one or two of $C_{1-3}$ alkoxy, $C_{1-4}$ alkylamino, di($C_{1-4}$ alkyl)amino, $C_{1-3}$ alkylthio, hydroxy, amino, carbonyl, aminocarbonyl, or oximido, or one to five of halo;

(iii) $C_{1-5}$ alkylthio;

(iv) $C_{1-5}$ alkylsulfinyl;

(v) $C_{1-5}$ alkylsulfonyl;

(vi) $C_{1-5}$ alkoxy;

(vii) $C_{1-5}$ alkoxycarbonyl;

(viii) cyano;

(ix) halo; or

(x) aryl;

or $R_1$ and $R_4$ may together form a cycloalkyl ring containing 5-7 members;

or $R_1$ and $R_2$ may together form a cycloalkyl ring containing 5-7 members;

and $R_3$ or $R_5$ are the same or different and are independently

(i) H;

(ii) $C_{1-8}$ alkyl;

(iii) $C_{1-8}$ alkenyl;

(iv) $C_{3-8}$ cycloalkyl;

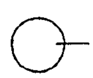

is aryl or heterocycle each unsubstituted or substituted with one or more of

(i) $C_{1-6}$ alkyl unsubstituted or substituted with one or more of A, wherein A is halo, hydroxy, hydroxy-$C_{1-4}$ alkyl, amino, $C_{1-6}$ alkylamino, di($C_{1-6}$ alkyl)amino $C_{1-6}$ alkoxy, or aryl;

(ii) $C_{1-6}$ alkenyl unsubstituted or substituted with one or more of A;

(iii) $C_{3-6}$ cycloalkyl unsubstituted or substituted with one or more of A;

(iv) $C_{1-6}$ alkoxy unsubstituted or substituted with one or more of A;

(v) aryl;

(vi) amino;

(vii) $C_{1-6}$ alkylamino;

(viii) di($C_{1-6}$ alkyl)amino;

(ix) amino-$C_{1-8}$ alkyl;

(x) $C_{1-8}$ alkyl-amino-$C_{1-8}$ alkyl;

(xi) di-($C_{1-6}$ alkyl)amino $C_{1-8}$ alkyl;

(xii) $C_{1-6}$ alkoxycarbonyl;

(xiii) aminocarbonyl;

(xiv) $C_{1-6}$ alkyl aminocarbonyl;

(xv) di($C_{1-6}$ alkyl)aminocarbonyl;
(xvi) $C_{1-6}$ alkylthio;
(xvii) $C_{1-6}$ alkylsulfinyl;
(xviii) $C_{1-6}$ alkylsulfonyl;
(xix) hydroxy;
(xx) halo;
(xxi) CN, or
(xxii) $NO_2$,

with the provisos that

I. $R_1$ or $R_2$ or both are not substituted with OH; and
II. heterocycle is not phthalimide;

or pharmaceutically acceptable salt hydrate or ester thereof.

A first embodiment is a compound of the formula:

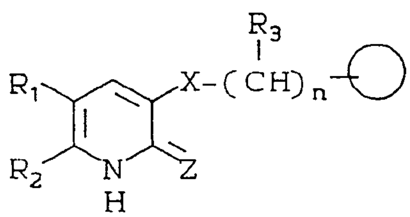

II

wherein

X is NH, 0, S, or $CH_2$;
Z is 0 or S;
n is 1-4;
$R_1$ is

(i) $C_{1-8}$ alkyl, unsubstituted or substituted with one or two of $C_{1-3}$alkoxy, halo, $C_{1-4}$alkylamino, amino, $C_{1-4}$-di-(alkyl)amino, or $C_{1-3}$ alkylthio;
(ii) $C_{1-3}$ alkylthio;
(iii) $C_{1-3}$ alkoxy; or
(iv) halo;

$R_2$ is H, methyl or ethyl, unsubstituted or substituted with one or two of methoxy, amino, methylamino, dimethylamino or methylthio;
$R_3$ is H or $C_{1-8}$ alkyl;

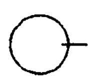

is aryl or heterocycle, each unsubstituted or substituted with one or more of

(a) $C_{1-6}$ alkyl,
(b) $C_{1-6}$ alkoxy unsubstituted or substituted with hydroxy-$C_{1-4}$ alkyl,
(c) amino,
(d) $C_{1-6}$ alkyl amino,
(e) di($C_{1-8}$ alkyl)amino,
(f) amino- $C_{1-8}$ alkyl,
(g) $C_{1-8}$ alkyl-amino-$C_{1-8}$ alkyl,
(h) di-($C_{1-6}$ alkyl)amino $C_{1-8}$ alkyl,
(i) hydroxyl,
(j) halogen,
(k) CN, or
(l) $NO_2$.

with the proviso that

I. $R_1$ or $R_2$ or both are not substituted with OH; and
II. heterocycle is not phthalimide.

A second embodiment is further limited to compounds wherein

$R_1$ is $C_{1-4}$ alkyl;

$R_2$ is H or $CH_3$;

aryl is

(a) naphthyl, unsubstituted or substituted with one or more of $C_{1-6}$ alkyl, halogen or hydroxyl;

(b) phenyl, unsubstituted or substituted with one or more of $C_{1-6}$ alkyl, $C_{1-6}$ alkoxy, halogen or hydroxyl; or

(c) biphenyl

A third embodiment is compounds of Formula II, further limited to aryl is

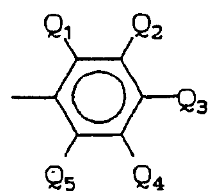

wherein

$Q_1$ is $C_{1-2}$alkoxy;

$Q_2$, $Q_3$, $Q_4$ and $Q_5$ are independently selected from H, $C_{1-3}$alkyl, $C_{1-2}$alkoxy, halogen, CN or $NO_2$.

A fourth embodiment is compounds of the second embodiment, further limited to aryl as
2-naphthyl, 2-naphthyl substituted with methyl,
2-naphthyl substituted with chloro; phenyl;
2-hydroxyphenyl, 2-hydroxyphenyl substituted with methyl, 2-hydroxyphenyl substituted with chloro,
2-methoxy-5-ethylphenyl, 2-methoxy-4,5-dimethylphenyl,
or 2,6-dimethoxyphenyl.

A fifth embodiment is compounds further limited to these wherein aryl is 2-naphthyl, phenyl or 2-hydroxyphenyl.

A sixth embodiment is compounds of the fourth embodiment, further limited to aryl as
2-methoxy-5-ethylphenyl, 2-methoxy-4,5-dimethyl-phenyl,
or 2,6-dimethoxyphenyl.

A seventh embodiment is compounds of Formula I or II, wherein heterocycle is thienyl, oxazolyl, thiazolyl, triazolyl, pyridyl unsubstituted or substituted with one or more of $C_{1-2}$alkoxy or $C_{1-3}$alkyl or hydroxyalkyl, pyridinonyl, pyrazinyl, benzothienyl, quinolinyl, 5,6,7,8-tetra-hydroquinolinyl, isoquinolinyl, benzimidazolyl, benzofuranyl, benzothiazolyl, benzoxazolyl unsubstituted or substituted with one or more of $C_{1-6}$ alkyl or halogen or hydroxyl, 4,5,6,7-tetra-hydrobenzoxazolyl, naphth [3,2-d] oxazolyl, oxazolo [4,5-b] pyridyl, chromone, or benzopyrimidinone.

An eighth embodiment covers compounds further limited to heterocycle as 2-oxazolyl, 2-pyridyl, 3-pyridyl unsubstituted or substituted with one or more of $C_{1-2}$alkoxy or $C_{1-3}$alkyl, 2-benzothienyl, 2-quinolinyl, 2-benzimidazolyl, 2-benzofuranyl, 2-benzothiazolyl, 2-oxazolo [4,5-b] pyridyl, or 2-benzoxazolyl unsubstituted or substituted with one or more of methyl, halogen or hydroxyl.

A ninth embodiment covers compounds further limited to heterocycle as 2-methoxy-5,6-dimethyl-3-pyridyl, 2-methoxy-5-ethyl-6-methyl-3-pyridyl, 3-methoxy-5,6-dimethyl-2-pyridyl, 2-benzoxazolyl, 7-methyl-2-benzoxazolyl, 4,7-dimethyl-2-benzoxazolyl, 7-chloro-2-benzoxazolyl, 4,7-dichloro-2-benzoxazolyl, 4,7-difluoro-2-benzoxazolyl, 4-fluoro-2-benzoxazolyl, or 4-chloro-2-benzoxazolyl.

One group of preferred compounds of this invention include, but is not limited to the following list:

3-{[(4,7-dichlorobenzoxazol-2-yl)methyl]amino}-5-ethyl-6-methyl-2-(1H)-pyridinone,
3-{[(4,,7-dimethylbenzoxazol-2-yl)methyl]amino}-5-ethyl-6-methyl-2-(1H)-pyridinone,
3-{[(7-chlorobenzoxazol-2-yl)methyl]amino}-5-ethyl-6-methyl-2-(1H)-pyridinone,
3-{[(7-methylbenzoxazol-2-yl)methyl]amino}-5-ethyl-6-methyl-2-(1H)-pyridinone,
3-{[(4-fluorobenzoxazol-2-yl)methyl]amino}-5-ethyl-6-methyl-2-(1H)-pyridinone,
3-{[(7-fluorobenzoxazol-2-yl)methyl]amino}-5-ethyl-6-methyl-2-(1H)-pyridinone,
3-{[(benzoxazol-2-yl)methyl]amino}-5-ethyl-6-methyl-2-(1H)-pyridinone,
3-{[(4-chlorobenzoxazol-2-yl)methyl]amino}-S-ethyl-6-methyl-2-(1H)-pyridinone,
3-{[(4-fluoro-7-chlorobenzoxazol-2-yl)methyl]amino}-5-ethyl-6-methyl-2-(1HH)-pyridinone,
3-[2-(4,7-Dichlorobenzoxazol-2-yl)ethyl]-5-ethyl-6-methyl-2-(1H)-pyridinone,
3-[2-(4,7-Difluorobenzoxazol-2-yl)ethyl]-5-ethyl-6-methyl-2-(1H)-pyridinone,

3-[2-(benzoxazol-2-yl)ethyl]-5-ethyl-6-methyl-2-(1H)-pyridinthione,
3-[2-(benzoxazol-2-yl)ethyl]-5-ethyl-6-methyl-2-(1H)-pyridinone,
3-[2-(4,7-dimethylbenzoxazol-2-yl)ethyl]-5-ethyl-6-methyl-2-(1H)-pyridinone,
3-[2-(4-methylbenzoxazol-2-yl)ethyl]-5-ethyl-6-methyl-2-(1H)-pyridinone,
3-[2-(7-methylbenzoxazol-2-yl)ethyl]-5-ethyl-6-methyl-2-(1H)-pyridinone,
3-[N-(5-Ethyl-2-methoxy-6-methyl-3-pyridylmethyl)-amino]-5-ethyl-6-methyl-2(1H)-pyridinone,
3-[N-(5-(2-Hydroxyethyl)-2-methoxy-6-methyl-3-pyridylmethyl)amino]-5-ethyl-6-methyl-2(1H)-pyridinone,
3-[N-(5-(1-Hydroxyethyl)-2-methoxy-6-methyl-3-pyridylmethyl)amino]-5-ethyl-6-methyl-2(1H)-pyridinone,
3-[N-(5,6-Dimethyl-2-methoxy-3-pyridylmethyl)amino]-5-ethyl-6-methyl-2(1H)-pyridinone,
3-[N-(5-Ethyl-2-methoxybenzyl)amino]-5-ethyl-6-methyl-2(1H)-pyridinone,
3-[N-(2-Methoxy-4,5-dimethylbenzyl)amino]-5-ethyl-6-methyl-2(1H)-pyridinone,
3-[N-(2,6-dimethoxybenzyl)amino]-5-ethyl-6-methyl-2(1H)-pyridinone,
3-{(4,7-dichlorobenzoxazol-2-yl)methyl]amino}-5-methylthio-6-methyl-2(1H)-pyridinone,
3-{(4,7-dichlorobenzoxazol-2-yl)methyl]thio}-5-ethyl-6-methyl-2(1H)-pyridinone,
3-[N-(2-methoxy-5-methylbenzyl)amino]-5-ethyl-6-methyl-2(1H)-pyridinone,
3-[(5-ethyl-2-methoxy-6-methyl-3-pyridinylmethyl)-amino]-5-cyclopropyl-6-methyl-2-(1H)-pyridinone,
3-[N-(2-methoxy-4-methylbenzyl)amino]-5-ethyl-6-methyl-2(1H)-pyridinone,
3-{N-[(4,7-dichlorobenzoxazol-2-yl)methyl]-N-methyl-amino}-5-ethyl-6-methyl-2(1H)-pyridinone,
3-[2-(4,7-dichlorobenzoxazol-2-yl)ethyl]-5-propyl-6-methyl-2(1H)-pyridinone,
3-[2-(4,7-diclorobenzoxazol-2-yl)ethyl]-5-ethyl-6-methyl-pyridin-2(1H)-thione, or
3-[2-(7-fluorobenzoxazol-2-yl)ethyl]-5-ethyl-6-methylpyridin-2(1H)-one,

or pharmaceutically acceptable esters thereof.

Another group of preferred compounds includes, but is not limited to the following list
3-{[(4,7-dichlorobenzoxazol-2-yl)methyl]amino}-5-ethyl-6-methyl-2(1H)-pyridinone,
3-{[(4,7-dimethylbenzoxazol-2-yl)methyl]amino}-5-ethyl-6-methyl-2(1H)-pyridinone,
3-{[(7-clorobenzoxazol-2-yl)methyl]amino}-5-ethyl-6-methyl-2(1H)-pyridinone,
3-{[(7-methylbenzoxazol-2-yl)methyl]amino}-5-ethyl-6-methyl-2(1H)-pyridinone,
3-[2-(4,7-dichlorobenzoxazol-2-yl)ethyl]-5-ethyl-6-methyl-2(1H)-pyridinone,
3-[2-(4,7-difluorobenzoxazol-2-yl)ethyl]-5-ethyl-6-methyl-2(1H)-pyridinone,
3-[2-(4-fluorobenzoxazol-2-yl)ethyl]-5-ethyl-6-methyl-2(1H)-pyridinone,
3-[2-(4-chlorobenzoxazol-2-yl)ethyl]-5-ethyl-6-methyl-2(1H)-pyridinone,
3-[2-benzoxazol-2-yl)ethyl]-5-ethyl-6-methyl-2(1H)-pyridinone,
3-[2-benzoxazol-2-yl)ethyl]-5-ethyl-6-methyl-2(1H)-pyridinthione,
3-[N-(5-Ethyl-2-methoxy-6-methyl-3-pyridylmethyl)-amino]-5-ethyl-6-methyl-2(1H)-pyridinone,
3-[N-(5,6-Dimethyl-2-methoxy-3-pyridylmethyl)-amino]-5-ethyl-6-methyl-2(1H)-pyridinone,
3-[N-(5-Ethyl-2-methoxybenzyl)amino]-5-ethyl-6-methyl-2(1H)-pyridinone,
3-[N-(2-Methoxy-4,5-dimethylbenzyl)amino]-5-ethyl-6-methyl-2(1H)-pyridinone, or
3-[N-(2,6-Dimethoxybenzyl)amino]-5-ethyl-6-methyl-2(1H)-pyridinone,

or pharmaceutically acceptable esters thereof.

A group of most preferred compounds includes, but is not limited to the following
3-[N-(5-Ethyl-2-methoxybenzyl)amino]-5-ethyl-6-methyl-2(1H)-pyridinone,
3-[N-(2-Methoxy-4,5-dimethylbenzyl)amino]-5-ethyl-6-methyl-2(1H)-pyridinone,
3-[N-(5,6-Dimethyl-2-methoxy-3-pyridylmethyl)-amino]-5-ethyl-6-methyl-2(1H)-pyridinone,
3-[2-(4,7-Dichlorobenzoxazol-2-yl)ethyl]-5-ethyl-6-methyl-2(18)-pyridinone,
3-{(4,7-dichlorobenzoxazol-2-yl)methyl]thio}-5-ethyl-6-methyl-2(1H)-pyridinone,
3-[2-(benzoxazol-2-yl)ethyl]-5-ethyl-6-methyl-2(1H)-pyridinthione,
3-[2-(4,7-Difluorobenzoxazol-2-yl)ethyl]-5-ethyl-6-methyl-2(1H)-pyridinone,
3-[N-(5-Ethyl-2-methoxy-6-methyl-3-pyridylmethyl)-amino]-5-ethyl-6-methyl-2(1H)-pyridinone,
3-[2-(4,7-dichlorobenzoxazol-2-yl)ethyl]-5-ethyl-6-methyl-pyridin-2(1H)-thione,
3-[2-(4-methylbenzoxazo1-2-yl)ethyl]-5-ethyl-6-methyl-2(1H)-pyridinone,
3-{[(4,7-dimethylbenzoxazol-2-yl)methyl]amino}-5-ethyl-6-methyl-2(1H)-pyridinone,
3-{[(4,7-dichlorobenzoxazol-2-yl)methyl]amino}-5-ethyl-6-methyl-2(1H)-pyridinone,
3-[2-(7-fluorobenzoxazol-2-yl)ethyl]-5-ethyl-6-methylpyridin-2(1H)-one,
3-[2-(benzoxazol-2-yl)ethyl]-5-ethyl-6-methyl-2(1H)-pyridinone,
3-[N-(2-methoxy-5-methylbenzyl)amino]-5-ethyl-6-methyl-2-(1H)-pyridinone,
3-[2-(4,7-dimethylbenzoxazol-2-yl)ethyl]-5-ethyl-6-methyl-2-(1H)-pyridinone,
3-[(5-ethyl-2-methoxy-6-methyl-3-pyridinylmethyl)-amino]-5-cyclopropyl-6-methyl-2-(1H)-pyridinone,
3-{(4,7-dichlorobenzoxazol-2-yl)methyl]amino}-5-methylthio-6-methyl-2-(1H)-pyridinone,

3-[N-(2-methoxy-4-methylbenzyl)amino]-5-ethyl-6-methyl-2-((1H)-pyridinone,
3-[N-(2,6-dimethoxybenzyl)amino]-5-ethyl-6-methyl-2-(1H)-pyridinone,
3-{N-[(4,7-dichlorobenzoxazol-2-yl)methyl]-N-methylamino}-5-ethyl-6-methyl-2-(1H)-pyridinone,
or,
3-[2-(4,7-dichlorobenzoxazol-2-yl)ethyl]-5-propyl-6-methyl-2-(1H)-pyridinone,
or pharmaceutically acceptable esters thereof.

Compounds with a transposed linker are also covered by this invention. Such compounds include those of the formula:

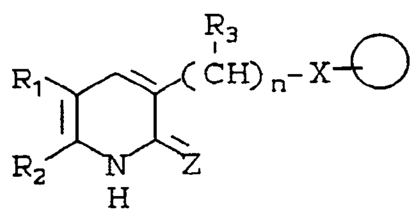

V

wherein
X is NH, O or S;
Z is O or S;
n is 1-4;
$R_1$ is
(i) $C_{1-8}$alkyl, unsubstituted or substituted with one or two of $C_{1-3}$alkoxy, halo, $C_{1-4}$alkylamino, amino, $C_{1-4}$-di-alkylamino, or $C_{1-3}$alkylthio,
(ii) $C_{1-3}$alkylthio
(iii) $C_{1-3}$alkoxy; or
(iv) halo;
$R_2$ is H, methyl or ethyl, unsubstituted or substituted with one or two of methoxy,amino, methylamino, dimethylamino or methylthio
$R_3$ is H or $C_{1-8}$ alkyl;

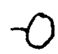

is aryl or heterocycle, each unsubstituted or substituted with one or more of
(a) $C_{1-6}$ alkyl,
(b) $C_{1-6}$ alkoxy unsubstituted or substituted with hydroxy-$C_{1-4}$ alkyl,
(c) amino,
(d) $C_{1-6}$ alkyl amino,
(e) di($C_{1-6}$ alkyl)amino,
(f) amino- $C_{1-8}$ alkyl,
(g) $C_{1-8}$ alkyl-amino-$C_{1-8}$ alkyl,
(h) di-($C_{1-6}$ alkyl)amino $C_{1-8}$ alkyl,
(i) hydroxyl, or
(j) halogen,
with the proviso that heterocycle is not phthalimide.

One embodiment of the present invention is Formula V compounds limited as follows:
$R_1$ is $C_{1-4}$ alkyl;
$R_2$ is H or $CH_3$;
aryl is
(a) naphthyl, unsubstituted or substituted with one or more of $C_{1-6}$ alkyl, halogen or hydroxyl;
(b) phenyl, unsubstituted or substituted with one or more of $C_{1-6}$ alkyl, halogen or hydroxyl; or
(c) biphenyl

A second embodiment are compounds further limited, wherein aryl is 2-naphthyl, 2-naphthyl substituted with methyl, 2-naphthyl substituted with chloro; phenyl; 2-hydroxyphenyl, 2-hydroxyphenyl substituted with methyl, or 2-hydroxyphenyl substituted with chloro.

A third embodiment encompasses compounds further limited, wherein aryl is 2-naphthyl, phenyl or 2-hydroxyphenyl.

A fourth embodiment encompasses compounds of Formula V wherein Heterocycle is thienyl, oxazolyl, thiazolyl, triazolyl, pyridyl, pyrazinyl, benzothienyl, quinolinyl, isoquinolinyl, benzimidazolyl, benzofuranyl, benzothiazolyl, benzoxazolyl unsubstituted or substituted with one or more of $C_{1-6}$ alkyl or halogen or hydroxyl, naphth [3,2-d] oxazolyl, oxazolo [4,5-b] pyridyl, chromone, or benzopyrimidinone.

A fifth embodiment covers compounds having heterocycle further limited to 2-oxazolyl, 2-pyridyl, 2-benzothienyl, 2-quinolinyl, 2-benzimidazolyl, 2-benzofuranyl, 2-benzothiazolyl, 2-benzoxazolyl, methyl substituted 2-benzoxazolyl, halo substituted 2-benzoxazolyl, hydroxyl substituted 2-benzoxazolyl, or 2-oxazolo [4,5-b] pyridyl.

The compounds of the present invention, may have asymmetric centers and occur as racemates, racemic mixtures and as individual diastereomers, with all isomeric forms being included in the present invention. ,

When any variable (e.g., aryl, heterocycle, $R_1$, $R_2$, $R_3$, etc.) occurs more than one time in any constituent or in formula I, its definition on each occurrence is independent of its definition at every other occurrence. Also, combinations of substituents and/or variables are permissible only if such combinations result in stable compounds.

As used herein except where noted, "alkyl" is intended to include both branched- and straight-chain saturated aliphatic hydrocarbon groups having the specified number of carbon atoms; "alkoxy" represents an alkyl group of indicated number of carbon atoms attached through an oxygen bridge. "Halogen" or "halo" as used herein, means fluoro, chloro, bromo and iodo. "Alkenyl" is intended to include hydrocarbon chains of either a straight or branched configuration and one or more unsaturated carbon-carbon bonds which may occur in any stable point along the chain, such as ethenyl, propenyl, butenyl, pentenyl and the like.

As used herein, with exceptions as noted, "aryl" is intended to mean any stable monocyclic, bicyclic or tricyclic carbon ring of up to 7 members in each ring, wherein at least one ring is aromatic. Examples of such aryl elements include phenyl, naphthyl, tetrahydronaphthyl, biphenyl, phenanthryl, anthryl or acenaphthyl.

The term heterocycle or heterocyclic, as used herein except where noted, represents a stable 5- to 7-membered monocyclic or stable 8- to 11-membered bicyclic or stable 11-15-membered tricyclic heterocyclic ring which is either saturated or unsaturated, and which consists of carbon atoms and from one to four heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. The heterocyclic ring may be attached at any heteroatom or carbon atom which results in the creation of a stable structure. Examples of such heterocyclicelements include piperidinyl, piperazinyl, 2-oxopiperazinyl, 2-oxopiperidinyl, 2-oxopynolidinyl, 2-oxoazepinyl, azepinyl, pynolyl, 4-piperidonyl, pynolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazoyl, benzopyranyl, benzothiazolyl, benzoxazolyl, furyl, tetrahydrofuryl, benzofuranyl, tetrahydropyranyl, thienyl, benzothienyl, thiamorpholinyl, thiamorpholinyl sulfoxide, thiamorpholinyl sulfone, and oxadiazolyl. Heterocycle does not include phthalimide. Pyridin-2(1H)-one is the same as 2(1H)-pyridinone.

In the compounds of formula I, $R_1$, $R_2$ and $R_3$ include but are not limited to the following substituents, listed in tabular form.

TABLE

| $R_1$ | $R_2$ | $R_3$ |
|---|---|---|
| $CH_3$ | H | H |
| $CH_2OCH_3$ | H | H |
| $CH_2NHCH_3$ | H | H |
| $CH_2CH_3$ | H | H |
| $CH_2CH_2Cl$ | H | H |
| $CH_2CH_2NHCH_3$ | H | H |
| $CH_3$ | $CH_3$ | H |
| $CH_2OCH_3$ | $CH_3$ | H |
| $CH_2NHCH_3$ | $CH_3$ | H |
| $CH_2CH_3$ | $CH_3$ | H |
| $CH_2CH_2Cl$ | $CH_3$ | H |
| $CH_2CH_2NHCH_3$ | $CH_3$ | H |
| $OCH_3$ | $CH_3$ | H |
| Cl | H | H |
| Cl | $CH_3$ | H |
| Cl | $CH_2CH_3$ | H |
| $CH_3$ | $CH_2CH_3$ | H |
| $CH_2OCH_3$ | $CH_2CH_3$ | H |
| $CH_2NHCH_3$ | $CH_2CH_3$ | H |
| $CH_2CH_3$ | $CH_2CH_3$ | H |
| $CH_2CH_2Cl$ | $CH_2CH_3$ | H |
| $CH_2CH_2NHCH_3$ | $CH_2CH_3$ | H |
| $CH_2CH_3$ | $CH_3$ | $CH_3$ |
| $CH_2CH_2Cl$ | $CH_3$ | $CH_2CH_3$ |
| $CH_2CH_2NHCH_3$ | $CH_3$ | $CH_2CH(CH_3)CH_3$ |
| $CH_2CH_3$ | Cl | $CH_3$ |

TABLE (CONT'D)

| $R_1$ | $R_2$ | $R_3$ |
|---|---|---|
| $CH(CH_3)CH_3$ | H | H |
| $CH(CH_3)CH_3$ | $CH_3$ | H |
| $CH(CH_3)CH_3$ | $CH_2CH_3$ | H |
| $CH(CH_3)CH_3$ | $CH_2CH_3$ | $CH_3$ |
| tert-butyl | H | H |
| tert-butyl | $CH_3$ | H |
| tert-butyl | $CH_2CH_3$ | H |
| tert-butyl | $CH_2CH_3$ | $CH_3$ |
| cyclopropyl | H | H |
| cyclopropyl | $CH_3$ | H |
| cyclopropyl | $CH_2CH_3$ | H |
| cyclopropyl | $CH_2CH_3$ | $CH_3$ |

In the synthesis of the amino pyridones (3) of this invention, the procedures and protocols of U.S. 3,721,676 can be followed for making many intermediates as well as some of the phthalimide products, which patent is incorporated by reference for these purposes. Applicants here provide preferred methods of synthesis, outlined below.

The 3-nitropyridone of the first step in the synthesis of 3 may be formed by a simultaneous condensation and cyclization of

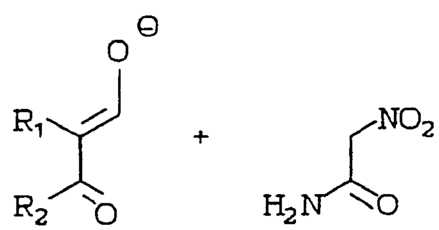

in the presence of a base such as piperidinium acetate. Nitroacetamide is prepared according to Brownstein, S.K., J. Org. Chem. 23, 113 (1958). The 3-nitropyridone products of the first step can also be prepared by direct nitration of 3-unsubstituted pyridones. The 3-nitropyridone product of the first step

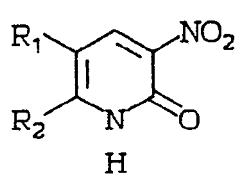

1

is reduced to

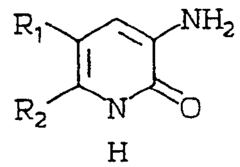

2

in a second step, preferably by catalytic reduction (when sulfur atoms are not present) in the presence of e.g. $H_2$ gas and Pd on carbon catalyst in a solvent such as ethanol. See, e.g., Okafor, C.O. et al., J. Heterocyclic Chem. 20, 199 (1983). Alternatively, the reduction of the second step (including when sulfur atoms are present) can be performed by chemical means, e.g. with NaSH, $Na_2S_2O_4$, Fe + $CaCl_2$, $H_2S$, or Sn + HCl. Reduction with iron in the presence of calcium chlorides is described in Okafor, C.O., J. Org. Chem. 47, 592 (1982). A third step, the final process giving rise to the compounds of this invention,

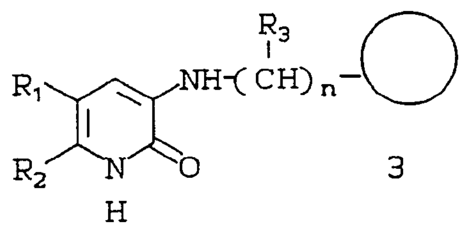

3

involves a coupling reaction, either by alkylation with an alkylhalide or a reductive alkylation with an aldehyde.

Carba derivatives (9), having alkylene bridges between the pyridone ring and the heterocyclic or aromatic entity attached at the 3-position, may be made by the following methods, the first being the preferred method.

In a first step 3-cyano-2-(1H) pyridinone is prepared by a simultaneous condensation and cyclization of

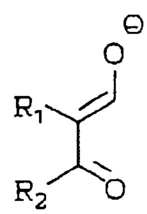

+

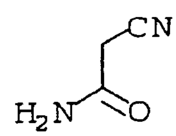

in the presence of base such as piperidinium acetate or pyrrolidine acetate. The resulting 3-cyano-2-(1H) pyridone,

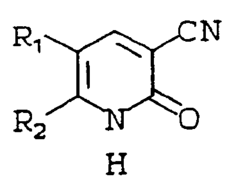

, 4

is heated in the presence of phosphorus pentachloride and/or $POCl_3$ to form the corresponding chloro pyridine,

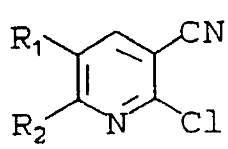

, 5

a conventional method of converting pyridones to chloropyridines. $PBr_3$ is useful for making the corresponding bromine derivative of product 5. Product 5 is then subjected to nucleophilic substitution to attach an alkoxy protecting group

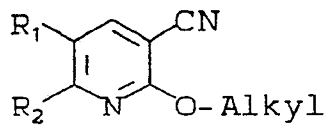

. 6

Reduction in the presence of, for example, diisobutyl aluminum hydride, yields

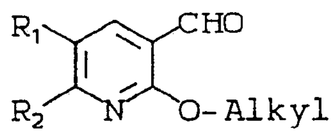

, 7

which is a conventional method of reducing nitriles to aldehydes. An alternative reducing agent for the synthesis of 7 is lithium trialkoxy aluminum hydride

Condensation in the presence of base at very low temperature (preferably at least -100°C) with an alkyl-substituted aryl or alkyl substituted heterocycle yields

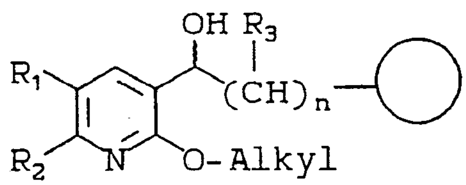

8

(n = 1).

Conditions will vary with the heterocycle to be attached. (The Wittig approach, infra, is preferable when n is more than 1.) Dehydration of the alcohol, and dealkylation followed by hydrogenation results in the compounds of the invention, wherein X = $CH_2$,

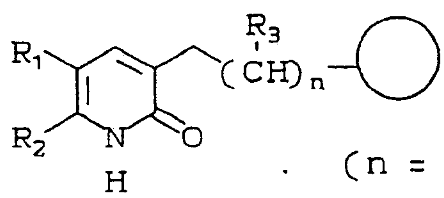

9

. (n = 1)

The dealkylation is typically carried out with pyridine hydrochloride in the presence of heat. The dehydration need not occur in the same reaction, but may be performed separately by conventional means. The standard catalyst for hydrogenation to produce product 9 is palladium, but other noble metals may substitute. Alternatively, the hydrogenation can be carried out with diimide or Raney nickel. $BCl_3$ or $BBr_3$ at low temperature is useful for performing dealkylation without dehydration. Dealkylation can also be carried out with KI in glacial acetic acid.

A second alternative variation on the final 3 steps is the Wittig approach, wherein the aldehyde derivative (product 7 above) is reacted with aryl or heterocycle, each substituted with alkylene triphenylphosphorane. The resulting condensation yields an unsaturated alkylene bridge, which is then hydrogenated to yield product 9 compounds above. The advantage of the Wittig approach is that it is preferable for synthesis of compounds of formula 9 wherein n is 2 or more, i.e. with longer alkylene bridges. It is useful when n=1. Also, the Wittig approach is preferable for compounds of Formula I having labile substituents on the heterocycle, particularly halogens.

A third, more lengthy method of making carba derivatives is illustrated in Example 4. Briefly, nitrile (4) is hydrolyzed with acid to the corresponding carboxylic acid. Then a three step conversion to an alcohol is performed, followed by oxidation to aldehyde 7. Condensation of the aryl/heterocycle ring is then carried out with

the resulting aldehyde as described above.

The transposed connecting chain amino derivatives (12) are prepared from product 6 above. Hydrogenation with, e.g., palladium on carbon, yields

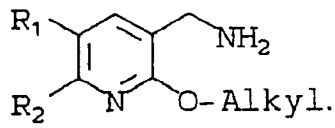

10

Other ways of hydrogenation of product 6 include reduction with $LiAlH_4$. Subsequent nucleophilic substitution with an appropriately activated halogenated aryl or an appropriately activated halogenated heterocycle produces the secondary amine,

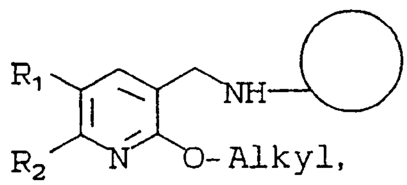

11

which is dealkylated with, for example, pyridine•HCl and heat, to produce the compounds of this invention

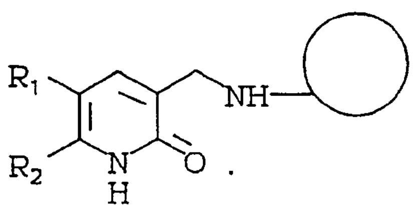

12

This is specifically illustrated by Example 10.

Alternatively, the transposed connecting chain amino derivatives (12) are prepared from product 7 above, by a reductive amination sequence with amino-substituted aryl or amino-substituted heterocycle. The initial acid-catalyzed condensation product is reduced with, e.g., $NaBH_4$ or $NaCNBH_3$, to yield

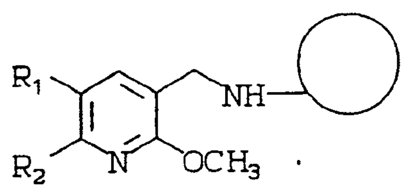

13

Dealkylation yields the pyridinone compounds

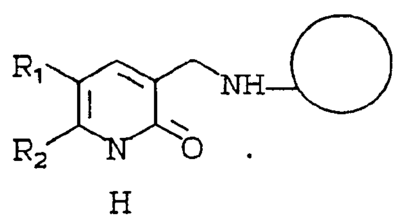

12

Typically, this final step is carried out with pyridine•HCl in the presence of heat. See also Example 12 for illustration.

2-Thio derivatives (15) are typically prepared by thiolating product 5 above with appropriate reagent such as t-butylmercaptan, to yield

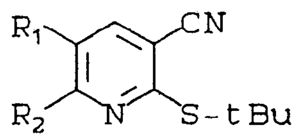

14

This compound now has a protecting group for the sulfur at the 2-position. The rest of the molecule can be constructed, and, as a final step, dealkylation in pyridine hydrochloride with heat results in the formation of:

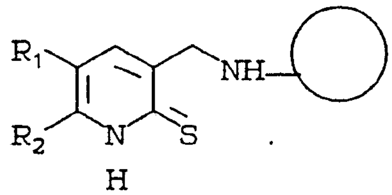

15

Example 11 illustrates this synthesis. Direct thiolation with Lawesson's Reagent is also useful for preparing 2-thio derivatives, as provided in specific detail in Example 13.

A procedure avails ether linkages at the 3-position of the pyridone ring (18), as provided by way of illustration in Example 9. Product 1 above is transformed in three steps into intermediate 16,

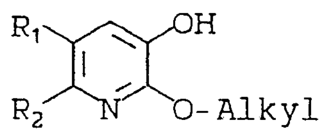

16

which is subsequently condensed with a halogenated aryl or halogenated heterocycle, in the presence of a base such as sodium hydride to yield

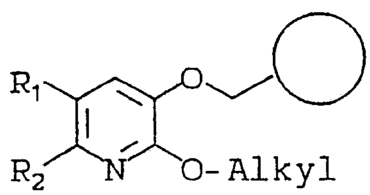

, 17

which is dealkylated with pyridine hydrochloride to yield

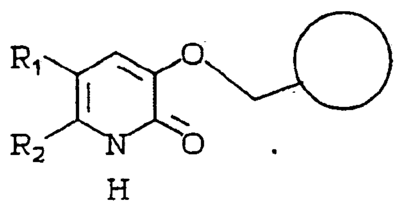

18

Modification by conventional methodology permits the preparation of the thio analog:

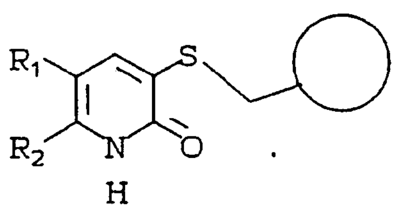

19

Dealkylation is conveniently achieved by heating with KI in glacial acetic acid.

Transposed ether linkages at the 3-position of the pyridones (21) in this invention can also be prepared by a simple two step procedure.

Intermediate 20

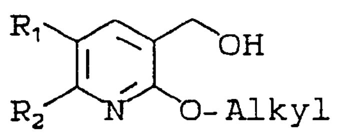

20

is reacted with a halogenated aryl or halogenated heterocycle and a base (NaH in DMF) to yield an alkylated product. Dealkylation is performed with e.g. pyridine•HCl to result in desired product 21:

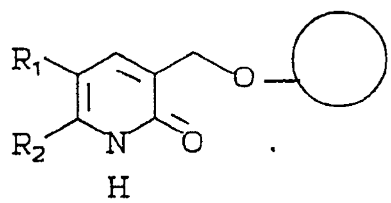

21

Phthalimide derivatives (22) are readily prepared in a one step synthesis. Intermediate 2 is refluxed with N-(hydroxymethyl)-phthalimide (which is commercially available) in ethanol. The resulting condensation product is

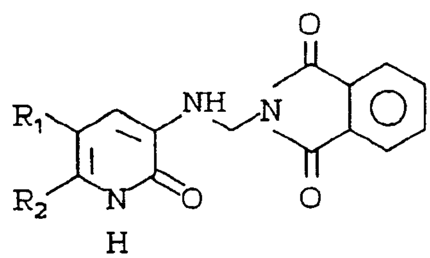

22

Carba derivatives, transposed linkages and 2-thiolated varieties are synthesized by conventional and known techniques analogous to those provided herein:

The compounds of the present inventions are useful in the inhibition of HIV reverse transcriptase, the prevention or treatment of infection by the human immunodeficiency virus (HIV) and the treatment of consequent pathological conditions such as AIDS. Treating AIDS or preventing or treating infection by HIV is defined as including, but not limited to, treating a wide range of states of HIV infection: AIDS, ARC (AIDS related complex), both symptomatic and asymptomatic, and actual or potential exposure to HIV. For example, the compounds of this invention are useful in treating infection by HIV after suspected past exposure to HIV by e.g., blood transfusion, organ transplant, exchange of body fluids, bites, accidental needle stick, or exposure to patient blood during surgery.

For these purposes, the compounds of the present invention may be administered orally, parenterally (including subcutaneous injections, intravenous, intramuscular, intrasternal injection or infusion techniques), by inhalation spray, or rectally, in dosage unit formulations containing . conventional non-toxic pharmaceutically-acceptable carriers, adjuvants and vehicles.

Thus, in accordance with the present invention there is further provided a method of treating and a pharmaceutical composition for treating HIV infection and AIDS. The treatment involves administering to a patient in need of such treatment a pharmaceutical composition comprising a pharmaceutical canier and a therapeutically-effective amount of a compound of the present invention.

These pharmaceutical compositions may be in the form of orally-administrable suspensions or tablets; nasal sprays; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweeteners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-initating excipient, such as cocoa butter, synthetic glyceride esters or polyethylene glycols, which are solid at ordinary temperatures, but liquidify and/or dissolve in the rectal cavity to release the drug.

The compounds of this invention can be administered orally to humans in a dosage range of 1 to 100 mg/kg body weight in divided doses. One preferred dosage range is 1 to 10 mg/kg body weight orally in divided doses. Another preferred dosage range is 1 to 20 mg/kg body weight orally in divided doses. It will be understood, however, that the specific dose level and frequency,of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The present invention is also directed to combinations of the HIV reverse transcriptase inhibitor compounds with one or more agents useful in the treatment of AIDS. For example, the compounds of this invention may be effectively administered, whether at periods of pre-exposure and/or post-exposure, in combination with effective amounts of the AIDS antivirals, immunomodulators, anti-infectives, or vaccines of Table I.

TABLE I

ANTIVIRALS

| Drug Name | Manufacturer | Indication |
|---|---|---|
| AL-721 | Ethigen (Los Angeles, CA) | ARC, PGL HIV positive, AIDS |
| Recombinant Human Interferon Beta | Triton Biosciences (Almeda, CA) | AIDS, Kaposi's sarcoma, ARC |
| Acemannan | Carrington Labs (Irving, TX) | ARC (See also immunomodulators) |
| Cytovene Ganciclovir | Syntex (Palo Alto, CA) | sight threatening CMV peripheral CMV retinitis |
| d4T Didehydrodeoxy-thymidine | Bristol-Myers (New York, NY) | AIDS, ARC |
| ddI Dideoxyinosine | Bristol-Myers (New York, NY) | AIDS, ARC |
| EL10 | Elan Corp, PLC (Gainesville, GA) | HIV infection (See also immunomodulators) |

| Drug Name | Manufacturer | Indication |
|---|---|---|
| Trisodium Phosphonoformate | Astra Pharm. Products, Inc. (Westborough, MA) | CMV retinitis, HIV infection, other CMV infections |
| Dideoxycytidine; ddC | Hoffman-La Roche (Nutley, NJ) | AIDS, ARC |
| Novapren | Novaferon Labs, Inc. (Akron, OH) Diapren, Inc. (Roseville, MN, marketer) | HIV inhibitor |
| Peptide T Octapeptide Sequence | Peninsula Labs (Belmont, CA) | AIDS |
| Zidovudine; AZT | Burroughs Wellcome (Rsch. Triangle Park, NC) | AIDS, adv, ARC pediatric AIDS, Kaposi's sarcoma, asymptomatic HIV infection, less severe HIV disease, neurological involvement, in combination w/other therapies, post-exposure prophylaxis in health care workers |

| Drug Name | Manufacturer | Indication |
|---|---|---|
| Ansamycin LM 427 | Adria Laboratories (Dublin, OH) Erbamont (Stamford, CT) | ARC |
| Dextran Sulfate | Ueno Fine Chem. Ind. Ltd. (Osaka, Japan) | AIDS, ARC, HIV positive asymptomatic |
| Virazole Ribavirin | Viratek/ICN (Costa Mesa, CA) | asymptomatic HIV positive, LAS, ARC |
| Alpha Interferon | Burroughs Wellcome (Rsch. Triangle Park, NC) | Kaposi's sarcoma, HIV in combination AZT |
| Acyclovir | Burroughs Wellcome (Rsch. Triangle Park, NC) | AIDS, ARC, asymptomatic HIV positive, in combination with AZT |

IMMUNO-MODULATORS

| Drug Name | Manufacturer | Indication |
|---|---|---|
| Antibody which neutralizes pH labile alpha aberrant Interferon in an immuno-adsorption column | Advanced Biotherapy Concepts (Rockville, MD) | AIDS, ARC |

| Drug Name | Manufacturer | Indication |
|---|---|---|
| AS-101 | Wyeth-Ayerst Labs. (Philadelphia, PA) | AIDS |
| Bropirimine | Upjohn (Kalamazoo, MI) | advanced AIDS |
| Acemannan | Carrington Labs, Inc. (Irving, TX) | AIDS, ARC (See also anti-virals) |
| CL246,738 | American Cyanamid (Pearl River, NY) Lederle Labs (Wayne, NJ) | AIDS, Kaposi's sarcoma |
| EL10 | Elan Corp, PLC (Gainesville, GA) | HIV infection (See also anti-virals) |
| Gamma Interferon | Genentech (S. San Francisco, CA) | ARC, in combination w/TNF (tumor necrosis factor) |
| Granulocyte Macrophage Colony Stimulating Factor | Genetics Institute (Cambridge, MA) Sandoz (East Hanover, NJ) | AIDS |

| Drug Name | Manufacturer | Indication |
| --- | --- | --- |
| Granulocyte Macrophage Colony Stimulating Factor | Hoeschst-Roussel (Somerville, NJ) Immunex (Seattle, WA) | AIDS |
| Granulocyte Macrophage Colony Stimulating Factor | Schering-Plough (Madison, NJ) | AIDS<br>AIDS, in combination w/AZT |
| HIV Core Particle Immunostimulant | Rorer (Ft. Washington, PA) | seropositive HIV |
| IL-2 Interleukin-2 | Cetus (Emerycille, CA) | AIDS, in combination w/AZT |
| IL-2 Interleukin-2 Interleukin-2 | Hoffman-La Roche (Nutley, NJ) Immunex | AIDS, ARC, HIV, in combination w/AZT |
| Immune Globulin Intravenous (human) | Cutter Biological (Berkeley, CA) | pediatric AIDS, in combination w/AZT |
| IMREG-1 | Imreg (New Orleans, LA) | AIDS, Kaposi's sarcoma, ARC, PGL |
| IMREG-2 | Imreg (New Orleans, LA) | AIDS, Kaposi's sarcoma, ARC, PGL |
| Imuthiol Diethyl Dithio Carbamate | Merieux Institute (Miami, FL) | AIDS, ARC |

| Drug Name | Manufacturer | Indication |
| --- | --- | --- |
| Alpha-2 Interferon | Schering Plough (Madison, NJ) | Kaposi's sarcoma w/AZT: AIDS |
| Methionine-Enkephalin | TNI Pharmaceutical (Chicago, IL) | AIDS, ARC |
| MTP-PE Muramyl-Tripeptide | Ciba-Geigy Corp. (Summit, NJ) | Kaposi's sarcoma |
| Granulocyte Colony Stimulating Factor | Amgen (Thousand Oaks, CA) | AIDS, in combination w/AZT |
| rCD4 Recombinant Soluble Human CD4 | Genentech (S. San Francisco, CA) | AIDS, ARC |
| rCD4-IgG hybrids | | AIDS, ARC |
| Recombinant Soluble Human CD4 | Biogen (Cambridge, MA) | AIDS, ARC |
| Roferon-A Interferon Alfa 2a | Hoffman-La Roche (Nutley, NJ) | Kaposi's sarcoma AIDS, ARC, in combination w/AZT |

| Drug Name | Manufacturer | Indication |
|---|---|---|
| SK&F106528 Soluble T4 | Smith, Kline & French Laboratories (Philadelphia, PA) | HIV infection |
| Thymopentin | Immunobiology Research Institute (Annandale, NJ) | HIV infection |
| Tumor Necrosis Factor; TNF | Genentech (S. San Francisco, CA) | ARC, in combination w/gamma Interferon |

ANTI-INFECTIVES

| Drug Name | Manufacturer | Indication |
|---|---|---|
| Clindamycin with Primaquine | Upjohn (Kalamazoo, MI) | PCP |
| Fluconazole | Pfizer (New York, NY) | cryptococcal meningitis, candidiasis |
| Pastille Nystatin Pastille | Squibb Corp. (Princeton, NJ) | prevention of oral candidiasis |
| Ornidyl Eflornithine | Merrell Dow (Cincinnati, OH) | PCP |
| Pentamidine Isethionate (IM & IV) | LyphoMed (Rosemont, IL) | PCP treatment |

| Drug Name | Manufacturer | Indication |
|---|---|---|
| Piritrexim | Burroughs Wellcome (Rsch. Triangle Park, NC) | PCP treatment |
| Pentamidine isethionate for inhalation | Fisons Corporation (Bedford, MA) | PCP prophylaxis |
| Spiramycin | Phone-Poulenc Pharmaceuticals (Princeton, NJ) | cryptosporidial diarrhea |
| Intraconazole-R51211 | Janssen Pharm. (Piscataway, NJ) | histoplasmosis; cryptococcal meningitis |
| Trimetrexate | Warner-Lambert | PCP |

OTHER

| Drug Name | Manufacturer | Indication |
|---|---|---|
| Recombinant Human Erythropoietin | Ortho Pharm. Corp. (Raritan, NJ) | severe anemia assoc. and AZT therapy |
| Megestrol Acetate | Bristol-Myers (New York, NY) | treatment of anorexia assoc. w/AIDS |

| Drug Name | Manufacturer | Indication |
|---|---|---|
| Total Enteral Nutrition | Norwich Eaton Pharmaceuticals (Norwich, NY) | diarrhea and malabsorption related |

It will be understood that the scope of combinations of the compounds of this invention with AIDS antivirals, immunomodulators, anti-infectives or vaccines is not limited to the list in the above Table, but includes in principle any combination with any pharmaceutical composition useful for the treatment of AIDS.

EXAMPLE 1

Preparation of 5-ethyl-6-methyl-3-(2-napthylmethyl-amino)-2-(1H)-pyridinone

Step A) Preparation of 5-ethyl-6-methyl-3-nitro-2-(1H)-pyridinone

A mixture of 2-ethyl-3-oxobutanal, sodium salt (7.5 g, 55 mmol), nitroacetamide (6.6 g, 63 mmol), aqueous piperidinium acetate (4.4 mL) [prepared from glacial acetic acid (42 mL), water (100 mL) and piperidine (72 mL)] in water (45 mL) was stirred at room temperature for 22 hours. The yellow precipitate was collected by filtration and air dried to yield 8.0 g (80%) of 5-ethyl-6-methyl-3-nitro-2-(1H)-pyidinone.

Step B) Preparation of 3-amino-5-ethyl-6-methyl-2-(1H)-pyridinone

A yellow solution of the 5-ethyl-6-methyl-3-nitro-2-(1H)-pyidinone (10 g, 55 mmol) in a mixture of methanol and tetrahydrofuran (100 mL, 1:1 v/v) was reduced catalytically in the presence of 7% palladium on charcoal (0.7 g) under an atmosphere of hydrogen (50 psi) at room temperature over a period of 3.5 hours. The resultant mixture was filtered through a small pad of Celite. The filtrate was concentrated under reduced pressure (15 torr) to provide 5.7 g (68%) of the corresponding aminopyridone.

Step C) Preparation of 5-ethyl-6-methyl-3-(2-napthyl-methylamino)-2-(1H)-pyridinone

A mixture of 3-amino-5-ethyl-6-methyl-2-(1H)-pyridinone (3.0 g, 20 mmol), 2-bromomethylnapthalene (4.4 g, 20 mmol), triethylamine (2.0 g, 20 mmol) in acetonitrile (200 mL) was heated under reflux for 4 hours. The resultant mixture was allowed to cool to room temperature and concentrated under reduced pressure (15 torr). The residue was then subjected to column chromatography on silica gel (300 g, elution with methanol-chloroform, 5:95 v/v). Collection and concentration of appropriate fractions provided 2.4 g (42%) of the napthylmethylamino-pyridone.

Anal. Calcd for $C_{19}H_{20}N_2O$:
C, 75.71; H, 7.02; N, 9.30.
Found: C, 75.73; H, 6.71; N, 9.13.

EXAMPLE 2

3-[(2-Benzoxazolylmethyl)amino]-5-ethyl-6-methyl-2-(1H)-pyridinone

A solution of 3-amino-5-ethyl-6-methyl-2-(1H)-pyridinone (152 mg,1.0 mmol), 2-chloromethyl-1,3-benzoxazole (1.07 mmol) and triethylamine (0.14 mL, 1.0 mmol) in acetonitrile (10 mmL) was stirred at reflux for 24 hrs. After concentrating under reduced pressure,the residue was flash chromatographed over silica gel. Elution with 57 MeOH- 95% $CHCl_3$ gave 132 mg of product which was recrystallized from EtOH-water to give 95 mg of analytically pure product, mp 202-203°C, with initial melting at 179° followed by resolidification.

Anal. Calcd for $C_{16}H_{17}N_3O_2$:
C, 67.83, H, 6.05; N, 14.83
Found: C, 67.71; H, 6.03; N, 14.76.

EXAMPLE 3

3-[(2-Benzothiazolylmethyl)amino]-5-ethyl-6-methyl-2- (1H)-pyridinone

A solution of 3-amino-5-ethyl-6-methyl-2-(1H)-pyridinone (152 mg, 1.0 mmol), 2-chloromethyl-1,3-benzothiazole (184 mg, 1.0 mmol) and triethylamine (0.14 mL, 1.0 mmol) in absolute ethanol (5 mL) was stirred at reflux for 24 hours. After filtering and concentrating under reduced pressure, the residue was flash chromatographed over silica gel. Elution with 4% MeOH- 96% $CHCl_3$ gave 233 mg of product. Recrystallization from EtOH-water gave 77 mg of analytically pure product, mp 209-210°C.

Anal. Calcd for $C_{16}H_{17}N_3OS$:
C, 67.83; H, 6.05; N, 14.83.
Found: C, 67.71; H, 6.03; N, 14.70.

EXAMPLE 4

3-[2-(Benzoxazol-2-yl)ethyl]-5-ethyl-6-methyl-2-(1H)-pyridinone

Step A: Preparation of 3-cyano-5-ethyl-6-methyl-2-(1H)-pyridinone

According to the method described in J.Heterocyclic Chem., 24, 351 (1987), a mixture of 2-ethyl-3-oxobutanal, sodium salt (37.5 g, 0.275 mmol),,cyanoacetamide (25.2 g, 0.30 mol), aqueous piperidinium acetate (22 mL) [prepared from glacial acetic acid (4.2 mL), water (10mL) and piperidine (7.2 mL)] in water (775 ml) was refluxed for four hours. Glacial acetic acid (30 ml) was added cautiously (much foaming) as the product precipitated. Upon cooling to room temperature,the product was collected by filtration, washed with cold water and air dried to yield 22.3 g (50%), m.p. 237-240°C.

Step B: Preparation of 5-ethyl-6-methyl-2-(1H)-Pyridinone-3-carboxylic acid

An initial suspension of 3-cyano-5-ethyl-6-methyl-2-(1H)-pyridinone (4.86 g, 30 mmol) in 6N HCl (100 mL) was heated at reflux for twenty hours. Upon cooling, the product crystallized and was collected by filtration, washed with cold water and air dried to yield 3.73 g (69%).

Step C: Preparation of methyl 2-chloro-5-ethyl-6-methyl nicotinate

A mixture of 5-ethyl-6-methyl-2-(1H)-pyridinone-3-carboxylic acid (3.62 g, 20 mmol) and phosphorus pentachloride (4.38 g, 21 mmol) was heated, under a nitrogen atmosphere, at 100-120°C for 1.5 hours. The cooled residue was diluted with chloroform (70 mL) and then methanol (15 mL) was added. After stirring for 2-16 hours, the solution was poured into ice/water. The organic layer was separated and washed successively with water, saturated aqueous $NaHCO_3$, dried ($Na_2SO_4$), filtered and the solvent evaporated. This dark amber oil was dissolved in hexane, filtered through a pad of charcoal and the solvent evaporated to yield 3.31 g (78%) of pure product as a light yellow oil.

Step D: Preparation of methyl 2-methoxy-5-ethyl-6-methylnicotinate

To a solution of sodium metal (0.55 g, 24 mmol) dissolved in anhydrous methanol (15 mL), under a nitrogen atmosphere, was added a solution of methyl 2-chloro-5-ethyl-6-methylnicotinate (3.18 g, 14.9 mmol) in dry methanol (5 mL). This solution was refluxed and monitored by tlc (thin layer chromatogram) until the starting material had been consumed (about 24 hours). The cooled mixture was diluted with diethyl ether (50 mL), washed with water, saturated aqueous $NaHCO_3$, dried ($Na_2SO_4$), filtered and the solvent evaporated to yield 2.28 g (73%) of pure product as a light yellow oil.

Step E: Preparation of 2-methoxy-3-hydroxymethyl-5-ethyl-6-methyl pyridine

To a solution of methyl 2-methoxy-5-ethyl-6-methylnicotinate (2.28 g, 10.9 mmol) in anhydrous tetrahydrofuran (50 mL), under a nitrogen atmosphere, was added cautiously lithium aluminum hydride (0.77 g, 20 mmol). After refluxing this mixture for 15-20 hours, saturated aqueous $Na_2SO_4$ was added carefully to quench the cooled reaction mixture. This mixture was diluted with more THF, dried ($Na_2SO_4$), filtered and the solvent evaporated. This residue was chased with ethanol/toluene to remove traces of water and triturated with hexane as the product slowly crystallized out to give 1.30 g (66%), mp 53-55°C.

Step F: Preparation of 2-methoxy-5-ethyl-6-methyl nicotinaldehyde

Activated manganese dioxide (2.0 g) was added to a solution of 2-methoxy-3-hydroxymethyl-5-ethyl-6-methylpyridine (1.18 g, 6.5 mmol) in dry benzene (20 mL) and refluxed 5-10 hours. The warm suspension was filtered through a pad of anhydrous $Na_2SO_4$ and evaporated to yield 1.05 g (90%) of a viscous oil which solidified.

Step G: Preparation of 2-[2(R/S)-hydroxy-2-(2-methoxy-5-ethyl-6-methylpyridin-3-yl)ethyl]-benzoxazole

To a solution of 2-methylbenzoxazole (226 mg, 1.7 mmol) in anhydrous THF (4 mL), cooled to -100°C under an argon atmosphere, was added 1.6M n-butyllithium/hexane (1.05 mL) slowly over 35 minutes. After 0.5 hour

a solution of 2-methoxy-5-ethyl-6-methylnicotinaldehyde (300 mg, 1.7 mmol) in dry THF (1 mL) was added dropwise. The reaction was allowed to warm to room temperature and poured onto crushed ice. This mixture was extracted with diethyl ether. The combined extracts were dried ($MgSO_4$) and the solvent removed to give an oil which was flash chromatographed over silica gel. Elution with ethyl acetate/hexane (1:19) gave 340 mg (65%) of analytically pure racemic product, mp 102-103°C.

Anal. Calcd for $C_{18}H_{20}N_2O_3 \cdot 0.1\ H_2O$:
C, 68.81; H, 6.48; N, 8.92.
Found: C, 68.80; H, 6.76; N, 8.95.

Step H: Preparation of 3-[2-(benzoxazol-2-yl)-ethenyl]-5-ethyl-6-methyl-2-(1H)-pyridinone

A mixture of 2-[2(R/S)-hydroxy-2-(2-methoxy-5-ethyl-6-methylpyridin-3-yl)ethyl]benzoxazole (72 mg, 0.23 mmol) and pyridine hydrochloride (133 mg, 1.2 mmol), under a nitrogen atmosphere, was placed in a preheated oil bath (165°C) for 5 minutes. The reaction flask was removed, cooled, and water added to give a solid. This crude product was extracted into chloroform, dried ($MgSO_4$) and the solvent evaporated to yield 49 mg (75%) of pure product.

Recrystallization from methanol gave 15 mg of analytically pure product, mp 262-264°C.

Anal. Calcd for $C_{17}H_{16}N_2O_2$:
C, 72.83; H, 5.75; N, 10.00.
Found: C, 72.93; H, 5.95; N, 9.99.

Step I: Preparation of 3-[2-(benzoxazol-2-yl)ethyl]-5-ethyl-6-methyl-2-(1H)-pyridinone

A solution of 80% pure 3-[2-(benzoxazol-2-yl)ethenyl]-5-ethyl-6-methyl-2-(1H)-pyridinone (200 mg) in methanol/ethanol/THF (25 mL, 1:1:1) was hydrogenated at atmospheric pressure over 5% palladium/charcoal for four hours. After filtering off the catalyst, the solvents were evaporated and the residue flash chromatographed over silica gel.

Elution with 2% methanol-98% chloroform gave 75 mg of analytically pure product, mp 155-156.5°C.

Anal. Calcd. for $C_{17}H_{18}N_2O_2$
C, 72.31; H, 6.43; N, 9.92.
Found: C, 72.45; H, 6.52; N, 9.99.

EXAMPLE 5

Another, simpler procedure for the synthesis of the product of Example 4 is as follows.

Step A: Preparation of 3-cyano-5-ethyl-6-methyl-2-(1H)-pyridinone

According to the method described in J. Heterocyclic Chem., 24, 351 (1987), a mixture 2-ethyl-3-oxobutanol, sodium salt (37.5 g, 0.275 mol), cyanoacetamide (25.2 g, 0.30 mol), aqueous piperidinium acetate (22 mL) [prepared from glacial acetic acid (4.2 mL), water (10 mL) and piperidine (7.2 mL)] in water (775 ml) was refluxed for four hours. Glacial acetic acid (30 ml) was added cautiously (much foaming) as the product precipitated. Upon cooling to room temperature, the product was collected by filtration, washed with cold water and air dried to yield 22.3 g (50%), m.p. 237-240°C.

New

Step B: Preparation of 2-chloro-3-cyano-5-ethyl-6-methylpyridine

3-Cyano-5-ethyl-6-methyl-2-(1H)-pyridinone (22.9 g, .141 mol) and phosphorus pentachloride (33.1 g, .159 mol) were intimately mixed and heated at 110-120°C for one hour. The liquified solids were poured onto crushed ice and water and the semi-solid was extracted into chloroform. This extract was washed with water, saturated aqueous $NaHCO_3$, dried ($Na_2SO_4$), filtered and evaporated. This amber oil was dissolved in hexane and the insoluble material was removed when filtered through a pad of charcoal. Removal of the solvent gave a light yellow oil which solidified (17.7 g). Trituration of this solid with cold hexane yielded 15.6 g (61%) of pure product, m.p. 63-64°C.

New

Step C: Preparation of 2-methoxy-3-cyano-5-ethyl-6-methylpyridine

Sodium metal (3.25 g, .141 mol) was dissolved in dry methanol (100 mL) under a nitrogen atmosphere. When solution was complete, a slurry of 2-chloro-3-cyano-5-ethyl-6-methylpyridine (17.95 g, 99.4 mmol) in dry methanol (70 mL) was added and the reaction was warmed at 60°C for 15-20 hours. After cooling the reaction mixture, diethyl ether (250 mL) and water (200 mL) were added. The ether layer was separated and washed with water, dried ($Na_2SO_4$), filtered and evaporated to give a light yellow solid (17.5 g). This solid was triturated with cold hexane to yield 14.4 g (82%) of pure product, m.p. 59-61°C.

New

Step D: Preparation of 2-methoxy-5-ethyl-6-methyl-nicotinaldehyde

To a solution of 2-methoxy-3-cyano-5-ethyl-6-methylpyridine (1.0 g, 5.68 mmol) in dry tetrahydrofuran (50 mL) under a nitrogen atmosphere and cooled to -70°C, was added 1.3M diisobutyl aluminum hydride/THF (17.4 mL, 22.7 mmol). The resulting mixture was allowed to warm to room temperature and stir for 15-20 hours. The reaction mixture was acidified with 1N hydrochloric acid and then neutralized with aqueous sodium bicarbonate. Water was then added and the product extracted into diethyl ether. The etheral extract was dried ($Na_2SO_4$), filtered and the solvent evaporated. This residue was flash chromatographed on silica gel eluting with 10% diethyl ether/pentane to give 610 mg(61%) of product.

Step E (old step G): Preparation of 2-[2(R/S)-hydroxy-2-(2-methoxy-5-ethyl-6-methyl-pyridin-3-yl)ethyl]-benzoxazole

To a solution of 2-methylbenzoxazole (226 mg, 1.7 mmol) in anhydrous THF (4 mL), cooled to -100°C under an argon atmosphere, was added 1.6M n-butyllithium/hexane (1.05 mL) slowly over 35 minutes. After 0.5 hour a solution of 2-methoxy-5-ethyl-6-methylnicotinaldehyde (300 mg, 1.7 mmol) in dry THF (1 mL) was added dropwise. The reaction was allowed to warm to room temperature and poured onto crushed ice. This mixture was extracted with diethyl ether. The combined extracts were dried ($MgSO_4$) and the solvent removed to give an oil which was flash chromatographed over silica gel. Elution with ethyl acetate/hexane (1:19) gave 340 mg (65%) of analytically pure racemic product, mp 102-103°C.

Anal. Calcd for $C_{18}H_{20}N_2O_3 \cdot 0.1\ H_2O$:
C, 68.81; H, 6.48; N, 8.92.
Found: C, 68.80; H, 6.76; N, 8.95.

Step F (old step H): Preparation of 3-[2-(benzoxazol-2-yl)-ethenyl]-5-ethyl-6-methyl-2-(1H)-pyridinone

A mixture of 2-[2(R/S)-hydroxy-2-(2-methoxy-5-ethyl-6-methylpyridin-3-yl)ethyl]benzoxazole (72 mg, 0.23 mmol) and pyridine hydrochloride (133 mg, 1.2 mmol), under a nitrogen atmosphere, was placed in a preheated oil bath (165°C) for 5 minutes. The reaction flask was removed, cooled, and water added to give a solid. This crude product was extracted into chloroform, dried ($MgSO_4$) and the solvent evaporated to yield 49 mg (75%) of pure product.

Recrystallization from methanol gave 15 mg of analytically pure product, mp 262-264°C.

Anal. Calcd for $C_{17}H_{16}N_2O_2$:
C, 72.83; H, 5.75; N, 10.00.
Found: C, 72.93; H, 5.95; N, 9.99.

Step G (old step I): Preparation of 3-[2-(benzoxazol-2-yl)ethyl]-5-ethyl-6-methyl-2-(1H)-pyridinone

A solution of 80% pure 3-[2-(benzoxazol-2-yl)ethenyl]-5-ethyl-6-methyl-2-(1H)-pyridinone (200 mg) in mettianol/ethanol/THF (25 mL, 1:1:1) was hydrogenated at atmospheric pressure over 5% palladium/charcoal for four hours. After filtering off the catalyst, the solvents were evaporated and the residue flash chromatographed over silica gel. Elution with 2% methanol-98% chloroform gave 75 mg of analytically pure product, mp 155-156.5°C.

Anal. Calcd. for $C_{17}H_{18}N_2O_2$
C, 72.31; H, 6.43; N, 9.92.

Found: C, 72.45; H, 6.52; N, 9.99.

EXAMPLE 6

3-[2-(4,7-Dimethylbenzoxazol-2-yl)ethyl]-5-ethyl-6-methyl-2-(1H)-pyridinone

Step A: Preparation of 2-methoxy-3-[2-(4,7-dimethyl-benzoxazol-2-yl)ethenyl]-5-ethyl-6-methyl pyridine

Sodium hydride (60% in mineral oil, 47 mg, 1.15 mmol) was added to a suspension of [(4,7-dimethylbenzoxazo1-2-yl)methyl]triphenylphosphonium chloride (485 mg, 1.06 mmol) [prepared by heating 2-chloromethyl-4,7-dimethylbenzoxazole with an equimolar amount of triphenylphosphine in refluxing toluene for 15-25 hours] in dry tetrahydrofuran (8 mL) under a nitrogen atmosphere at 25°C. After 15 minutes, solid 2-methoxy-5-ethyl-6-methyl nictotin-aldehyde (197 mg, 1.15 mmol) was added to the yellow suspension. This reaction mixture was heated at reflux for 15-25 hours. Upon cooling, this reaction was diluted with chloroform, washed with water, dried ($Na_2SO_4$), filtered and the solvent evaporated. This residue was flash chromatographed on silica gel and the product eluted with chloroform to yield 178 mg (55%) of a cis/trans mixture of product as an oil.

Step B: Preparation of 2-methoxy-3-[2-(4,7-dimethyl-benzoxazol-2-yl)ethyl]-5-ethyl-6-methyl pyridine

A solution of crude cis/trans 2-methoxy-3-[2-(4,7-dimethylbenzoxazol-2-yl)ethenyl]-5-ethyl-6-methylpyridine (178 mg, 0.55 mmol) in methanol (4 mL) and tetrahydrofuran (5 mL) containing 5% palladium on charcoal (67 mg) was hydrogenated at atmospheric pressure for 5-15 hours. The catalyst was filtered off and the solution evaporated to yield 161 mg (89%) of product as an oil.

Step C: Preparation of 3-[2-(4,7-dimethylbenzoxazol-2-yl)ethyl]-5-ethyl-6-methyl-2(1H)-pyridinone

A mixture of crude 2-methoxy-3-[2-(4,7-dimethylbenzoxazol-2-yl)ethyl]-5-ethyl-6-methylpyridine (161 mg, .49 mmol) and pyridine hydrochloride (574 mg, 4.97 mmol) was warmed in a pre-heated oil bath at 140°C for 15 minutes. The cooled residue was diluted with water and the product extracted into chloroform. This extract was dried ($Na_2SO_4$), filtered and the solvent evaporated. This residue was chromatographed on silica gel and eluted with a 0.5 to 2.5% methanol/chloroform gradient to yield 65 mg of pure product. The product was crystallized from diethyl ether to give 38 mg, m.p. 151.5-153°C.

Anal. Calcd. for $C_{19}H_{22}N_2O_2 \cdot 0.15\ H_2O$

C, 72.88; H, 7.18; N, 8.95.

Found: C, 72.89; H, 7.16; N, 8.87.

EXAMPLE 7

Preparation of 3-[((4,7-Dimethylbenzoxazol-2-yl)-methyl)amino]-5-ethyl-6-methyl-2(1H)-pyridinone

Step A: Preparation of 2-chloromethyl-4,7-dimethyl-benzoxazole

To a solution of 2,5-dimethyl-6-aminophenol (0.67 g, 4.9 mmol) in methylene chloride, solid ethyl 2-chloroiminoacetate hydrochloride (0.85 g, 4.9 mmol) was added. The resultant slurry was stirred at room temperature for 18 hours, then filtered through a plug of diatomaceous earth and concentrated under reduced pressure (15 torr). The solid residue was subjected to column chromatography on silica gel (50 g, eluted with 1% methanol in chloroform).

Collection and concentration of appropriate fractions yielded 0.85 g (89%) of the benzoxazole.

Step B: Preparation of 3-[((4,7-dimethylbenzoxazol-2-yl)methyl)amino]-5-ethyl-6-methyl-2-(1H)-pyridinone

A mixture of 3-amino-5-ethyl-6-methyl-2-(1H)-pyridinone (0.23 g, 1.5 mmol), 2-chloromethyl-4,7-dimethylbenzoxazole (0.29 g, 1.5 mmol), diisopropyl-ethylamine (0.39 g, 3 mmol) in acetonitrile (50 mL) was refluxed under an atmosphere of nitrogen for 12 hours. The resultant mixture was concentrated under reduced pressure (15 torr). The residue was then subjected to column chromatography on silica gel (100 g, elution with 4% methanol in chloroform).

Collection and concentration of appropriate fractions provided 0.2 g (44%) of the benzoxazolylmethylaminopyridone.

Anal. Calcd for $C_{18}H_{21}N_3O_2$:
C, 69.43; H, 6.80; N, 13.49.
Found: C, 69.32; H, 6.66; N, 13.47.

EXAMPLE 8

Preparation of 5-ethyl-6-methyl-3-(2-napthylmethyl-amino)-2-(1H)-pyridinethione

Step A: Preparation of 2-chloro-5-ethyl-6-methyl-3-nitropyridine

A mixture of 5-ethyl-6-methyl-3-nitro-2-(1H)-pyridinone (2.38 g, 13 mmol) and phosphorus pentachloride (3.3 g, 15.6 mmol) was heated under an atmosphere of nitrogen at 140-150°C for 15 minutes. The resultant brown oil was then treated with water and the product extracted into chloroform. The organic extract was washed three times with brine, dried over anhydrous sodium sulfate, filtered and concentrated under reduced pressure (15 torr). The residue was passed through a small plug of silica gel, eluted with 2% methanol in chloroform. Collection and concentration of appropriate fractions yielded 1.5 g (57.5%) of the corresponding chloropyridine as a clear pale brown oil.

Step B: Preparation of 2-tert-butylthio-5-ethyl-6-methyl-3-nitropyridine

To a suspension of sodium hydride (0.23 g, 9.8 mmol) indimethylformamide (DMF, 20 mL) under an atmosphere of nitrogen, 2-methyl-2-propanethiol (1 mL, 8 mmol) was added. After stirring at room temperature for 15 minutes, a clear solution was obtained. Then a solution of 2-chloro-5-ethyl-6-methyl-3-nitropyridine (1.5 g, 7.5 mmol) in DMF (2 mL) was added, and the reacting mixture turned dark green to black in color. After addition was completed, the mixture was stirred for an additional 15 minutes, and then concentrated under reduced pressure (0.1 torr). The residue was subjected to column chromatography on silica gel, eluted with 60% chloroform in hexane. Collection and concentration of appropriate fractions yielded 0.5 g (26%) of the corresponding 2-tert-butylthiopyridine as a bright yellow solid.

Step C: Preparation of 3-amino-2-tert-butylthio-5-ethyl-6-methylpyridine

To a yellow solution of 2-tert-butylthio-5-ethyl-6-methyl-3-nitropyridine (0.5 g, 2 mmol) in methanol (50 mL) at room temperature, a solution of sodium hydrosulfite ($Na_2S_2O_4$, 2.5 g, 14 mmol) in water (25 mL) was added. The resulting white suspension was stirred for an additional five minutes and concentrated under reduced pressure (15 torr). The residue solid was triturated three times with chloroform, and the extracts were combined, dried over sodium sulfate, and concentrated under reduced pressure (15 torr). The resulting residue was then passed through a small plug of silica gel and eluted with 3% methanol in chloroform. Collection and concentration of appropriate fractions yielded 0.3 g (68%) of the aminopyridine.

Step D: Preparation of 2-tert-butylthio-5-ethyl-6-methyl-3-(2-napthylmethylamino)pyridine

A mixture of 3-amino-2-tert-butylthio-5-ethyl-6-methylpyridine (0.15 g, 0.67 mmol), 2-bromomethylnapthalene (0.15 g, 0.67 mmol), diisopropylethylamine (86 mg, 0.67 mmol) in acetonitrile (5 mL) was refluxed under an atmosphere of nitrogen for 16 hours. The resultant mixture was concentrated under reduced pressure (15 (torr). The residue was subjected to column chromatography on silica gel (50 g, elution with 1% methanol in chloroform). Collection and concentration of appropriate fractions provided 98 mg (40%) of the napthylmethylaminopyridine.

Step E: Preparation of 5-ethyl-6-methyl-3-(2-napthylmethylamino)-2-(1H)-pyridinthione

A mixture of 2-tert-butylthio-5-ethyl-6-methyl-3-(2-napthylmethylamino)pyridine (98 mg, 0.27 mmol) and pyridine hydrochloride (0.45 g, 3.8 mmol) was heated under an atmosphere of nitrogen at 140°C until evolution of gas ceased (about 45 minutes). Water was added and the resultant mixture was extracted three times with chloroform. The organic extracts were combined, dried over anhydrous sodium sulfate, filtered and concentrated under reduced pressure (I5 torr). The residue was then subjected to column chromatography on silica gel (eluted with 2% methanol in ethyl acetate). Collection and concentration of appropriate fractions yielded 32 mg (36%) of the pyridinethione.
Anal. Calcd for $C_{19}H_{20}N_2S \cdot 0.25$ EtOAc:

C, 72.68; H, 6.71; N, 8.48.
Found: C, 72.41; H, 6.59; N, 8.86.

EXAMPLE 9

3-[(Benzoxazol-2-yl)methoxy]-5-ethyl-6-methyl-2(1H)-pyridinone

Step A: Preparation of 2-chloro-3-nitro-5-ethyl-6-methylpyridine

A mixture of 3-nitro-5-ethyl-6-methylpyridin-2(1H)-one (0.91 g, 5.0 mmol) and phosphorus pentachloride (1.25 g, 6.0 mmol), under a nitrogen atmosphere, was heated at 140°C for 0.5 hours. The cooled mixture was diluted with chloroform and ice water. The separated chloroform layer was then washed with water, saturated aqueous $NaHCO_3$ and dried ($Na_2SO_4$). After filtering through a pad of charcoal, solvent was evaporated to yield 672 mg (67%) of product.

Step B: Preparation of 2-methoxy-3-nitro-5-ethyl-6-methylpyridine

Sodium metal (100 mg, 4.3 mmol) was dissolved in methanol (5 mL) under a nitrogen atmosphere. A solution of 2-chloro-3-nitro-5-ethyl-6-methylpyridine (677 mg, 3.37 mmol) in methanol (5 mL) was added dropwise. The reaction was warmed at 50°C for four hours. The reaction was cooled, diluted with diethyl ether, the ether layer washed with water, dried ($Na_2SO_4$), filtered through a pad of charcoal and evaporated to yield 535 mg (80%) of product.

Step C: Preparation of 2-methoxy-3-amino-5-ethyl-6-methylpyridine

A solution of 2-methoxy-3-nitro-5-ethyl-6-methylpyridine (535 mg, 2.72 mmol) in methanol (8 mL) and tetrahydrofuran (8 mL) containing 5% palladium on carbon (83 mg) was hydrogenated at atmospheric pressure for 5 hours. The catalyst was filtered off the solvent evaporated to yield 436 mg (98%) of oily product.

Step D: Preparation of 2-methoxy-3-hydroxy-5-ethyl-6-methylpyridine

A batch of 2-methoxy-3-amino-5-ethyl-6-methylpyridine (171 mg, 1.05 mmol) was dissolved in 5% aqueous sulfuric acid (4 mL), cooled in an ice bath, then a solution of sodium nitrite (78 mg, 1.13 mmol) in water (1 mL) was added dropwise. After 0.5 hour, the resulting mixture was added dropwise to 5% aqueous sulfuric acid (6 mL) warmed at 110°C. The solution was stirred for 0.5 hour, cooled and the product extracted into chloroform, then dried ($Na_2SO_4$), filtered and evaporated to yield 77 mg (43%) of product.

Step E: Preparation of 3-[(benzoxazol-2-yl)methoxy]-2-methoxy-5-ethyl-6-methylpyridine

A quantity of 60% sodium hydride in mineral oil (24 mg, 0.6 mmol) was added to a solution of 2-methoxy-3-hydroxy-5-ethyl-6-methylpyridine (77 mg, 0.46 mmol) in dry dimethylformamide (2 mL). After gas evolution ceased, 2-(chloromethyl)benzoxazole (100 mg, 0.6 mmol) was added and the reaction mixture warmed at 60°C for one hour. The reaction was then cooled, diluted with diethyl ether, the ether extract washed with water, dried ($Na_2SO_4$), filtered and evaporated to give 151 mg of crude mixture. This mixture was flash chromatographed on silica gel, eluting with 0.5% methanol/chloroform. Combined appropriate fractions gave 46 mg (32%) of oily product.

Step F: Preparation of 3-[(benzoxazol-2-yl)methoxy]-5-ethyl-6-methyl-2(1H)-pyridinone

3-[(Benzoxazol-2-yl)methoxy]-2-methoxy-5-ethyl-6-methylpyridine (140 mg, .47 mmol) was dissolved in methylene chloride (5 ml) and cooled in an ice bath under an atmosphere of nitrogen. 1M Boron tribromide (2.3 mL, 2.3 mmol) in hexane was added to this solution and the reaction mixture was allowed to warm to room temperture over a 0.75 hour period. This mixture was re-cooled in an ice bath and saturated aqueous $NaHCO_3$ (5 mL) was added to quench the reaction. The methylene chloride layer was dried ($Na_2SO_4$), filtered and evaporated. The residue was triturated with diethyl ether to give 69 mg of crude product. Recrystallization from methanol yielded 60 mg (45%), m.p. 198-200°C.

Anal. Calcd for $C_{16}H_{16}N_2O_3$:
C, 67.59; H, 5.67; N, 9.86.

Found: C, 67.59; H, 5.66; N, 9.83.

EXAMPLE 10

3-{[(Benzoxazol-2-yl)amino]methyl}-5-ethyl-6-methyl-2-(1H)-pyridinone

Step A: Preparation of 2-methoxy-3-(aminomethyl)-5-ethyl-6-methylpyridine

A solution of 2-methoxy-3-cyano-5-ethyl-6-methylpyridine (344 mg, 1.95 mmol) in methanol (8 mL) and 4.9 M methanolic HCl (5 mL) containing 5% palladium on carbon (100 mg) was hydrogenated at atmospheric pressure for 10-20 hours. The catalyst was filtered off and the solvent evaporated. This residue was made basic with sodium hydroxide and the product was extracted into methylene chloride, dried ($Na_2SO_4$), filtered and evaporated to give 313 mg (89% yield) of oily product.

Step B: Preparation of 2-methoxy-3-{[(benzoxazol-2-yl)aminolmethyl)-5-ethyl-6-methylpyridine

To a solution of 2-methoxy-3-(aminomethyl)-5-ethyl-6-methylpyridine (98 mg, 0.54 mmol) in methanol (1 mL), under a nitrogen atmosphere was added 2-chlorobenzoxazole (0.07 mL, 0.61 mmol), followed by triethylamine (0.076 mL, 0.55 mmol). After two hours, product began to crystallize out. The mixture was stirred for 12 hours and the product (133 mg, 82%) collected by filtration. This material was dissolved in diethyl ether, filtered through a charcoal pad and hexane added to the solution. As the ether was boiled off, product crystallized out to give 96 mg, m.p. 141.5-142.5°C.

Anal. Calcd. for $C_{17}H_{19}N_3O_2$:
C, 68.67; H, 6.44; N, 14.13.
Found: C, 68.68; H, 6.36; N, 14.07.

Step C: Preparation of 3-{[(benzoxazol-2-yl)amino]-methyl)-5-ethyl-6-methyl-2-(1H)-pyridinone

A mixture of 2-methoxy-3-{[(benzoxazol-2- yl)amino]methyl}-5-ethyl-6-methylpyridine (80 mg, 0.269 mmol) and pyridine hydrochloride (335 mg, 2.9 mmol), under a nitrogen atmosphere, was warmed in an oil bath preheated to 150°C for 5 minutes. This solidified mixture was cooled, diluted with water and the crude precipitate produce collected by filtration. This material was dissolved in methylene chloride, filtered through a charcoal pad and then diluted with hexane. As the methylene chloride was boiled off the product crystallized out to give 40 mg (53%), m.p. 211-213°C.

Anal. Calcd. for $C_{16}H_{17}N_3O_2$:
C, 67.82; H, 6.05; N, 14.83.
Found: C, 68.08; H, 6.03; N, 14.86.

EXAMPLE 11

3-{[(Benzoxazol-2-yl)amino]methyl}-5-ethyl-6-methyl-2-(1H)-pyridinethione

Step A: Preparation of 2-t-butylthio-3-cyano-5-ethylpyridine

t-Butylmercaptan (0.40 mL, 3.55 mmol) was added to a suspension of 60% sodium hydride in mineral oil (158 mg, 3.85 mmol) in dry dimethylformamide (5 mL). After 10 minutes, when gas evolution ceased, 2-chloro-3-cyano-5-ethyl-6-methylpyridine (542 mg, 3.0 mmol) was added. After stirring for one hour, water was added and product extracted into diethyl ether. This extract was dried ($Na_2SO_4$), filtered and the solvent removed to give 648 mg (92%) of crystalline product, m.p. 63-66°C.

Step B: Preparation of 2-t-butylthio-3-(aminomethyl)-5-ethyl-6-methylpyridine

To a suspension of lithium aluminum hydride (54 mg, 1.42 mmol) in diethyl ether (10 mL), under a nitrogen atmosphere, was added dropwise a solution of 2-t-butylthio-3-cyano-5-ethylpyridine (222 mg, 0.95 mmol) in diethyl ether (6 mL). After stirring for 3.5 hours at room temperature, the reaction was quenched with saturated aqueous sodium sulfate and the ether layer dried ($Na_2SO_4$), filtered and evaporated to give 219 mg (96%) of crude oily product.

Step C: Preparation of 2-t-butylthio-3-{[(benzoxazol-2-yl)amino]methyl}-5-ethyl-6-methylpyridine

To a solution of crude 2-t-butylthio-3-(aminomethyl)-5-ethyl-6-methylpyridine (219 mg, 0.9 mmol) in methanol (2 mL) was added 2-chlorobenzoxazole (0.12 mL, 1.0 mmol), followed by triethyl amine (0.14 mL, 1.0 mmol). After stirring for 12 hours, the solvent was evaporated and the residue dissolved in diethyl ether, which was washed with water, dried ($Na_2SO_4$), filtered and evaporated. This crude residue was flash chromatographed on silica gel eluting with 12% ethyl acetate/hexane. The appropriate fractions were combined, the solvent evaporated and the residue triturated with hexane to give 114 mg (35%) of pure product, m.p. 141-143°C.

Anal. Calcd. for $C_{30}H_{25}N_3OS$:
C, 67.57; H, 7.09; N, 11.82.
Found: C, 67.78; H, 7.21; N, 11.77.

Step D: Preparation of 3-{[(benzoxazol-2-yl)amino]-methyl)-5-ethyl-6-methyl-2(1H)-pyridinethione

A mixture of 2-t-butylthio-3-{[(benzoxazol-2-yl)amino]methyl}-5-ethyl-6-methylpyridine (78 mg, 0.22 mmol) and pyridine hydrochloride (325 mg, 2.81 mmol) was warmed in a pre-heated oil bath at 150°C for 25 minutes. The mixture was cooled, diluted with water and the yellow precipitated product was collected by filtration. This residue was dissolved in methylene chloride, filtered through a pad of charcoal and then hexane was added and the methylene chloride boiled off as the product crystallized out to yield 42 mg (64%), m.p. 238-240°C.

Anal Calcd. for $C_{16}H_{17}N_3OS$:
C, 64.18; H, 5.72; N, 14.04.
Found: C, 63.86; H, 5.69; N, 13.81.

EXAMPLE 12

3-{[1-naphthylamino]methyl}-5-ethyl-6-methyl-2-(1H)-pyridinone

Step A: Preparation of 2-methoxy-3-{[1-naphthylaminolmethyl)-5-ethyl-6-methylpyridine

A solution of 2-methoxy-5-ethyl-6-methylnicotinaldehyde (100 mg, 0.6 mmol) and 1-aminonaphthalene (80 mg, 0.6 mmol) in ethanol (1 mL) containing p-toluenesulfonic acid (~5 mg) was warmed at 60°C for 5-15 hours, then cooled and sodium borohydride (40 mg, 1.0 mmol) added. After 0.5 hour, the reaction mixture was diluted with water, acidified with dilute aqueous hydrochloric acid and product extracted into diethyl ether. The extract was dried ($MgSO_4$), filtered a through pad of charcoal and evaporated to yield 177 mg of greenish oil. Trituration of this residue with methanol gave 40 mg of crystalline product, m.p. 82-83°C.

Anal. Calcd for $C_{20}H_{22}N_2O$:
C, 77.48; H, 7.28; N, 9.04
Found: C, 77.42; H, 7.18; N, 8.94.

Step B: Preparation of 3-{[1-napthylamino]methyl}-5-ethyl-6-methyl-2-(1H)-pyridinone

A mixture of 2-methoxy-3-{[1-naphthylamino]methyl}-5-ethyl-6-methylpyridine (125 mg, 0.41 mmol) and pyridine hydrochloride (500 mg, 4.3 mmol) was warmed at 145-160°C for 20 minutes. The reaction mixture was then cooled, water added, and the mixture made acidic with dilute aqueous hydrochloride acid. The product was then extracted into chloroform. The chloroform extracts were concentrated and the residue flash chromatographed on silica gel eluting with 5% methanol/95% chloroform. Appropriate fractions were combined and evaporated. The residue was triturated with diethyl ether to give 18.9 mg (15%) of product., m.p. 165-167°C.

Anal. Calcd. for $C_{19}H_{20}N_2O$:
C, 78.05; H, 6.90; N, 9.58
Found: C, 77.81; H, 7.11; N, 9.22.

EXAMPLE 13

3-[2-(Benzoxazol-2-yl)ethyl]-5-ethyl-6-methyl-2(1H)-pyridinethione

A mixture of 3-[2-(benzoxazol-2-yl)ethyl]-5-ethyl-6-methyl-2(1H)-pyridinone (150 mg, 0.53 mmol) and 2,4-bis(4-methoxyphenyl)-1,3-dithia-2,4-diphosphetane-2,4-disulfide (Lawesson's Reagent)(485 mg, 1.2 mmol) in

dry toluene (4 mL) was refluxed for 6-10 hours. The solvent was evaporated and the reaction residue was flash chromatographed on silica gel eluting with 0.5% methanol/chloroform. The appropriate fractions were combined and crystallized from ethyl acetate to yield 80 mg of slightly impure product. This material was re-chromatographed on silica gel eluting with chloroform. The appropriate fractions were combined, the solvent evaporated, and the residue recrystallized from boiling diethyl ether, after addition of sufficient methylene chloride to effect complete dissolution, to yield 39 mg (24%) of pure product, m.p. 199-201°C.

| | |
|---|---|
| Anal. | Calcd. for $C_{17}H_{18}N_2OS$: |
| | C, 68.42; H, 6.08; N, 9.39. |
| Found: | C, 68.82; H, 5.86; N, 9.32. |

EXAMPLE 14 3-{[N-Phthalimidomethyl]amino}-5-ethyl-6-methyl-2(1H)-pyridinone

To a suspension of 3-amino-5-ethyl-6-methyl-pyridin-2(1H)-one (73 mg, 0.48 mmol) in absolute ethanol (1.5 mL), under a nitrogen atmosphere, was added N-(hydroxymethyl)phthalimide (89 mg, 0.50 mmol). This mixture was refluxed for three hours during which complete dissolution of reagent occurred, followed by precipitation of the bright yellow product. Upon cooling, the precipitated product was collected by filtration and rinsed with ethanol and the diethyl ether to yield 130 mg (87%), m.p. 245-247°C.

| | |
|---|---|
| Anal. | Calcd. for $C_{17}H_{17}N_3O_3$: |
| | C, 65.58; H, 5.51; N, 13.50. |
| Found: | C, 65.42; H, 5.52; N, 13.51. |

EXAMPLE 15

3-(2-Phthalimidoethyl)-5-ethyl-6-methyl-2(1H)-pyridinone

Step A: Preparation of 2-methoxy-3-(2-amino-1-(R/S)-hydroxy ethyl)-5-ethyl-6-methylpyridine

A mixture of 2-methoxy-5-ethyl-6-methylnicotinaldehyde (1.05 g, 5.86 mmol) and trimethylsilyl cyanide (0.85 mL, 6.37 mmol) containing zinc iodide (10 mg) was stirred, under a nitrogen atmosphere, at ambient temperature for two hours. This liquid was diluted with anhydrous diethyl ether, filtered and then added dropwise to a suspension of lithium aluminum hydride (225 mg, 5.9 mmol) in diethyl ether (15 mL) under nitrogen. After two hours the reaction was quenched with saturated aqueous sodium sulfate, diluted with methylene chloride, filtered and the solvents evaporated to give a semi-solid. This residue was triturated with cold diethyl ether and the crystalline product was collected by filtration to yield 346 mg (28%).

Step B: Preparation of 2-methoxy-3-(2-phthalimido-1-(R/S)-hydroxyethyl)-5-ethyl-6-methylpyridine

To a suspension of 2-methoxy-3-(2-amino-1(R/S)hydroxyethyl)-5-ethyl-6-methylpyridine (345 mg, 1.64 mmol) in ethanol (5 mL) was added N-(carbethoxy)phthalimide (373 mg, 1.70 mmol). The reaction was stirred for six hours as the suspension dissolved. The solvent was evaporated and the viscous residue triturated with ethyl acetate/diethyl ether and product crystallized. By filtration 273 mg (49%) of pure product was collected, m.p. 134-135°C. These mother liquors were flash chromatographed on silica gel and eluted with 20% ethyl acetate/80% hexane to give an additional 83 mg (15%) of product.

| | |
|---|---|
| Anal. | calcd. for $C_{19}H_{20}N_2O_4$: |
| | C, 67.04; H, 5.92; N, 8.23. |
| Found: | C, 67.12; H, 5.92; N, 8.21. |

Step C: Preparation of 3-(2-Phthalimidoethenyl)-5-ethyl-6-ethyl-2(1H)-pyridinone

A mixture of 2-methoxy-3-(2-phthalimido-1(R/S)-hydroxyethyl)-5-ethyl-6-methylpyridine (172 mg, 0.50 mmol) and pyridine hydrochloride (600 mg, 5.3 mmol) was placed in a preheated oil bath at 150°C for ten minutes. The mixture was cooled, diluted with water and the precipitated product collected by filtration and air dried to yield 145 mg (93%).

Recrystallization from methanol gave analytically pure product m.p. 296-298°C.

| | |
|---|---|
| Anal. | Calcd. for $C_{18}H_{16}N_2O_3$: |
| | C, 70.11; H, 5.23; N, 9.09. |
| Found: | C, 69.81; H, 4.99; N, 9.05. |

Step D: Preparation of 3-(2-Phthalimidoethyl)-5-ethyl-6-methyl-2(1H)-pyridinone

A partial suspension of 3-(2-phthalimidoethenyl)-5-ethyl-6-methyl-2(1H)-pyridinone (145 mg, 0.47 mmol) in methanol (20 mL) and tetrahydrofuran (20 mL) containing 5% palladium/carbon (117 mg) was hydrogenated at atmospheric pressure for 10-20 hours. The catalyst was filtered off and the solvents evaporated to give product. This material was recrystallized from methanol to yield 110 mg (75%), m.p. 232-233°C.

Anal. Calcd. for $C_{18}H_{18}N_2O_3$:
C, 69.66; H, 5.85; N, 9.03.
Found: C, 69.44; H, 5.68; N, 8.98.

EXAMPLE 16

3-(2-phthalimidoethyl)-5-ethyl-6-methyl-2(1H)-pyridinone

Step A: Preparation of 2-methoxy-3-(2-nitroethenyl)-5-ethyl-6-methylpyridine

A mixture of 2-methoxy-5-ethyl-6-methyl-nicotinaldehyde (2.25 g, 12.6 mmol), nitromethane (1.9 g, 31.1 mmol), monomethylamine hydrochloride (69 mg) and sodium hydroxide (20 mg) in absolute ethanol (1.3 mL) was stirred at ambient temperature for three days. This mixture was diluted with methanol and the precipitated yellow crystalline product was collected by filtration to yield 2.39 g (86%), m.p. 105-107°C.

Step B: Preparation of 2-methoxy-3-(2-aminoethyl)-5-ethyl-6-methylpyridine

A suspension of 2-methoxy-3-(2-nitroethenyl)-5-ethyl-6-methylpyridine (445 mg, 2.0 mmol) in methanol (10 mL) and 4.7M methanolic hydrogen chloride (2 mL) containing 5% palladium/carbon (110 mg) was hydrogenated at atmospheric presure over 10-20 hours. The catalyst was filtered off and the solvent evaporated. The residue was made basic with sodium hydroxide solution and the product extracted into methylene chloride, dried, filtered and the solvent evaporated to yield 332 mg of crude oily product. No further purification was performed.

Step C: Preparation of 2-methoxy-3-(2-phthalimidoethyl)-5-ethyl-6-methylpyridine

To a solution of crude 2-methoxy-3-(2-aminoethyl)-5-ethyl-6-methylpyridine (332 mg) in ethanol (7 mL) was added N-(carbethoxy)phthalimide (406 mg, 1.85 mmol). After stirring at ambient temperature for three hours the solvent was evaporated and the residue was chromatographed on silica gel by gradient elution with 20-100% ethyl acetate/hexane. The appropriate fractions were combined, the solvent evaporated and the residue triturated with hexane as the product slowly crystallized out to yield 202 mg of pure product, m.p. 77-79°C.

Anal. Calcd. for $C_{19}H_{20}N_2O_3$:
C, 70.35; H, 6.22; N., 8.64.
Found: C, 70.24; H, 6.10; N, 8.49.

Step D: Preparation of 3-(2-phthalimidoethyl)-5-ethyl-6-methyl-2(1H)-pyridinone

2-Methoxy-3-(2-phthalimidoethyl)-5-ethyl-6-methyl pyridine was heated at 150°C for 5-10 minutes in the presence of excess pyridine hydrochloride to give the above titled product.

EXAMPLE 17

3-{[(N-Phthalimido)methyl]amino}-5-butyl-6-methyl-2(1H)-pyridinone

Step A: Preparation of 3-cyano-5-butyl-6-methyl-2(1H)-pyridinone

According to the method described in J. Heterocyclic Chem., 24, 351 (1987), a mixture of crude sodium 2-butyl-3-oxobutanal (7.39 g), cyanoacetamide (4.15 g, 39 mmol), aqueous piperidinum acetate (3.5 mL) [prepared from glacial acetic acid (.85 ml), water (2.0 mL) and piperidine (1.45 ml)] in water (32 mL) was refluxed for 3 hours. Acetic acid (2.5 mL) was cautiously added. After cooling to room temperature and stirring overnight, the precipitated solids were collected by filtration. These residues (2.87 g) were chromatographed on silica gel by gradient elution with 1-3% methanol/ethyl acetate. The appropriate fractions were combined and evaporated. Crystallization from methanol gave 1.01 g of pure product.

Step B: Preparation of 5-butyl-6-methyl-2(1H)-pyridinone

A suspension of 3-cyano-5-butyl-6-methyl-2(1H)-pyridinone (950 mg, 5.0 mmol) in 6N hydrochloric acid (36 mL) was refluxed for 3 days. The reaction was cooled and extracted with methylene chloride to isolate product. It was then dried with sodium sulfate, filtered and evaporated. The residue was triturated with diethyl ether to yield product (563 mg, 68%).

Step C: Preparation of 3-nitro-5-butyl-6-methyl-2(1H)-pyridinone

A solution of 5-butyl-6-methyl-2(1H)-pyridinone (562 mg, 3.40 mmol) in concentrated sulfuric acid (4.3 mL) was cooled in an ice bath and 70% nitric acid (0.4 mL) was added dropwise. After one hour, the reaction mixture was poured into ice/water and the yellow product extracted into methylene chloride. This solution was dried, filtered and evaporated. The residue (468 mg) was chromatographed on silica gel eluting with 1-2% methanol/chloroform to yield 293 mg (41%) of pure product.

Step D: Preparation of 3-amino-5-butyl-6-methyl-2(1H)-pyridinone

A solution of 3-nitro-5-butyl-6-methyl-2(1H)-pyridinone (293 mg, 1.40 mmol) in methanol (10 mL) and tetrahydrofuran (10 mL) containing 5% palladium/carbon (120 mg) was hydrogenated at atmospheric pressure for 10-20 hours. The catalyst was filtered off and the solvent evaporated. This residue was triturated with diethyl ether to give 224 mg (89%) of grayish product.

Step E: Preparation of 3-{[N-Phthalimidomethyl]-amino)-5-butyl-6-methyl-2(1H)-pyridinone

A mixture of 3-amino-5-butyl-6-methyl-2-(1H)-pyridinone (90 mg, 0.5 mmol) and N-(hydroxymethyl)phthalimide (143 mg, 0.8 mmol) in ethanol (3 mL) was refluxed for 7 hours. Upon cooling the product crystallized out and was filtered off to yield 125 mg (73%) of product. This material was crystallized from ethanol to give 52 mg of pure product, m.p. 209-210°C.

Anal. Calcd. for $C_{19}H_{21}N_3O_3$:
C, 67.24; H, 6.24; N, 12.38.
Found: C, 66.98; H, 6.17; N, 12.12.

EXAMPLE 18

Preparation of 3-{[(2-Benzoxazolyl)methyl]amino}-5-methylthio-6-methyl-2(1H)-pyridinone and 3-{[(2-Benzoxazolyl)methyl]amino}-5-ethylthio-6-methyl-2(1H)-pyridinone

Step A: Preparation of 6-Methyl-5-methylthio-3-nitro-2(1H)-pyridinone

To a mixture of the sodium salt of 2-methylthio-3-oxo-1-butanal (1.54 g, 0.010 mol) [prepared according to procedure in J. Org. Chem 49, 3494 (1984)] and 2-nitroacetamide (1.10 g (0.0105 mol) in water (10 mL) was added aqueous 3.4M piperidinium acetate (0.71 mL). After stirring overnight at room temperature the yellow solids were removed by filtration, followed by washing with water. A yield of 0.874 g (43.7%) of analytically pure yellow solid, m.p. 199-203°C was obtained.

Anal. Calcd. for $C_7H_8N_2O_3S$:
C, 41.99; H, 4.03, N, 13.99
Found: C, 41.82; H, 3.92; N, 14.00.

The corresponding 6-methyl-5-ethylthio-3-nitro-2(1H)-pyridinone, prepared in a similar way, but starting with the sodium salt of 2-ethylthio-3-oxo-1-butanol had m.p. 190-191°C. (Yield 43.6%).

Step B: Preparation of 3-{[(2-Benzoxazolyl)methyl]-amino)-5-methylthio-6-methyl-2(1H)-pyridinone

To a mixture of 5-methylthio-6-methyl-3-nitro-2(1H)-pyridinone (0.60 g, 0.003 mol) in MeOH (30 mL) at room temperature was added an aqueous solution of sodium hydrosulfite ($Na_2S_2O_4$) (1.74 g, 0.01 mol). When the yellow color disappeared after addition of additional sodium hydrosulfite (1.7 g), the reaction mixture was extracted with $CHCl_3$ (2 x 200 mL). The $CHCl_3$ extracts were washed with saturated aqueous $NaHCO_3$ solution,

brine and then dried ($MgSO_4$). On concentration under vaccum there was obtained 0.20 g (39.4 % yield) of 3-amino-5-methylthio-6-methyl-2(1H)-pyridinone as a light yellow solid which was used without further purification.

A mixture of 3-amino-5-methylthio-6-methyl-2(1H)-pyridinone (0.201 g, 0.0013 mol), 2-chloromethylbenzoxazole (0.239 g, 0.0014 mol), diisopropylethylamine (0.181g , 0.0014 mol), and acetonitrile (6 mL) was heated at reflux under nitrogen for 35 hours. The dark solids which had formed were removed by filtration (0.089 g), and the filtrate evaported to dryness. The two fractions were combined and chromatographed on a 30 mm column containing 230-400 mesh silica gel. The column was prepared with methylene chloride and the crude product applied in methylene chloride. Development of the column was with 2% 2-propanol/methylene chloride (500 mL), 5% 2-propanol/methylene chloride (500 mL), and 10% 2-propanol/methylene chloride (1000 mL). Concentration of the fractions containing the desired product gave 0.133 g (33.5% yield) of 3-[((2-benzoxazolyl)methyl)amino]-5-methylthio-6-methyl-2(1H)-pyridinone m.p. 180-181°C.

Anal. Calcd for $C_{15}H_{15}N_3O_2S{\cdot}0.2H_2O$
C, 59.08; H, 5.09; N, 13.78.
Found: C, 59.27; H, 5.03; N, 13.75.

The corresponding 5-ethylthio analog was prepared in the same way except that bis-1,8-dimethylaminonaphthalene was used as base in the alkylation step; m.p. 197-200°C, (34.9% yield).

Anal. Calcd for $C_{16}H_{17}N_3O_2S$:
C, 60.93; H, 5.43; N, 13.32.
Found: C, 60.95; H, 5.46; N, 13.26.

EXAMPLE 19

Preparation of 3-{N-[2-(4,7-dichlorobenzoxazolyl) methyl]-amino}-5-ethyl-6-methyl-2(1H)-pyridinone

Step A: Preparation of 2-amino-3,6-dichlorophenol

A yellow solution of 2,5-dichloro-6-nitrophenol (10.0 g 48.0 mmol) in ethanol (200 mL) and acetic acid (13.8 mL) at 0°C was catalytically reduced in the presence of 5% platinum on charcoal (0.15 g) under an atmosphere of hydrogen (25 psi) for 1 hour in a Parr hydrogenator. The resultant colorless solution was filtered and concentrated under reduced pressure (15 torr). The residue was then dried under high vacuum (0.02 torr) overnight to yield 8.52 g (100%) of 2-amino-3,6-dichlorophenol.

Step B: Preparation of 2-chloromethyl-4,7-dichlorobenzoxazole

To a solution of 2-amino-3,6-dichlorophenol (23.91 g, 134 mmol) in methylene chloride (270 mL), solid ethyl chloroiminoacetate hydrogen chloride (31.9 g, 202 mmol) was added. The resultant slurry was stirred at room temperature overnight, then filtered through a plug of Celite, and concentrated under reduced pressure (15 torr). The solid residue was subjected to column chromatography on silica gel (elution with chloroform). Collection and concentration of appropriate fractions yielded 26.6 g (86%) of 2-chloromethyl-4,7-dichlorobenzoxazole.

Step C: Preparation of 3-{N-[2-(4,7-dichlorobenzoxazolyl)methyl]amino}-5-ethyl-6-methyl-2(1H)-pyridinone

A mixture of 3-amino-5-ethyl-6-methylpyridine-2(2H)-one (0.93 g, 6.1 mmol), 2-chloromethyl-4,7-dichlorobenzoxazole (1.45 g, 6.1 mmol), diisopropylethylamine (1.06 mL, 6.1 mmol) in acetonitrile (30 mL) was reluxed under an atmosphere of nitrogen for 20 hours. The resultant mixture was cooled to 0°C. The solid which precipitated was filtered and subjected to column chromatography on silica gel (elution with 4% methanol in chloroform). Collection and concentration of appropriate fractions provided 0.76 g of a white solid which was then recrystallized from ethanol to yield 0.66 g (31%) of 3-{N-[2-(4,7-dichlorobenzoxazolyl)methyl]amino}-5-ethyl-6-methyl-2(1H)-pyridinone, m.p. 198.0-198.5°C.

Anal. Calcd. for $C_{16}H_{15}C_{12}N_3O_2$:
C, 54.56; H, 4.29; N, 11.93
Found C, 54.43; H, 4.12; N, 11.89

EXAMPLE 20

Preparation of 3-[N-(5-ethyl-2-methoxy-6-methyl- 3-pyridylmethyl)amino]-5-ethyl-6-methyl-2(1H)-pyridinone

A solution of 3-amino-5-ethyl-6-methyl-2(1H)-pyridinone (12.66 g, 83.2 mmol, see Example 1, Step B), 5-ethyl-2-methoxy-6-methylnicotinaldehyde (15.0 g, 83.2 mmol, see Example 4, Step F), and acetic acid (5 drops in methanol (83 mL) was stirred at room temperature for 18 hours under an atmosphere of nitrogen. The yellow-orange precipitate was filtered, washed with a small amount of methanol, and then dissolved in a mixture of methanol and chloroform (3:1 v/v) with warming. The resultant solution was allowed to cool back to room temperature and sodium cyanoborohydride was added until all the Schiff base was reduced. The product solution was then concentrated under vacuum. Water and chloroform were added to the residue. The aqueous layer was separated and extracted three more times with chloroform. The organic extracts were combined, dried over anhydrous sodium sulfate, filtered and concentrated under vacuum. The residue was then subjected to column chromatography on silica gel and eluted with 5% methanol in chloroform. Collection and concentration of appropriate fraction, followed by recrystallization (ethanol) yielded 10.5 g (40%) of the title compound, 165-167°C.

Anal. Calcd. for $C_{18}H_{25}N_3O_2$ :
C, 68.54; H, 7.99; N, 13.32.
Found C, 69.17; H, 7.99; N, 13.36%.

EXAMPLE 21

3-[N-(2,6-Dimethoxybenzyl)amino]-5-ethyl-6-methyl-2,(1H)-pyridinone

3-Amino-5-ethyl-6-methyl-2(1H)-pyridinone (152 mg, 1.00 mmol) and 2,6-dimethoxybenzaldehyde (168 mg, 1.00 mmol) were dissolved in methanol (5 mL). Two drops of glacial acetic acid were added and the solution stirred for 2 hours. Sodium borohydride (378 mg, 10 mmol) was added portionwise to the suspension and the reaction stirred for 30 minutes at room temperature. The mixture was diluted with water and extracted with ethyl acetate. The ethyl acetate was washed with water, saturated brine, and dried over magnesium sulfate. The drying agent was filtered and the filtrate concentrated in vacuo to give a white solid (290 mg, 96% yield). This solid was recrystallized from ethanol to give the title compound as white fluffy needles (140 mg) mp 220-222°C.

Anal. Calcd. for $C_{17}H_{22}N_2O_3$:
C, 67.52; H, 7.34; N, 9.26.
Found C, 67.69; H, 7.50; N, 9.18.

$^1$H NMR (DMSO-$d_6$, 300 MHz): δ 11.1 (1H, s), 7.20 (1H, t, J=8 Hz), 6.65 (2H, d, J=8 Hz), 6.30 (1H, s) 4.80 (1H, s), 4.16 (2H, s), 3.80 (6H, s) 2.26 (2H, q, J=7 Hz), 2.00 (3H, s), 1.00 (3H, t, J=7 Hz).

EXAMPLE 22

3-[N-(5-Ethyl-2-methoxybenzyl)amino]-5-ethyl-6-methyl-2(1H)-pyridinone

Step A: 4-Ethylanisole

To a suspension of powdered KOH (9.18 g, 0.16 mol) in DMSO (80 mL) was added 4-ethylphenol (500 g, 0.041 mol) followed by iodomethane (5.0 mL, 0.081 mol). The reaction was stirred for 10 minutes, poured into water (800 mL), and extracted with benzene (3 X 200 mL). The organic extracts were washed with water, saturated brine and dried over sodium sulfate. Filtration and concentration of the filtrate in vacuo gave the crude product (6.21 g) which was chromatographed on silica gel with 5% ethyl acetate in hexane. The title compound was obtained as a clear oil (4.67 g, 84% yield).

$^1$H NMR ($CDCl_3$, 300 mHz): δ 7.38 (1H, s), 7.11 (1H, d, J=7 Hz), 6.83 (1H, d, J=7 1z), 3.79 (3H, s), 3.58 (2H, q, J=7 Hz), 1.21 (3H, t, J=7 Hz).

Step B: 5-Ethyl-2-methoxybenzaldehyde

A solution of 4-ethylanisole (4.54 g, 0.033 mol) in dry ether (45 mL) was cooled to 0°C under a nitrogen atmoshpere. To this solution was added n-butyllithium (13.3 mL of a 2.5 M solution in hexane) via syringe and the reaction stirred at room temperature overnight. The reaction was cooled to -78°C and DMF (5.17 mL, 0.066 mol) (dry, distilled) was added. The reaction was stirred at -78°C for 5 minutes, 0°C for 25 minutes and then

10% HCL added. The layers were separated and the organic phase washed with water, saturated brine and dried over sodium sulfate. The crude product was obtained as a oil (5.56 g). Of this material 4.35 g was chromatographed on silica gel with 5% ethyl acetate in hexane. The title compound was obtained as a clear oil (2.18 g, 51% yield).
$^1$H NMR ($CDCl_3$3, 300 MHz): δ 10.45 (1H, s), 7.66 (1H, d, J=2 Hz), 7.39,(1H, dd, J=2, 8 Hz), 6.92 (1H, d, J=8 Hz), 3.91 (3H, s), 2.62 (2H, q, J=7 Hz), 1.22 (3H, t, J=7 Hz).

Step C. 3-[N-(5-Ethyl-2-methoxybenzyl)amino]-5-ethyl-6-methyl-2(1H)-pyridinone

5-Ethyl-2-methoxybenzaldehyde (0.561 g, 3.42 mmol) and 3-amino-5-ethyl-6-methyl-2(1H)-pyridinone (0.520 g, 3.42 mmol) were reacted according to the procedure described for the preparation of 3-[N-(2,6-dimethoxybenzyl)amino]-5-ethyl-6-methyl-2(1H)-pyridinone. The crude product was recrystallized from ethanol to give the title compound as pale yellow prisms, mp 149-152°C (0.370 g).

Anal. Calcd. for $C_{18}H_{24}N_2O_2$:
C, 71.71; H, 8.05; N, 9.32.
Found C, 71.93; H, 8.05; N, 9.18.
$^1$H NMR ($CDCl_3$, 300 MHz): δ 11.33 (1H, br s), 7.13 (1H, d, J=2 Hz), 7.06 (1H, dd, J=2, 8 Hz), 6.80 (1H, d, J=8 Hz), 6.21 (1H, s), 5.08 (1H, br, m), 4.28 (2H, d, J=6 Hz), 3.83 (3H, s) 2.55 (2H, q, J=7 Hz), 2.32 (2H, q J=7 Hz), 2.19 (3H, s), 1.16 (3H, t, J=7 Hz), 1.05 (3H, t, J=7 Hz).

EXAMPLE 23

3-[N-(2-methoxy-4,5-dimethylbenzyl)amino]-5-ethyl-6-methyl-2(1H)-pyridinone

Step A. 4,5-Dimethyl-2-methoxybenzaldehyde

A solution of 3,4-dimethylanisole (9.74 g, 0.071 mol) in dry ether (150 mL) was cooled under nitrogen to 0°C. To this stirred solution was added n-butyllithium (28.6 mL 2.5 M in hexane) via syringe. The reaction was stirred at 0°C for 1.5 hours then at room temperature for 16 hours. The reaction was cooled to 0°C and DMF (11.0 mL, 2 eq.) was added. The reaction was stirred at 0°C for 1.5 hours, diluted with 10% aqueous HCl and the layers separated. The ether solution was washed with water, saturated brine, and dried over magnesium sulfate. The drying agent was filtered and the solvent removed in vacuo to give two products Rf = 0.37 and Rf = 0.25 with 5% ethyl acetate in hexane on a silica gel tlc plate. The crude products were separated on silica gel with 5% ethyl acetate in hexane. The Rf = 0.37 material was isolated as a clear oil which solidified on standing and was identified as 5,6-dimethyl-2-methoxybenzaldehyde (1.4 g, 11% yield). The Rf = 0.25 material was the title compound and was obtained as a white solid, mp 63-66°C (3.6 g, 30% yield)
$^1$H NMR (300 MHz, $CDCl_3$): 6 10.38 (1H, s), 7.57 (1H, s), 6.76 (1H, s), 3.89 (3H, s), 2.32 (3H, s), 2.22 (3H, s).

Step B. 3-[N-(2-methoxy-4,5-dimethylbenzyl)amino]-5-ethyl=6-methyl-2(1H)-pyridinone

4,5-Dimethyl-2-methoxybenzaldehyde (240 mg, 1.45 mmol) and 3-amino-5-ethyl-6-methyl-2(1H)-pyridinone (220 mg, 1.45 mmol) were reacted according to the procedure described for the preparation of 3[N-(2,6-dimethoxybenzyl)]-amino-5-ethyl-6-methyl-2(1H)-pyridinone. The crude product (426 mg, 97% yield) was recrystallized from methanol (25 mL) to give the title compound as a pale yellow solid (273 mg) mp 175.5-177°C.
$^1$H NMR (300 MHz, DMSO-$d_6$): δ 11.1 (1H, br, s), 6.97 (1H, s), 6.78 (1H, s), 6.08 (1H, s), 5.3 (br, s, 1H), 4.10 (2H, s), 3.77 (3H, s), 2.22 (2H, q, J=7.6 Hz), 2.18 (3H, s), 2.09 (3H,s), 2.01 (3H, s) 0.97 (3H, t, J=7.5 Hz).

EXAMPLE 24

3-{[(4,7-Dichlorobenzoxazol-2-yl)methyl]amino}-5-methylthio-6-methylpyridin-2(1H)-one

This compound was prepared by the procedure of Example 18, Step B, except that 1,8-bis(dimethylamino)naphthalene was employed as base in place of diisopropylethylamine and 2-chloromethyl-4,7-dichlorobenzoxazole in place of 2-chloromethylbenzoxazole. The compound has mp 254-257°C.

Anal. Calcd. for $C_{15}H_{13}C_{12}N_3O_2S$:
C, 48.66; H, 3.54; N, 11.35.
Found: C, 48.67; H, 3.43; N, 11.37.
$^1$H NMR ($CDCl_3$) δ 2.29 (s, 3H), 2.41 (s, 3H), 4.86 (d, 2H, J=4 Hz), 5.45 (s, 1H), 6.74 (s, 1H), 7.27 (d, 1H, J=7.2

Hz).

EXAMPLE 25

3-{[(4,7-Dichlorobenzoxazol-2-yl)methyl]thio}-5-ethyl-6-methylpyridin-2(1H)-one

Step A: 2-Methoxy-3-ethoxythiocarbonylthio-5-ethyl-6-methylpyridine

To a solution of 2-methoxy-3-amino-5-ethyl-6-methylpyridine (Example 9, Step C)(1.24 g, 0.0075 mol) in water (1.9 ml) plus conc. HCl (1.9 ml), cooled in an ice-acetone bath, was added dropwise a cold solution of $NaNO_2$ (0.55 g, 0.0077 mol) in water (3 ml). The mixture was stirred for 20 minutes. The cold reaction mixture was added dropwise to potassium ethyl xanthate (1.4 g, 0.0088 mol) in water (1.8 ml) heated to 40-45°C. The mixture was stirred for 24 minutes and then extracted three times with chloroform. The chloroform extract was washed with saturated aqueous sodium bicarbonate solution, brine and then dried ($MgSO_4$). Evaporation gave a crude residue (1.47 g) which, after combining with 0.17 g of product from a previous probe run, was chromatographed on a 50 mm diameter column containing 6 inches of 230-400 mesh silica gel. The column was prepared with, and the sample applied in, hexane. Development was done with 5% $CH_2Cl_2$-hexane, followed by increasing the $CH_2Cl_2$ content to 10% and then 20%. The desired intermediate (0.34 g) was obtained as an oil (FAB-MS showed an $M+H^+$=272).

Step B: 2-Methoxy-3-mercapto-5-ethyl-6-methylpyridine

A mixture of 2-methoxy-3-ethoxythiocarbonylthio-5-ethyl-6-methylpyridine (0.30 g, 0.0011 mol), 1N NaOH (3.0 mL) and ethanol (9 mL) was stirred under nitrogen for 9.5 hours. 1N HCl was added to bring the pH to 3 and the mixture evaporated to dryness. The residue was extracted with $CH_2Cl_2$ to give 0.21 g of crude product which was utilized without purification in the next step. (Rf=0.2, 30% $CH_2Cl_2$-hexane, silica gel plate).

Step C: 2-Methoxy-3-{[(4,7-dichlorobenzoxazol-2-yl)-methyl]thio)-5-ethyl-6-methylpyridine

A mixture of 2-methoxy-3-mercapto-5-ethyl-6-methylpyridine (0.20 g, 0.00011 mol), 2-(2-chloromethyl)-4,7-dichlorobenzoxazole (0.26 g, 0.0011 mol) and diisopropylethylamine (0.16 g, 0.0012 mol) in $CHCl_3$ (5 mL) was allowed to react at room temperature under nitrogen for 3.5 hours. After washing with water, drying, and evaporating there was obtained 0.43 g of crude product which was chromatographed using a 30 mm column containing 5 inches of 230-400 mesh silica gel. The product (0.35 g), which was eluted with 30-70 $CH_2Cl_2$-hexane, had mp 105-110°C.

Anal. Calcd for $C_{17}H_{16}Cl_2N_2O_2S$ 0.1 $CH_2Cl_2$
C, 52.42; H, 4.17; N, 7.15.
Found: C, 52.48; H, 4.10, N, 7.05.

Step D: 3-{[(4,7-Dichlorobenzoxazol-2-yl)methyl]-thio)-5-ethyl-6-methylpyridin-2(1H)-one

A mixture of 2-methoxy-3-{[(4,7-dichlorobenzoxazol-2-yl)methyl]thio}-5-ethyl-6-methylpyridine (0.22 g, 0.0012 mol) and potassium iodide (0.191 g, (0.0012 mol) in acetic acid (10 mL) was heated at 70°C for 7 hours. Chloroform was then added and the dark solution washed with aqueous sodium thiosulfate, followed by water and brine. Evaporation gave crude product (0.21 g). After digestion with boiling acetonitrile and cooling, there was obtained 0.18 g (83% yield) of product mp 233-235°C; FAB-MS 369 ($M+H^+$).

Anal. Calcd for $C_{16}H_{14}C_{12}N_2O_2S$. 0.05 $CHCl_3$
C, 51.37; H, 3.77; N, 7.46.
Found: C, 51.42; H, 3.69; N, 7.37.

$^1H$ NMR (DMSO-$d_6$) δ 0.96 (t, 3H, J=7.5 Hz), 2.14 (s, 3H), 2.28 (q, 2H, J=7.5 Hz), 4.54 (s, 2H), 7.45 (s, 1H), 7.48 (d, 1H, J=8.7), 7.48 (d, 1H, J=8.7 Hz), 11.73 (s, 1H).

EXAMPLE 26

1-Methyl-4-{[(4,7-dichlorobenzoxazol-2-yl)methyl]-amino}-5,6,7,8_tetrahydroisoquinolin-3(2H)-one

Step A: 1-Methyl-4-cyano-5,6,7,8-tetrahydroisoquinolin-3(2H)-one

A mixture of 2-acetylcyclohexanone (2.80 g, 0.02 mol), 2-cyanoacetamide (1.68 g, 0.02 mol), and diethylamine (0.8 mL) in ethanol (40 mL) was heated at 50°C overnight. After cooling to room temperature the solid product, a mixture of two regioisomers, was removed by filtration (2.48 g, 66% yield). Recrystallization from hot dimethylformamide gave nearly pure desired isomer, mp >319°C.
$^1H$ NMR (DMSO-$d_6$) δ 1.67 (m, 4H), 2.21 (s, 3H), 2.37 (m, 2H), 2.72 (m, 2H).
Anal. Calcd for $C_{11}H_{12}N_2O$:
C, 70.19; H, 6.43; N, 1.4.88
Found: C, 70.10; H, 6.41; N, 14.77.

Step B: 1-Methyl-4-nitro-5,6,7,8-tetrahydroisoquinolin-3(2H)-one

A mixture of I-methyl-4-cyano-5,6,7,8-tetrahydroisoquinolin-3(2H)-one (0.50 g, 0.0027 mol) and conc Hcl (4 ml) was heated in a sealed tube at 150°C overnight. On cooling, the slurry was evaporated to dryness. Water was added to the residue and the mixture made basic with conc $NH_4OH$. A grey solid (0.39 g) was obtained, mp 207-220°C. Nitration of this intermediate was accomplished by dissolving in conc $H_2SO_4$, (2.65 mL), and, after cooling in an ice-acetone bath, adding dropwise 70% nitric acid (0.23 mL). After stirring for one hour the reaction was quenched with ice-water and the solids collected (0.23 g, 50% yield). FAB-MS 209 ($M+H^+$).

Step C: 1-Methyl-4-{[(4,7-dichlorobenzoxazol-2-yl)-methyl]amino}-5,6,7,8-tetrahydroisoquinolin-3(2H)-one

The product from Step B in ethanol (60 mL) to which was added 10% palladium on charcoal (0.043 g) was hydrogenated under an atomsphere of hydrogen gas for 6 hours. The mixture was filtered through filter-aid and the filtrate evaporated to a solid residue. To the residue was added acetonitrile (6 mL), 2-chloromethyl-4,7-dichlorobenzoxazole (0.33 g, 0.0014 mol), and 1,8-bis(dimethylamino)naphthalene (0.30 g, 0.0014 mol). The mixture was refluxed for 36 hours under nitrogen. On cooling, the solids were removed by filtration and washed with acetonitrile to give a first crop of 0.226 g. Recrystallization of a portion (0.11 g) gave pure product (0.07 g) mp 243-248°C.
Anal Calcd for $C_{18}H_{17}Cl_2N_3O_2$:
C, 57.16; H, 4.53; N, 11.11.
Found: C, 56.81; H, 4.52; N, 11.15.

EXAMPLE 27

6-Methyl-5-ethyl-3-{[(5-methyl-4-ethoxycarbonyl-2-furanyl)methyl]amino}pyridin-2(1H)-one

A mixture of 6-methyl-5-ethyl-3-aminopyridin-2(1H)-one (100 mg, 0.66 mmol) and 5-methyl-4-ethoxycarbonyl-2-furancarboxaldehyde (128 mg, 0.7 mmol) in 5 ml of ethanol was stirred at room temperature until a solution was obtained. To this solution was added three drops of acetic acid followed by a slight excess of sodium cyanoborohydride. After one hour the reaction was found to be complete by TLC analysis. Water (30 ml) was added and a solid separated which was recovered by filtration and dried. The yield was 125 mg (60%) of pale yellow solid. Crystallization from butyl chloride afforded pure material, m.p. 130-132°C.
Anal. Calcd for $C_{17}H_{22}N_2O_4$:
C, 64.13; H, 6.97; N, 8.80.
Found: C, 64.12; H, 6.97; N, 8.82

EXAMPLE 28

3-[2-(Benzo[b]furan-2-yl)ethyl]-5-ethyl-6-methylpyridin-2(1H)-one

Step A: 2-[2(R/S)-Hydroxy-2-(2-methoxy-5-ethyl-6-methyl-pyridin-3-yl)ethyl]-benzo[b]furan

To a solution of 2-methylbenzo[b]furan (264 mg, 2.0 mmole) in THF (5.0 ml) cooled to -78°C was added

1.6 M n-butyllithium in hexane (1.313 ml, 2.1 mmole) dropwise. The reaction was stirred at -78°C for four hours. To the above solution was added 2-methoxy-5-ethyl-6-methylnicotinaldehyde in THF (2.0 ml). The reaction was allowed to warm to room temperature and poured onto crushed ice/water. The mixture was extracted with diethyl ether, dried ($MgSO_4$), and the solvent removed to yield 503 mg of a semi solid which was flash chromatographed over silica gel. Elution with 5% ethylacetate-95% hexane yielded 226 mg of an oil (36%).

Step B: 3-[2-(Benzo[b]furan-2-yl)ethenyl]-5-ethyl-6-methylpyridin-2(1H)-one

2-[2(R/S)-Hydroxy-2-(2-methoxy-5-ethyl-6-methyl-pyridin-3-yl)ethyl]-benzo[b]furan (175mg, 0.6 mmole) was mixed with solid pyridine hydrochloride (1.0 gm, 8.7 mmole) and placed into a preheated (140°C) oil bath for five minutes under argon. The reaction was cooled to room temperature and treated with water. The resulting mixture was extracted with chloroform. The combined extract was dried ($MgSO_4$) and solvent removed to yield 175 mg of crude material. Proton NMR indicates that this is essentially the trans isomer.

Step C: 3-[2-(Benzo[b]furan-2-yl)ethyl]-5-ethyl-6-methyl-pyridin-2(1H)-one

A solution of crude 3-[2-(benzo[b]furan-2-yl)ethenyl]-5-ethyl-6-methyl-pyridin-2(1H)-one (175 mg) in THF-/EtOH (20 ml/10ml) was hydrogenated over (5%) palladium on charcoal (excess) for four hours. After filtering and concentrating under reduced pressure the residue was flash chromatographed over silica-gel. Elution with 1% MeOH-99% $CHCl_3$ gave 50 mg of analytically pure product mp 120-122°C.

Anal. Calcd for $C_{18}H_{19}NO_2$ .05 $CHCl_3$
C, 75.74; H, 6.71; N, 4.79.
Found: C, 75.74; H, 6.71; N, 4.79.

$^1H$ NMR (300 MHz, $CDCl_3$): δ 7.47-7.39 (m, 2H); 7.22-7.14 (m, 2H); 7.05 (s, 1H); 6.38 (d, 1H, J=.78 Hz); 3.11 (t, 2H, J=7.93 Hz); 2.95(t, 2H, J=7.57); 2.30(q, 2H, J=7.57); 2.25(s, 3H); .99(t, 3H, J=7.46).

EXAMPLE 29

3-[(4,7-Dichlorobenzoxazol-2-yl)methoxy]-5-ethyl-6-methylpyridin-2(1H)-one

Step A: Preparation of 2-methoxy-3-hydroxy-5-ethyl-6-methylpyridine

3-Chloroperbenzoic acid (80% pure, 1.42 g, 6.6 mmol) was added in portions to a solution of 2-methoxy-5-ethyl-6-methylnicotinaldehyde (1.08 g, 6.0 mmol) in methylene chloride (15 mL). Diethyl ether (5 mL) was added to dissolve the formed precipitate and stirring continued for 1.5 hours. The reaction was diluted with methylene chloride and washed with saturated aqueous sodium bicarbonate. The dried organic layer ($Na_2SO_4$) was filtered and evaporated. The residue was suspended in 50% aqueous acetic acid and warmed at 80°C for one hour. This reaction was poured into water and the product extracted into diethyl ether. The ether extract was washed with aq. $NaHCO_3$, dried ($Na_2SO_4$) and evaporated. This residue (924 mg) was chromatographed on silica gel, eluting with chloroform to give 510 mg (50% yield) of oily product.

Step B: Preparation of 3-[(4,7-dichlorobenzoxazol-2-yl)methoxy]-2-methoxy-5-ethyl-6-methylpyridine

Sodium hydride/mineral oil (60%, 59 mg, 1.5 mmol) was added to a solution of 2-methoxy-3-hydroxy-5-ethyl-6-methylpyridine (60% pure, 275 mg, 1.0 mmol) in dry dimethylformamide (3 mL) under a nitrogen atmosphere. After 1 hour 2-chloromethyl-4,7-dichlorobenzoxazole (236 mg, 1.0 mmol) was added to the reaction and stirred for 1 hour. The reaction was neutralized with dilute (10%) hydrochloric acid, made basic with sodium bicarbonate and the product was extracted into diethyl ether, the solution dried ($Na_2SO_4$) and evaporated. The residue (288 mg) was chromatographed on silica gel and eluted with 1% methanol/chloroform. The appropriate fractions were combined, solvents evaporated and the residue triturated with diethyl ether/hexane to give 84 mg (23% yield) of crystalline product.

Step C: Preparation of 3-[(4,7-dichlorobenzoxazol-2-yl)methoxy]-5-ethyl-6-methylpyridin-2(1H)-one

Boron tribromide/hexane (1M, 1 mL, 1.0 mmol) was added to a solution of 3-[(4,7-dichlorobenzoxazol-2-yl)methoxy]-2-methoxy-5-ethyl-6-methylpyridine (83 mg, 0.226 mmol) in anhydrous methylene chloride (3 mL) cooled in an ice bath under a nitrogen atmosphere. After 1 hour, saturated aqueous $NaHCO_3$ was added and the mixture stirred for 0.5 hours. The product was extracted into chloroform, dried ($Na_2SO_4$), and the solvent

evaporated to give a residue which was digested in warm methanol and then cooled. The collected precipitate was analytically pure, 24 mg (30% yield), mp: 243-245°C;
$^1H$ NMR (300 mHz, $CDCl_3$) δ 7.32 (2H, s), 7.15 (1H, s), 5.50 (2H, s), 2.40 (2H, AB q., J=7.6 Hz), 2.29 (3H, s), 1.11 (3H, t, J=7.6 Hz).

| | |
|---|---|
| Anal. | Calcd for $C_{16}H_{14}Cl_2N_2O_3 \cdot 0.15\ H_2O$ |
| | C, 53.99; H, 4.05; N, 7.87. |
| Found: | C, 53.96; H, 4.00; N, 7.83. |

EXAMPLE 30

3-[(2-Methoxy-5-ethyl-6-methylpyridin-3-yl)methoxy]-5-ethyl-6-methylpyridin-2(1H)-one

Step A: Preparation of 2-methoxy-3-hydroxymethyl-5-ethyl-6-methyl-pyridine

Solid 2-methoxy-5-ethyl-6-methylnicotinaldehyde (2.15 g, 12 mmol) was added in portions to a suspension of Lithium aluminum hydride (480 mg, 12.6 mmol) in dry tetrahydrofuran (20 mL) under a nitrogen atmosphere. The mixture was refluxed for 1.5 hours and then cooled to room temperature and saturated aqueous sodium sulfate was carefully added to the reaction. After stirring for 0.5 hours, solid anhydrous $Na_2SO_4$ was added and the salts were removed by filtration. This THF solution was evaporated and the residue chased with toluene. Upon trituration with cold hexane product crystallized out. This product (863 mg) was collected by filtration and the filtrate was chromatographed on silica gel, eluting with a 0-2% methanol/chloroform gradient to give, after trituration with cold hexane, an additional 712 mg for a combined yield of 72%, mp 53-54°C.

Step B: Preparation of 2-methoxy-3-[(2-methoxy-5-ethyl-6-methyl-pyridin-3-yl)methoxy]-5-ethyl-6-methyl-pyridine

Diethyl azodicarboxylate (0.15 mL, .93 mmol) was added dropwise to a solution of 2-methoxy-3-hydroxy-5-ethyl-6-methyl-pyridine (155 mg, 0.93 mmol), 2-methoxy-3-hydroxymethyl-5-ethyl-6-methylpyridine (168 mg, 0.93 mmol) and triphenyl phosphine (247 mg, 0.94 mmol) in dry tetrahydrofuran (10 mL) under a nitrogen atmosphere. This solution was stirred at room temperature overnight. The solvent was evaporate.d and the residue was chromatographed on silica gel with a 0-0.25% methanol/chloroform gradient to give 197 mg (64% yield) of solid product. An analytical sample was obtained by crystallization from cold hexane, mp 86-87°C.

| | |
|---|---|
| Anal. | Calcd for $C_{19}H_{26}N_2O_3$: |
| | C, 69.06; H, 7.93; N, 8.48. |
| Found: | C, 69.16; H, 8.10; N, 8.52. |

Step C: Preparation of 3-[(2-methoxy-5-ethyl-6-methylpyridin-3-yl)methoxy]-5-ethyl-6-methylpvridin-2(1H)-one

A solution of 2-methoxy-3-[(2-methoxy-5-ethyl-6-methylpyridin-3-yl)methoxy]-5-ethyl-6-methylpyridine (173 mg, 0.52 mmol) and potassium iodide (99 mg, 0.60 mmol) in glacial acetic acid (4.5 mL) was warmed at 50-60°C for two hours. The acetic acid was evaporated and the residue dissolved in methylene chloride and washed with water and aqueous $NaHCO_3$, dried ($Na_2SO_4$) and the solvent evaporated. This residue (139 mg) was chromatographed on silica gel and eluted with 0.5-1.5% methanol/chloroform gradient. The appropriate fractions were combined and evaporated and the residue triturated with diethyl ether to 69 mg (42% yield) of pure product, mp 213-214°C;
$^1H$ NMR (300 MHz, $CDCl_3$) δ 7.51 (1H s), 6.70 (1H, s), 5.08 (2H, s), 3.96 (3H, s), 2.53 (2H, ABq., J=7.6 Hz), 2.42 (3H, s), 2.33 (2H, ABq., J=7.6 Hz), 2.28 (3H, s) 1.13 (3H, t, J=7.6 Hz), 1.06 (3H, t, J=7.6 Hz).

| | |
|---|---|
| Anal. | Calcd for $C_{18}H_{24}N_2O_3$: |
| | C, 68.33; H, 7.65; N, 8.86. |
| Found: | C, 67.93; H, 7.60; N, 8.74. |

EXAMPLE 31

3-[(4,7-Dichlorobenzo[b]furan-2-yl)methoxy]-5-ethyl-6-methyl-pyridin-2(1H)-one

Step A: Preparation of 2-methoxy-3-[(4,7-dichlorobenzo[b]furan-2-yl)methoxy]-5-ethyl-6-methylpyridin-2(1H)-one

Sodium hydride/mineral oil (60%, 52 mg, 1.3 mmol) was added to a solution of 2-methoxy-3-hydroxy-5-ethyl-6-methylpyridine (169 mg, 1.0 mmol) in dry dimethylformamide (3 mL) at room temperature under a nitrogen atmosphere. After 15 minutes 2-chloromethyl-4,7-dichlorobenzo[b]furan (235 mg, 1.0 mmol) was added to the reaction. After three hours the reaction was diluted with diethyl ether, neutralized wtih dilute HCl and then basified with $NaHCO_3$. The ethereal extract was washed with water, filtered through a pad of charcoal and evaporated. The residue was triturated with cold hexane to 88 mg (24% yield) of pure product, mp 94-95°C.

Step B: Preparation of 3-[(4,7-dichlorobenzo[b]furan-2-yl)methoxy]-5-ethyl-6-methylpyridin-2(1H)-one

A mixture of 2-methoxy-3-[(4,7-dichlorobenzo-[b]furan-2-yl)methoxy]-5-ethyl-6-methylpyridine (85 mg, 0.23 mmol) and pyridine hydrochloride (458 mg, 4.0 mmol) was placed in a pre-heated oil bath at 150°C for 12 minutes. The cooled residue was triturated with water and the product was extracted into chloroform. This chloroform solution was washed with dilute HCl, dried ($Na_2SO_4$) and evaporated. This residue was triturated with diethyl ether to give 59 mg (72% yield) of product. Digestion in diethyl ether/methylene chloride gave 52 mg of analytical product, mp 209-210°C;

$^1$H NMR (300 MHz, $CDCl_3$) $\delta$ 7.20 (2H, ABq., J=8.4 Hz), 7.04 (1H, s), 6.94 (1H, s), 5.33 (2H, s), 2.41 (2H, ABq., J=7.6 Hz), 2.32 (3H, s), 1.11 (3H, t, J=7.6 Hz).

Anal. Calcd for $C_{17}H_{15}Cl_2NO_3$:
C, 57.97; H, 4.29; N, 3.98.
Found: C, 57.62; H, 4.19; N, 3.88.

EXAMPLE 32

3-[2-(4,7-Dichlorobenzo[b]furan-2-yl)ethyl]-5-ethyl-6-methyl-pyridin-2(1H)-one

Step A: Preparation of 2-[2-(2-methoxy-5-ethyl-6-methylpyridin-3-yl)ethenyl]-4,7-dichlorobenzo[b]furan

Sodium hydride/mineral oil (60%, 166 mg, 4.15 mmol) was added to a suspension of (4,7-dichlorobenzo[b]furan-2-yl)methyltriphenyl phosphonium chloride (1.74 g, 3.50 mmol)[prepared by heating a 1:1 mixture of triphenylphosphine and 2-chloromethyl-4,7-dichlorobenzo[b]furan in refluxing toluene for 20-25 hours] in distilled tetrahydrofuran (20 mL). After three hours, 2-methoxy-5-ethyl-6-methylnicotinaldehyde (631 mg, 3.52 mmol) was added to the deep yellow suspension and the mixture was refluxed for 5 hours. The cooled reaction was diluted with chloroform and water was added. The chloroform layer was dried ($Na_2SO_4$), filtered through a pad of charcoal and evaporated. This residue was digested with hot hexane and the insoluble triphenylphosphine oxide was filtered off to give nearly pure product (1.16 g). This material was triturated with cold hexane to give several crops of crystalline product (858 mg, 68% yield). The NMR indicated that essentially cis olefin was isolated (two doublets at 6.75 and 6.50, each J=12.7 Hz).

Step B: Preparation of 2-[2-(2-methoxy-5-ethyl-6-methylpyridin-3-yl)ethyl]-4,7-dichlorobenzo[b]furan

A solution of 2-[2-(2-methoxy-5-ethyl-6-methylpyridin-3-yl)ethenyl]-4,7-dich1orobenzo[b]-furan (561 mg, 1.54 mmol) in methanol (7 mL) and tetrahydrofuran (7 mL) containing a few drops of acetic acid and 5% palladium/charcoal (50 mg) was hydrogenated at atmospheric pressure for two hours. The catalyst was removed by filtration and the solvent evaporated to give essentially pure product as an oil (572 mg, 99%).

Step C: Preparation of 3-[2-(4,7-dichlorobenzo[b]-furan-2-yl)ethyl]-5-ethyl-6-methylpyridin-2(1H)-one

A mixture of 2-[2-(2-methoxy-5-ethyl-6-methylpyridin-3-yl)ethyl]-4,7-dichlorobenzo[b]furan (572 mg, 1.54 mmol) and pyridine hydrochloride (1.87 mg, 15.8 mmol) was placed in a pre-heated oil bath at 150°C for 15 minutes. The cooled mixture was diluted with water and the product was extracted into chloroform. The chloroform extract was washed with dilute HCl, dried ($Na_2SO_4$) and evaporated. This residue (570 mg) was

chromatographed on silica gel eluting with 0-2% methanol/chloroform gradient to give recovered starting material (140 mg) and purified product (377 mg, 91% yield based on recovered starting material). Analytically pure product was obtained by digestion of solid in diethyl ether to give 241 mg,-mp 153-154°C;
$^1$H NMR (300 MHz, $CDCl_3$) δ 7.11 (2H, ABq. J=8.4 Hz), 7.09 (1H, s), 6.53 (1H, s), 3.18 (2H, t, J=7.6 Hz), 2.98 (2H, t, J=7.6 Hz), 2.33 (2H, ABq., J=7.7 Hz), 2.29 (3H, s), 1.02 (3H, t, J=7.7 Hz).

Anal. Calcd for $C_{18}H_{17}Cl_2NO_2$:
C, 61.73; H, 4.89; N, 4.00.
Found: C, 62.12; H, 4.93; N, 3.94.

EXAMPLE 33

3-[2-(2-Methoxy-5-ethyl-6-methylpyridin-3-yl)ethyl]-5-ethyl-6-methylpvridin-2(1H)-one

Step A: Preparation of 3-[2-(2-methoxy-5-ethyl-6-methylpyridin-3-yl)ethenyl]-5-ethyl-6-methylpyridin-2(1H)-one

A mixture of 2-methoxy-3-hydroxymethyl-5-ethyl-6-methylpyridine (363 mg, 2.0 mmol) and triphenylphosphine hydrobromide (690 mg, 2.0 mmol) in acetic acid (3 mL) was refluxed for 24 hours. The reaction was cooled and diluted with diethyl ether to give a gummy precipitate. This gummy residue was dissolved in chloroform, dried ($Na_2SO_4$) and the solvent evaporated to give a glassy residue which by NMR is crude (5-ethyl-6-methylpyridin-2(1H)-on-3-yl)methyltriphenylphosphonium bromide and by-product methyltriphenylphosphonium bromide.

This glassy residue (815 mg) was suspended in dry tetrahydrofuran (10 mL) and stirred for 1.5 hours as the residue became a fine suspension. Sodium hydride/mineral oil (60%, 95 mg, 2.37 mmol) was added and the mixture was warmed at 50°C for I5 minutes before 2-methoxy-5-ethyl-6-methylnicotinaldehyde (323 mg, 1.8 mmol) was added. The reaction was refluxed for 5 hours. The reaction was cooled and diluted with chloroform and water. The chloroform layer was separated off, dried ($Na_2SO_4$) and evaporated to give a crude residue (960 mg) which was chromatographed on silica gel eluting with 0-2% methanol/chloroform gradient to give 106 mg (19% yield) of an oily product which by NMR was a 1:1 mixture of cis and trans isomers.

Step B: Preparation of 3-[2-(2-methoxy-5-ethyl-6-methylpyridin-3-yl)ethyl]-5-ethyl-6-methylpyridin-2(1H)-one

The cis/trans mixture of 3-[2-(2-methoxy-5-ethyl-6-methylpyridin-3-yl)ethenyl]-5-ethyl-6-methylpyridin-2(1H)-one (106 mg, 0.33 mmol) was dissolved in methanol (8 mL) and tetrahydrofuran (8 mL) containing 5% palladium/charcoal (92 mg) and hydrogenated at atmospheric pressure for 20 hours. The catalyst was filtered and the solvent evaporated. This residue was chromatographed on silica gel eluting with 0-1.25% methanol/chloroform gradient to give 53 mg (51% yield) of product. This material was crystallized from diethyl ether/hexane to give 38 mg of analytically pure product, mp 130-131°C;
$^1$H NMR (300 MHz, $CDCl_3$) δ 7.09 (1H, s), 6.96 (1H, s), 3.91 (3H, s), 2.72-2.87 (4H, m), 2.48 (2H, ABq., J=7.6 Hz), 2.39 (3H, s), 2.33 (2H, ABq., J=7.5 Hz), 2.30 (3H, s), 1.08 (3H, t, J=7.5 Hz), 1.03 (3H, t, J=7.6 Hz).

Anal. Calcd for $C_{19}H_{26}N_2O_2$:
C, 72.57; H, 8.34; N, 8.94.
Found: C, 72.36; H, 8.43; N, 8.86.

EXAMPLE 34

Preparation of 3-[2-(benzoxazol-2-yl)ethyl]-5-ethyl-6-hydroxymethylpyridin-2(1H)-one

Step A: Preparation of 4-benzyloxy-3-oxo-2-ethylbutanal

A solution of 907 pure 1-(N-morpholino)-1-butene (9.3 g, 60 mmol) and triethylamine (8.4 mL, 60 mmol) in tetrahydrofuran (85 mL), under a nitrogen atmosphere, was warmed to 70°C and benzyloxyacetyl chloride (9.45 mL, 60 mmol) was added dropwise via syringe. This cloudy yellow solution was warmed for 45 minutes and then cooled to room temperature. An aqueous solution of 10% HCl (75 mL) was added and the two phase mixture was stirred for 1 hour. This mixture was partitioned into methylene chloride and the organic layer was separated, dried ($Na_2SO_4$), filtered and the solvent evaporated to give a yellow oil (14.7 g) which contained the desired product. This oil was used directly in the next step.

Step B: Preparation of 3-cyano-5-ethyl-6-benzyloxymethylpyridin-2(1H)-one

Acetic acid (2.0 mL, 35 mmol) was added to a solution of crude 4-benzyloxy-3-oxo-2-ethylbutanal (6.4 g, ~24 mmol) and malononitrile (2.22 g, 33 mmol) in ethanol (30 mL) followed by dropwise addition of piperidine (2.37 mL, 24 mmol) to give a dark reddish brown solution. After stirring for 14 hours, a copious precipitate formed. This mixture was warmed at 70°C for 15 hours and then allowed to cool to room temperature. This blackish mixture was diluted with ethanol and the precipitated product was filtered, rinsed with ethanol and diethyl ether to give off-white product with m.p. of 141-143°C. Additional material was obtained by evaporation of the filtrate. The residue was extracted into chloroform, and after washing the extract with saturated aqueous $NaHCO_3$, filtered through a pad of charcoal. This amber solution was evaporated and the residue triturated with diethyl ether to give additional product.

Step C: Preparation of 2-benzyloxy-3-cyano-5-ethyl-6-benzyloxymethylpyridine

To a partial suspension of 3-cyano-5-ethyl-6-benzyloxymethylpyridin-2(1H)-one (1.36 g, 5.08 mmol) in dry benzene (20 mL) was added benzyl bromide (0.80 mL, 6.7 mmol) and then silver carbonate (1.43 g, 5.2 mmol). This mixture was covered with aluminum foil and allowed to stir at room temperature. After 24 hours, the reaction was estimated to be ~ 80% complete. Additional silver carbonate (.40 g, 1.45 mmol) was added and the mixture was stirred for another 24 hours until complete. The silver salts were removed by filtration, rinsed with benzene and the combined benzene washings were evaporated to give the pure product as an oil.

Step D: Preparation of 2-benzyloxy-5-ethyl-6-benzyloxymethylnicotinaldehyde

To a solution of 2-benzyloxy-3-cyano-5-ethyl-6-benzyloxymethylpyridine (2.57 g, 6.2 mmol) in dry toluene (10 mL), under a nitrogen atmosphere in an ice/acetone bath, was added dropwise a solution of 1.5 M diisobutylaluminum hydride/toluene (4.6 mL, 6.9 mmol). After stirring at room temperature for 15 hours, the reaction was cautiously poured into 10% HCl (30 mL), stirred for 0.5 hours and the product extracted into diethyl ether. The ethereal solution was dried, filtered through a pad of charcoal and evaporated to give a pale yellow oil. This oil was further purified by passing a benzene solution through a plug of silica gel to give pure product upon evaporation.

Step E: Preparation of 3-[2-(benzoxazol-2-yl)ethenyl]-5-ethyl-6-benzyloxymethyl-2-benzyloxypyridine

To a suspension of [(benzoxazol-2-yl)methyl]-triphenylphosphonium chloride (1.77 g, 4.12 mmol) in dry tetrahydrofuran (20 mL), under a nitrogen atmosphere, was added 60% NaH/mineral oil (.42 g, 10 mmol). After 0.5 hour a solution of 2-benzyloxy-5-ethyl-6-benzyloxymethyl-nicotinaldehyde (1.44 g, 4.0 mmol) in tetrahydrofuran (10 mL) was added and the reaction mixture was refluxed for 23 hours. The reaction was cooled, neutralized with acetic acid and partitioned between water and chloroform. The chloroform layer was washed with $NaHCO_3$ solution, dried, filtered through a pad of charcoal and evaporated. This material was dissolved in chloroform and flash chromatographed through silica gel to give pure product as a yellow oil. This was a mixture of cis and trans olefins.

Step F: Preparation of 3-[2-(benzoxazol-2-yl)ethyl]-5-ethyl-6-benzyloxymethylpyridin-2(1H)-one

A solution of cis/trans 3-[2-(benzoxazol-2-yl)ethenyl]-5-ethyl-6-benzyloxymethyl-2-benzyloxy-pyridine (1.40 g, 2.94 mmol) in dry tetrahydrofuran (20 mL) and methanol (15 mL) containing 10% Pd on charcoal (215 mg) as a catalyst was hydrogenated at atmospheric pressure for 20 hours. The catalyst was filtered off and the solution evaporated to give a white residue. This residue was triturated with diethyl ether to give pure product with m.p. of 140-142°C.

Step G: Preparation of 3-[2-(benzoxazol-2-yl)ethyl]-5-ethyl-6-hydroxymethylpyridin-2(1H)-one

A solution of 3-[2-(benzoxazol-2-yl)ethyl]-5-ethyl-6-benzyloxymethylpyridin-2(1H)-one (198 mg, 0.51 mmol) in dry methylene chloride (6 mL) was cooled in an ice/acetone bath and 1M boron tribromide/hexane (1.5 mL, 1.5 mmol) was added dropwise to give a white precipitate. This suspension was stirred for one hour and then the reaction was quenched by addition of saturated aqueous $NaHCO_3$ (10 mL). After stirring for 0.5 hours, the product was extracted into $CH_2Cl_2$, dried, filtered and the solvent evaporated to give a solid. Trituration with diethyl ether gave product as a tan solid. Recrystallization from ethyl acetate afforded a light yellow

solid with m.p. of 155-156°C.

Anal. Calcd. for $C_{17}H_{18}N_2O_3$:
C, 68.44; H, 6.08; N, 9.39.
Found: C, 68.15; H, 6.03; N, 9.07.

EXAMPLE 35

3-[2-(Benzoxazol-2-yl)ethyl]-5-ethyl-6-aminomethyl-pyridin-2(1H)-one

Step A: Preparation of 2-benzyloxy-3-[2-(benzoxazol-2-yl)ethyl]-5-ethyl-6-hydroxymethylpyridine

A suspension of 3-[2-(benzoxazol-2-yl)ethyl]-5-ethyl-6-hydroxymethylpyridin-2-(1H)one (598 mg, 2.0 mmol) in dry benzene (15 mL) was stirred with silver carbonate (891 mg, 3.23 mmol) and benzyl bromide (.39 mL, 3.36 mmol) in the dark (covered with aluminum foil) for 3-4 days. The salts were removed by filtration and the benzene solution was evaporated. The residue was chromatographed on silica gel and eluted with a 0-.75% methanol/chloroform gradient to give 734 mg (94% yield) of title product as an oil.

Step B: Preparation of 2-benzyloxy-3-[2-(benzoxazol-2-yl)ethyl]-5-ethyl-6-azidomethylpyridine

To a solution of 2-benzyloxy-3-[2-(benzoxazol-2-yl)ethyl]-5-ethyl-6-hydroxymethylpyridine (387 mg, 1.0 mmol) in distilled tetrahydrofuran (10 mL), under a nitrogen atmosphere, was added triphenyl phosphine (315 mg, 1.2 mmol), diphenylphosphoryl azide (.258 mL, 1.1 mmol) and finally diethyl azodicarboxylate (.19 mL, 1.2 mmol). The reaction mixture was stirred at ambient temperature for two hours, diluted with water, and extracted into chloroform. The chloroform extract was evaporated and the residue chromatographed on silica gel by elution with chloroform to give 370 mg (897 yield) of title compound as an oil.

Step C: Preparation of 2-benzyloxy-3-[2-(benzoxazol-2-yl)ethyl]-5-ethyl-6-aminomethylpyridine

To a suspension of stannous chloride dihydrate (254 mg, 1.13 mmol) in dry toluene (6 mL), under a nitrogen atmosphere and cooled in ice bath, was added thiophenol (.34 mL, 3.37 mmol) and then dropwise triethylamine (.47 mL, 3.37 mmol) to give a yellow solution. After 1 hour a precipitate formed and a solution of 2-benzyloxy-3-[2-(benzoxazol-2-yl)-ethyl]-5-ethyl-6-azidomethylpyridine (320 mg, .75 mmol) in dry toluene (4.5 mL) was added. After stirring the reaction mixture for three hours, methylene chloride was added and the solution was washed three times with 0.1 N NaOH. The organic solution was dried ($Na_2SO_4$) and evaporated to give 146 mg of crude product. This material was chromatographed on silica gel and eluted with 0-3% methanol ($NH_4OH$)/ chloroform gradient to give 122 mg (42% yield) of title compound as an oil.

Step D: Preparation of 3-[2-(benzoxazol-2-yl)ethyl]-5-ethyl-6-aminomethylpyridine-2(1H)-one

A solution of 2-benzyloxy-3-[2-(benzoxazol-2-yl)ethyl]-5-ethyl-6-aminomethylpyridine (122 mg, 0.31 mmol) in methanol (5 mL) and tetrahydrofuran (5 mL) containing 5% palladium/charcoal (29 mg) was hydrogenated at atmospheric pressure for 15-20 hours. The catalyst was filtered off and the solvents evaporated. The residue was chromatographed on silica gel by elution with 0-3.5% methanol ($NH_4OH$)/ chloroform. The appropriate fractions were combined, the solvents evaporated and the residue triturated with diethyl ether to give 30 mg (32% yield) of title compound as a tan solid, m.p. 159-161°C.

$^1H$ NMR ($CDCl_3$, 300 MHz) $\delta$ 7.63-7.67 (1H, m), 7.44-7.48 (1H, m), 7.27-7.29 (2H, m), 7.13 (1H, s), 3.84 (2H, s), 3.28 (2H, t, J=7.4 Hz), 3.09 (2H, t, J=7.4 Hz), 2.28 (2H, ABq, J=7.6 Hz), .98 (3H, t, J=7.6 Hz).

EXAMPLE 36

3-[2-(4,7-Dichlorobenzoxazol-2-yl)ethyl]-5-propyl-6-methylpyridin-2(1H)-one

Step A: Preparation of 3-cyano-5-propyl-6-methyl-gyridin-2(1H)-one

According to the method described in J. Heterocyclic Chem., 24, 351 (1987), solid sodium methoxide (20 g, .351 mol) was added to a solution of 2-hexanone (35.2 g, .351 mol) in diethyl ether (270 mL) and absolute ethanol (35 mL) which was cooled in an ice bath under a nitrogen atmosphere. Ethyl formate (28.4 mL, .351 mol) was added dropwise to this reaction and mixture was stirred overnight. This reaction was diluted with

diethyl ether and the precipitate was filtered off (6.65 g). Additional gummy precipitates were also collected (10.9 g and 31 gm).

This precipitate (6.65 g, 44 mmol) was dissolved in water and cyanoacetamide (4.15 g, 47 mmol) and aqueous piperidinium acetate [prepared from acetic acid (1 mL), water (2.5 mL) and piperidine (1.8 mL)] was added to the reaction which was refluxed for 3.5 hours. Acetic acid (5 mL) was added and the reaction was cooled to room temperature and the product mixture extracted into methylene chloride. This solution was dried ($Na_2SO_4$), filtered through a pad of charcoal and evaporated to 1.33 g of crude product mixture.

The two other precipitates (10.9 g and 31 gm) were treated as described above on a proportional scale-up to give 34.1 g of crude product mixture.

All reaction products were combined (35.3 g) and chromatographed on silica gel eluting with 0-1% methanol/chloroform gradient. The appropriate fractions were combined, evaporated and the residue triturated with diethyl ether to give 6.9 g (11% yield) of title product, mp 214-216°C.

Step B: Preparation of 2-chloro-3-cyano-5-propyl-6-methylpyridine

A mixture of 3-cyano-5-propyl-6-methyl-pyridin-2(1H)-one (4.41 g, 25 mmol) and phosphorus pentachloride (5.68 g, 27.2 mmol) was heated under a nitrogen atmosphere at 130°C for 0.75 hours. The syrup was poured into ice/water and extracted with chloroform. The chloroform extract was washed with water, dried ($Na_2SO_4$) and filtered through charcoal. The solvent was removed under vacuum and the residue redissolved in hexane, filtered through a pad of charcoal and evaporated to give 3.86 g (79% yield) of title product which slowly solidified upon standing.

Step C: Preparation of 2-methoxy-3-cyano-5-propyl-6-methylpyridine

Sodium metal (660 mg, 28.7 mmol) was dissolved in anhydrous methanol (15 mL) under a nitrogen atmosphere and a solution of 2-chloro-3-cyano-5-propyl-6-methylpyridine (3.86 g, 19.8 mmol) in methanol (12 mL) was added. The reaction was refluxed for two hours and then cooled and diluted with diethyl ether. The ethereal solution was washed with water, dried ($Na_2SO_4$) and filtered through charcoal. The solvent was evaporated and the residue was triturated with cold hexane to give 2.61 g (69% yield) of title compound, mp 72-74°C.

Step D: Preparation of 2-methoxy-5-propyl-6-methylnicotinaldehyde

To a solution of- 1M diisobutylaluminum hydride/tetrahydrofuran (20 mL, 20 mmol) was added 2-methoxy-3-cyano-5-propyl-6-methylpyridine (2.56 g, 13.5 mmol) in one portion at room temperature under a nitrogen atmosphere. The yellow solution was stirred for two hours and then poured into 1N aq. HCl (30 mL). This solution was extracted with diethyl ether and the ether layer was dried ($Na_2SO_4$), filtered through charcoal and evaporated to give 2.09 g (80% yield) of oily title compound.

Step E: Preparation of 2-methoxy-3-[2-(4,7-dichlorobenzoxazol-2-yl)ethenyl]-5-propyl-6-methylpyridine

Sodium hydride/mineral oil (60%, 137 mg, 3.4 mmol) was added to a suspension of [(4,7-dichlorobenzoxazol-2-yl)methyl]triphenylphosphonium chloride (1.55 g, 3.1 mmol) in dry tetrahydrofuran (8 mL) under a nitrogen atmosphere. After 0.5 hours, 2-methoxy-5-propyl-6-methylnicotinaldehyde (600 mg, 3.0 mmol) in tetrahydrofuran (2 mL) was added and the reaction mixture was refluxed overnight. The reaction was cooled and diluted with chloroform. The chloroform solution was washed with water, dried ($Na_2SO_4$) and filtered through a flash column of silica gel to give purified title compound as an oil (799 mg, 70% yield). NMR indicated that this oil was a 4.8:1 mixture of trans/cis isomers.

Step F: Preparation of 2-methoxy-3-[2-(4,7-dichlorobenzoxazol-2-yl)ethyl]-5-propyl-6-methylpyridine

2-Methoxy-3-[2-(4,7-dichlorobenzoxazol-2-yl)-ethyl]-5-propyl-6-methylpyridine (823 mg, 2.1 mmol) was dissolved in methanol (8 mL) and tetrahydrofuran (8 mL), 5% palladium/charcoal (102 mg) was added and this solution was hydrogenated at atmospheric pressure overnight. The catalyst was filtered off and the solvents evaporated to give a residue which was chromatographed on silica gel eluting with 5% ethyl acetate-hexane to give 621 mg (78% yield) of oily title compound.

Step G: Preparation of 3-[2-(4,7-dichlorobenzoxazol-2-yl)ethyl]-5-propyl-6-methylpyridin-2(1H)-one

A mixture of 2-methoxy-3-[2-(4,7-dichlorobenzoxazol-2-yl)ethyl]-5-propyl-6-methylpyridine (621 mg, 1.6 mmol) and pyridine hydrochloride (1.94 g, 224 mmol) was placed in a preheated oil bath at 150°C for 35 minutes. Although demethylation was incomplete (as indicated by TLC), the dark burgundy colored mixture was cooled, diluted with water, and extracted with a mixture of chloroform/methanol. This solution was dried ($Na_2SO_4$) and filtered through charcoal to give a yellow solution. After evaporation of the solvents, the residue was triturated with diethyl ether to give 138 mg of a ring-opened benzoxazole by-product. The ethereal solution was evaporated and the residue (277 mg) was chromatographed on silica gel eluting with 10-100% ethyl acetate/hexane. The appropriate fractions were combined and evaporated to give 85 mg (14% yield) of the title compound. This material was crystallized from methanol and then digested with diethyl ether to give 23 mg, mp 163-165°C of analytically pure product;
$^1H$ NMR (300 MHz, $CDCl_3$) δ 7.29 (1H, s), 7.23 (2H, q., J= Hz), 3.37 (2H, t, J=7.0 Hz), 3.13 (2H, t, J=7.0 Hz), 2.32 (2H, t, J=7.4 Hz), 2.30 (3H, s), 1.43 (2H, quintet), 0.83 (3H, t, J=7.4 Hz).

Anal. calcd. for $C_{18}H_{18}Cl_2N_2O_2$:
C, 59.19; H, 4.97; N, 7.67.
Found: C, 58.85; H, 4.95; N, 7.59.

EXAMPLE 37

Preparation of 3-{N-[(4,7-dichlorobenzoxazol-2-yl)-methyl]-N-methylamino}-5-ethyl-6-methylpyridin-2-(1H)-one

A solution of 3-{[N-[(4,7-dichlorobenzoxazol-2-yl)methyl]amino}-5-ethyl-6-methylpyridin-2(1H)-one (74 mg, 0211 mmol), paraformaldehyde (63.35 mg, 2.11 mmol), sodium cyanoborohydride (40 mg, 0.63 mmol), and acetic acid (51.3 mg, 0.63 mmol) in acetonitrile (0.57 mL) was stirred at room temperature overnight. The resultant solution was concentrated and the residue subjected to column chromatography on silica gel eluting with 5% methanol in chloroform. Collection and concentration of appropriate fractions yielded 3-{N-[(4,7-dichlorobenzoxazol-2-yl)methyl]amino}-5-ethyl-6-methylpyridin-2(1H)-one; mp 230-232°C (EtOH-$CHCl_3$);
$^1H$ NMR (300 MHz, DMSO) δ 7.49 (2H, AB, q, J=8.5 Hz), 6.60 (1H, s), 5.12 (2H, s), 2.82 (3H, s), 2.29 (2H, q, J=7.5Hz), 2.09 (3H, s), 1.01 (3H, t, J=7.5 Hz).

Anal. Calcd. for $C_{17}H_{17}Cl_2N_3O_2$:
C, 55.75; H, 4.68; N, 11.47.
Found C, 55.65; H, 4.63; N, 11.47.

EXAMPLE 38

Preparation of 3-{N-[(4,7-dimethylbenzoxazol-2-yl)-methyl]-N-methylamino}-5-ethyl-6-methyl-pyridin-2-(1H)-one

Following the procedure described in Example 37 treatment of 3-{N-[(4,7-dimethylbenzoxazol-2-yl) methyl]amino}-5-ethyl-6-methylpyridin-2(1H)-one with paraformaldehyde and sodium cyanoborohydride produced 3-{N-[(4,7-dimethylbenzoxazol-2-yl)methyl]-N-methylamino}-5-ethyl-6-methylpyridin-2(1H)-one; mp 175-177°C (EtOH-$CHCl_3$);

Anal. Calcd. for $C_{19}H_{23}N_3O_2$:
C, 70.13; H, 7.12; N, 12.91.
Found C, 69.83; H, 7.14; N, 12.80.

EXAMPLE 39

Preparation of 3-[(4,7-dichlorobenzo[b]furan-2-yl)-methylamino]-5-ethyl-6-methylpyridin-2-(1H)-one

Step A: Preparation of 2,5-dichlorophenyl-2-chloroprop-2-enyl ether

A mixture of 2,5-dichlorophenol (41 g, 0.25 mol), 2,3-dichloropropene (28 g, 0.25 mol), and potassium carbonate (35 g, 0.25 mol) in acetone (100 mL) was refluxed overnight. The resultant slurry was filtered and the filtrate concentrated. The residue was dissolved in diethyl ether, washed two times with 1 M aq. sodium hydroxide and three times with brine. The ethereal solution was then dried over sodium sulfate, filtered and concentrated to yield 41 g (68%) of 2,5-dichlorophenyl-2-chloroprop-2-enyl ether

Step B: Preparation of 2-(2-chloroprop-2-enyl)-3.6-dichlorophenol

A solution of 2,5-dichlorophenyl 2-chloroprop-2-enyl ether (10 g, 23 mmol) in 1,4-diisopropylbenzene (50 mL) was refluxed under an atmosphere of nitrogen for 48 hours. The resultant mixture was then subjected to column chromatography eluted firstly with hexane, then with 2% dichloromethane in hexane. Collection and conentration of appropriate fractions yielded 8.7 g (87%) of 2-(2-chloroprop-2-enyl)-3,6-dichlorophenol.

Step C: Preparation of 2-(2-chloroprop-2-enyl)-3,6-dichlorophenyl acetate

A solution of 2-(2-chloroprop-2-enyl)-3,6-dichlorophenol (8.7 g, 37 mmol), acetic anhydride (4:5 g, 44 mmol), diisopropylethylamine (5.7 g, 44 mmol) and N,N-dimethylaminopyridine (0.45 g, 3.7 mmol) in dichloromethane was stirred at room temp overnight. The resultant dark brown solution was concentrated and the residue subjected to column chromatogrpahy on silica gel eluted with 4% dichloromethane in hexane. Collection and concentration of appropriate fractions yielded 8.9 g (87%) of 2-(2-chloroprop-2-enyl)-3,6-dichlorophenyl acetate.

Step D: Preparation of 1-(2-acetoxy-3,6-dichlorophenyl)-3-chloro-2-propanone

A solution of 2-(2-chloroprop-2-enyl)-3,6-dichlorophenyl acetate (8.9 g, 32 mmol), and m-chloroperbenzoic acid (8-0%, 10 g) in dichloromethane (85 mL) was stirred at room temp for 8 days. The resultant mixture was then concentrated and the residue was dissolved in ether. The ethereal solution was washed successively with saturated aq. sodium bicarbonate, and brine. Th organic solution was then dried over sodium sulfate and concentrated under vacuum to give the corresponding chloroepoxide.

Without further purification, the chloroepoxide was dissolved in dichloromethane and stirred under an atmosphere of hydrogen chloride gas at room temp for 2 days. The resultant solution was concentrated to yield quantitatively 1-(2-acetoxy-3,6-dichlorophenyl)-3-chloro-2-propanone.

Step E: Preparation of 2-iodomethyl-4,7-dichlorobenzo(b)furan

A suspension of 1-(2-acetoxy-3,6-dichlorophenyl)-3-chloro-2-propanone (2.0 g, 6.8 mmol) in conc. hydrochloric acid (50 mL) was heated at 90°C for 1 hour. After cooling to room temp, the white solid that precipitated out of solution was dissolved in chloroform. The organic solution was washed three times with brine, dried over sodium sulfate, filtered, and concentrated. The residue was then subjected to column chromatography on silica gel eluted with chloroform-hexane, 1:1 v/v. Collection and concentration of appropriate fractions yielded 0.67 g (42%) of 2-chloromethyl-4,7-dichlorobenzo[b]furan. Treatment of the chloromethyl compound with sodium iodide in acetone at 60°C overnight yielded the corresponding 2-iodomethyl-4,7-dichlorobenzo[b]furan in 90% yield.

Step F: Preparation of 3-[(4,7-dichlorobenzo[b]-furan-2-yl)methylamino]-5-ethyl-6-methyl-pyridin-2(1H)-one

A mixture of 3-amino-5-ethyl-6-methylpyridin-2(1H)-one (0.186 g, 1.22 mmol), 2-iodomethyl-4,7-dichlorobenzo[b]furan (0.40 g, 1.22 mmol), diisopropylethylamine (0.32 g, 2.44 mmol) in acetonitrile (12 mL) was heated at 70-75°C under an atmosphere of nitrogen for 2 hours. The resultant mixture was cooled to 0°C. The pale brown solid precipitate was filtered, dried, and subjected to column chromatography on silica gel (elution with 5% methanol in chloroform). Collection and concentration of appropriate fractions, followed by recrystallization (95% aq. ethanol-chloroform) provided 3-[(4,7-dichtorobenzo[b]furan-2-yl)-methylamino]-5-ethyl-6-methyl-pyridin-2(1H)-one; mp 217-220°C;

$^1H$ NMR (300 MHz, $CDCl_3$) δ 7.15 (2H, AB, q, J=8.4 Hz), 6.74 (1H, s), 6.33 (1H, s), 4.53 (2H, s), 2.35 (2H, q, J=7.6 Hz), 2.24 (3H, s), 1.08 (3H, t, J=7.6 Hz).

| | |
|---|---|
| Anal. | Calcd. for $C_{17}H_{16}Cl_2N_2O_2 \cdot 0.2\ H_2O$ |
| | C, 57.54; H, 4.66; N, 7.90 |
| Found | C, 57.51; H, 4.51; N, 7.86 |

EXAMPLE 40

Preparation of 3-{N-[(4,7-difluorobenzoxazol-2-yl)methyl]amino}-5-ethyl-6-methyl-pyridin-2(1H)-one

Step A: Preparation of 2,5-difluoro-6-nitrophenol

To a mixture of concentrated sulfuric acid (15 g) and 20% fuming sulfuric acid (20 g) at 0°C, was added 2,5-difluorophenol (10 g, 77 mmol). The resultant slurry was stirred with a mechanical stirrer and heated to 80-90°C for 30 minutes. After cooling to 0°C, the slurry was diluted with concentrated sulfuric acid (15 mL), and a mixture of concentrated nitric acid (15 g) and 20% fuming sulfuric acid (8 mL) added dropwise over a period of 30 minutes. This mixture was stirred at 0°C for 30 minutes and then at room temperature overnight. The resultant mixture was then heated to 60°C over a period of 2 hours and held at that temperature for 3 hours. After cooling to room temperature, water (20 mL) was added and the product steam distilled. The yellow distillate was extracted twice with methylene chloride, and the extracts combined and washed with brine. The organic layer was dried over anhydrous sodium sulfate, filtered, and concentrated. The residue was subjected to column chromatography on silica gel eluted with 1% methanol in chloroform. Concentration and collection of appropriate fractions gave 2,5-difluoro-6-nitrophenol.

Step B: Preparation of 2-amino-3,6-difluorophenol

A yellow solution of 2,5-difluoro-6-nitrophenol (1.22 g, 7 mmol) in ethanol (70 mL) and acetic acid (0.42 g) was catalytically reduced in the presence of 5% platinum on charcoal (61 mg) under an atmosphere of hydrogen at room temperature overnight. The resultant colorless solution was filtered through a pad of Celite and concentrated to yield 0.83 g (85%) of 2-amino-3,6-difluorophenol.

Step C: Preparation of 2-Iodomethyl-4,7-difluorobenzoxazole

Following procedures similar to those described in Example 7, Step A, 2-amino-3,6-difluorophenol was transformed into 2-chloromethyl-4,7-difluorobenzoxazole. Reaction with sodium iodide in refluxing acetone gave 2-iodomethyl-4,7-difluorobenzoxazole.

Step D: Preparation of 3-{N-[(4,7-difluorobenzoxazol-2-yl)methyl]amino}-5-ethyl-6-methyl-pyridin-2(1H)-one

Following the procedure described in Step B, Example 7, reaction of 3-amino-5-ethyl-6-methylpyridin-2(1H)-one (0.23 g, 1.51 mmol), 2-iodomethyl-4,7-difluorobenzoxazole (0.45 g, 1.51 mmol) and diisopropylethylamine (0.2 g) in acetonitrile (15 mL) at 70-75°C under an atmosphere of nitrogen for 2 hours provided 3-{N-[(4,7-difluorobenzoxazol-2-yl)methyl]amino}-5-ethyl-6-methyl pyridin-2(1H)-one mp 216-217°C (EtOH-$CHCl_3$)

| | |
|---|---|
| Anal. | Calcd. for $C_{16}H_{15}F_2N_3O_2$: |
| | C, 60.18; H, 4.73; N, 13.16 |
| Found | C, 60.11; H, 4.67; N, 13.12 |

EXAMPLE 41

Preparation of 3-[N-{5-ethyl-2-methoxy-3-pyridinylmethyl}amino]-5-ethyl-6-methyl-pyridin-2(1H)-one

Step A: Preparation of 2-bromo-5-ethylnicotinonitrile

According to the method described in J. Organic Chem., 43, 2529 (1978) a solution of butyraldehyde (7.2 g, 0.10 mol), malononitrile (7.4 g, 0.11 mol) and DL-alanine (300 mg) in benzene (300 mL) containing acetic acid (6 mL) was refluxed for 2.5 hours with a Dean-Stark trap to remove water. The benzene solution was washed with water, dried ($Na_2SO_4$) and evaporated to give 10.7 g (89% yield) of 1,1-dicyanopentene.

This material was mixed with acetic anhydride (22 mL) and triethylorthoformate (16 mL) containing zinc chloride (300 mg) and was heated at 145°C for 24 hours. All volatiles below 110°C were distilled off and fresh acetic anhydride (4 mL) and triethylorthoformate (3.4 mL) was added and the reaction heated at 145°C for another 24 hours. The cooled reaction was poured into ice/water and the product was extracted into chloroform and washed with water, saturated $NaHCO_3$, dried ($Na_2SO_4$) and filtered through charcoal. Evaporation of the solvent gave 15.6 g (99% yield) of crude oily 1,1-dicyano-3-ethyl-4-ethoxy-1,3-butadiene.

Hydrogen bromide/acetic acid (30%) (60 mL) was added dropwise to a solution of 1,1-dicyano-3-ethyl-4-ethoxy-1,3-butadiene (12 g, 74 mmol) in acetic acid (40 mL) warmed at 50°C. After one hour, the reaction was poured into ice/water and the product extracted into a 1:1 mixture of benzene/hexane. The extract was dried ($Na_2SO_4$), filtered through charcoal and the solvent evaporated. This residue (3.7 g) was chromatographed on silica gel eluting with chloroform. Appropriate fractions were combined, the solvent evaporated and the residue triturated with cold hexane to give 2.03 g (13% yield) of crystalline title compound, mp 64-66°C.

Anal. Calcd. for $C_8H_7BrN_2$:
C, 45.52; H, 3.34; N, 13.27
Found C, 45.39; H, 3.24; N, 13.27

Step B: Preparation of 2-methoxy-5-ethylnicotinonitrile

Sodium metal (.35 g, 15.2 mmol) was dissolved in anydrous methanol (15 mL) under a nitrogen atmosphere and then 2-bromo-5-ethyl nicotinonitrile (2.02 g, 9.6 mmol) was added. The reaction was warmed at 55°C for one hour. After cooling, the reaction was diluted with diethyl ether and the ethereal solution was washed with water, dried ($Na_2SO_4$) and evaporated to give 1.52 g (98% yield) of oily title compound.

Step C: Preparation of 2-methoxy-5-ethylnicotinaldehyde

A solution of 2-methoxy-5-ethylnicotinonitrile (1.52 g, 9.38 mmol) in dry tetrahydrofuran (6 mL) was added, under a nitrogen atmosphere, to 1 M diisobutylaluminum/tetrahydrofuran (14 mL, 14 mmol) in an ice bath. After stirring for 4 hours the yellow solution was poured into 1N aq. HCl (20 mL) and extracted with diethyl ether. The ethereal extract was dried ($Na_2SO_4$), filtered through charcoal and evaporated. The residue was chromatographed on silica gel eluting with 10% ethyl acetate/hexane to give 706 mg (45% yield) of purified oily title compound.

Step D: Preparation of 3-[N-(5-ethyl-2-methoxy-3-pyridinylmethyl)amino]-5-ethyl-6-methylpyridin-2(1H)-one

Following the procedure described in Example 20, reaction of 3-amino-5-ethyl-6-methylpyridin-2(1H)-one with 5-ethyl-2-methoxy-nicotinaldehyde yielded 3-[N-(5-ethyl-2-methoxy-3-pyridinylmethyl)-amino]-5-ethyl-6-methyl-pyridin-2(1H)-one; mp 174-175°C (EtOH-$CHCl_3$).

Anal. Calcd. for $C_{17}H_{23}N_3O_2$:
C, 67.75; H, 7.69; N, 13.94
Found C, 67.64; H, 7.64; N, 13.96

## EXAMPLE 42

Preparation of 3-[N-{5,6-dimethyl-2-methoxy-3-pyridinylmethyl}amino]-5,6-dimethylpyridin-2(1H)-one

Following the procedure described in Examples 1 and 5, the compounds 3-amino-5,6-dimethylpyridin-2(1H)-one and 5,6-dimethyl-2-methoxynicotinaldehyde were prepared.

Following the procedure described in Example 20, reaction of 3-amino-5,6-dimethylpyridin-2(1H)-one with 5,6-dimethyl-2-methoxynicotinaldehyde gave 3-[N-(5,6-dimethyl-2-methoxy-3-pyridinylmethyl)amino]5,6-dimethylpyridin-2(1H)-one; mp 216-218°C (EtOH-$CHCl_3$)

Anal. Calcd. for $C_{16}H_{21}N_3O_2$:
C, 66.88; H, 7.37; N, 14.62
Found C, 66.56; H, 7.39; N, 14.52

## EXAMPLE 43

Preparation of 3-{N-[(2-methoxy-5,6,7,8-tetrahydroquinolin-3-yl)methyl]amino}-5,6-dimethylpyridin-2(1H)-one

Following the procedure described in Example 5, Steps A-D, 2-methoxy-5,6,7,8-tetrahydroquinolyl-3-aldehyde was prepared from the sodium salt of 2-formylhexanone.

Following the procedure described in Example 20, reaction of 3-amino-5,6-dimethylpyridin-2(1H)-one with 2-methoxy-5,6,7,8-tetrahydroquinolyl-3-aldehyde gave 3-{N-[(2-methoxy-5,6,7,8-tetrahydroquinol-3-yl)methyl]amino}-5-ethyl-6-methylpyridin-2(1H)-one; mp 194-196°C (EtOH-$CHCl_3$).

Anal. Calcd. for $C_{19}H_{25}N_3O_2$:

C, 69.70; H, 7.70; N, 12.83
Found C, 69.67; H, 7.64; N, 12.63

EXAMPLE 44

Preparation of 3-{N-[(2-methoxyquinolin-3-yl)methyl]-amino)-5-ethyl-6-methylpyridin-2(1H)-one

Following the procedure described in Example 5, Step D, the compound 3-cyano-2-methoxyquinoline was reduced to 2-methoxyquinolyl-3-carboxaldehyde.

Following the procedure described in Example 20, reaction of 3-amino-5,6-dimethylpyridin-2(1H)-one with 2-methoxyquinolinyl-3-carboxaldehyde provided 3-{N-[(2-methoxyquino1in-3-yl)-methyl]amino}-5-ethyl- 6-methylpyridin-2(1H)-one; mp 201-203°C (MeOH-$CHCl_3$).
$^1$HNMR (300 MHz, $CDCl_3$) δ 7.90 (1H, s), 7.85 (1H, d, J=8 Hz), 7.66 (1H, d, J=8 Hz), 7.59 (1H, dd, J=8.8 Hz), 7.35 (1H, dd, J=8.8 Hz), 6.19 (1H, s), 4.44 (2H, s), 4.15 (3H, s), 2.29 (2H, q, J=7.2 Hz), 2.23 (3H, s), 1.01 (3H, t, J=7.2 Hz)
Anal. Calcd. for $C_{19}H_{21}N_3O_2{\cdot}0.4$ MeOH:
C, 69.30; H, 6.78; N, 12.50
Found C, 68.93; H, 6.52; N, 12.77

EXAMPLE 45

Preparation of 5-ethyl-3-[N-(3-methoxy-6-methyl-2-pyridinylmethyl)amino]-6-methylpyridin-2(1H)-one

Step A: Preparation of 3-methoxy-6-methylpyridine-2-carboxaldehyde

To a slurry of sodium hydride (0.58 g, 24 mmol) in DMF (20 mL) at 0°C, 3-hydroxy-2-hydroxymethyl-6-methylpyridine (2.8 g, 20 mmol) was added. The resultant solution was stirred at room temperature for 1 hour, and recooled to 0°C. Iodomethane (2.84 g, 20 mmol) was added. The reaction mixture was then stirred at 0°C for 3 hours and at room temperature overnight. The resultant solution was concentrated and the residue subjected to column chromatography on silica gel eluted with 5% methanol-chloroform. An approximately 1:1 mixture of monomethylated products was obtained. Without further purification, the above product mixture was treated with a slurry of activated manganese (IV) oxide in carbon tetrachloride at room temp overnight. The resultant slurry was filtered and the filtrate concentrated. The residue was subjected to column chromatography on silica gel and eluted with 2% methanol-chloroform to give 3-methoxy-6-methylpyridine-2-carboxaldehyde.

Step B: Preparation of 5-ethyl-3-{N-[(3-methoxy-6-methyl-2-pyridinyl)methyl]amino}-6-methylpyridin-2-(1H)-one

Following the procedure described in Example 20, reaction of 3-amino-5-ethyl-6-methylpyridin-2(1H)-one with 3-methoxy-6-methylpyridine-2-carboxaldehyde provided 5-ethyl-3-{N-[3-methoxy-6-methyl-2-pyridinylmethyl]amino}-6-methyl-pyridin-2-(1H)-one; mp 213-215°C (EtOH-$CHCl_3$);
$^1$H NMR (300 mHz, $CDCl_3$) δ 7.09 (1H, d, J=8.4 Hz), 7.02 (1H, d, J=8.4 Hz), 6.46 (1H, s), 4.39 (2H, s), 3.86 (3H, s), 2.52 (3H, s), 2.37 (2H, q, J=7.6 Hz), 2.21 (3H, s), 1.11 (3H, t, J=7.6 Hz)
Anal. Calcd. for $C_{18}H_{21}N_3O_2{\cdot}0.15$ EtOH
C, 66.52; H, 7.50; N, 14.28
Found C, 66.32; H, 7.32; N, 14.31

EXAMPLE 46

Preparation of 3-{[(3-methoxybenzo[b]furan-2-yl)-methyl]amino}-5-ethyl-6-methyl-pyridin-2(18)-one

Step A: Preparation of methyl (2-carbomethoxyphenoxy)acetate

A mixture of methyl salicylate (15.2 g, 0.1 mol), methyl bromoacetate (15.3 g, 0.1 mol), and potassium carbonate (13.8 g, 0.1 mol) in acetone (250 mL) was refluxed overnight. The resultant slurry was filtered and the filtrate concentrated to yield 22.4 g of methyl (2-carbomethoxyphenoxy)acetate.

Step B: Preparation of methyl 3-hydroxybenzo[b]-furan-2-carboxylate

To a stirred solution of methyl (2-carbomethoxyphenoxy) acetate (22.4 g, 0.10 mol) in toluene at room temperature, sodium methoxide (12 g, 0.22 mol) was added portionwise. The resultant slurry was refluxed for 24 hours. After cooling, the solid was filtered and dissolved in water (200 mL). The resultant solution was acidified with acetic acid and a pale yellow solid precipitated out of solution. The solid was filtered and subjected to column chromatography on silica gel eluting with 3% methanol in chloroform. Collection and concentration of appropriate fractions yielded 5.6 g of methyl 3-hydroxybenzo[b]furan-2-carboxylate.

Step C: Preparation of methyl 3-methoxybenzo[b]-furan-2=carboxylate

To a solution of methyl 3-hydroxybenzo[b]-furan-2-carboxylate (5.6 g, 28 mmol) in a mixture of methanol and benzene (1:10, v/v, 100 mL) at room temperature, a 10% solution of trimethylsilyldiazomethane in hexanes was added slowly. The reaction was monitored continuously with TLC on silica gel eluted with 2% methanol in chloroform. After all the starting material was consumed, excess diazomethane was destroyed by addition of acetic acid. The product mixture was concentrated and the residue subjected to column chromatography on silica gel eluted with 1% methanol in chloroform. Collection and concentration of appropriate fractions yielded 5.2 g of methyl 3-methoxybenzo[b]furan-2-carboxylate.

Step D: Preparation of 3-methoxybenzo[b]furan-2-carboxaldehyde

To a solution of methyl 3-methoxybenzo[b]-furan-2-carboxylate (0.6 g, 2.8 mmol) in THF at 0°C, a solution of lithium aluminum hydride in THF (1M, 2.8 mL, 2.8 mmol) was added. The resultant solution was stirred at room temperature for 3 hours, then water(0.15 mL), 15% aq. sodium hydroxide (0.15 mL), and water (0.45 mL), were successively added. The resultant slurry was stirred at room temperature for 30 minutes, and filtered through a plug of Celite. The filtrate was then concentrated to give the required alcohol. Without further purification, the alcohol was dissolved in carbon tetrachloride (30 mL). Activated manganese (IV) oxide (4 g) was added and the resultant slurry was stirred at room temperature overnight. The product mixture was filtered and the filtrate concentrated to give 3-methoxybenzo[b]furan-2-carboxyaldehyde (0.26, 63%).

Step E: Preparation of 3-{[(3-methoxybenzo[b]-furan-2-yl)methyl]amino}-5-ethyl-6-methylpyridin-2(1H)-one

Following the procedure described in Example 20, reaction of 3-amino-5-ethyl-6-methylpyridin-2(1H)-one with 3-methoxybenzo[b]furan-2-carboxaldehyde provided 3-{[(3-methoxybenzo[b]-furan-2-yl)methyl] amino}-5-ethyl-6-methyl-pyridin-2(1H)-one; mp 198-195°C (EtOH-$CHCl_3$);

$^1H$ NMR (300 MHz, $CDCl_3$) δ 7.59 (1H, d, J=7 Hz), 7.37 (1H, d, J=7 Hz), 7.22 (2H, m), 6.51 (1H, s), 4.43 (2H, s), 4.04 (3H, s), 2.37 (2H, q, J=7.6 Hz), 2.20 (3H,s), 1.11 (3H, t, J=7.6 Hz).

| | |
|---|---|
| Anal. | calcd. for $C_{18}H_{20}N_2O_3$: |
| | C, 69.21; H, 6.45; N, 8.97 |
| Found: | C, 68.89; H, 6.32; N, 8.79 |

EXAMPLE 47

3-[N-(2-Benzyloxy-5-ethyl-6-methyl-3-pyridinylmethyl)amino]-5-ethyl-6-methylpyridin-2(1H)-one

A mixture of 2-benzyloxy-5-ethyl-6-methylnicotinaldehyde (0.31 g, 1.21 mmol), 3-amino-5-ethyl-6-methylpyridin-2(1H)-one (0.184 g, 1.21 mmol) and glacial acetic acid (3 drops) in MeOH (20 mL) was stirred at room temperature for 2 hours. $NaBH_4$ (0.38 g, 10 mmol) was added in portions over 30 minutes with ice-bath cooling. After warming to room temperature over 30 minutes, water and acetic acid were added to acidify the reaction mixture. Product was extracted into ethyl acetate which was washed with brine, dried ($Na_2SO_4$), filtered and concentrated. The residue was flash chromatographed over silica gel and 0.43 g (91%) of pure product eluted with 3% MeOH-97% $CHCl_3$. An analytical sample, mp=167-69°C, was obtained after recrystallization from MeOH-EtOAc-hexane.

| | |
|---|---|
| Anal. | calcd. for $C_{24}H_{29}N_3O$: |
| | C, 73.63; H, 7.47; N, 10.73. |
| Found: | C, 73.47; H, 7.39; N, 10.73. |

EXAMPLE 48

3-{[(5-Ethyl-6-methylpyridin-2(1H)-on-3-yl)methyl]-amino)-5-ethyl-6-methylpyridin-2(1H)-one

A solution of the benzyl ether of Example 47 (0.40 g, 1.02 mmol) in 150 mL of a 1:1 mixture of EtOH-EtOAc was hydrogenated over a 5% Pd/C catalyst (0.30 g) at 30 psi in a Paar shaker for 5 hours. After filtering, solvents were removed under reduced pressure and the residue flash chromatographed over silica gel. Pure product (0.090 g, 29%) was eluted with 15% MeOH-85% $CHCl_3$. An analytical sample, mp 258-62°C, dec, was prepared by recrystallization from MeOH-EtOAC-hexane.

Anal. calcd. for $C_{17}H_{23}N_3O_2$ :
C, 67.75; H, 7.69; N, 13.94.
Found: C, 67.53; H, 7.66; N, 13.86.

EXAMPLE 49

3-[(2,4-Dimethoxy-3-pyridinylmethyl)amino]-5-ethyl-6-methylpyridin-2(1H)-one

Step A: 2,4-Dimethoxynicotinaldehyde

Following the procedure of Example 5, Step D, the compound 2,4-dimethoxynicotinonitrile was reduced to 2,4-dimethoxynicotinaldehyde.

Step B: 3-[(2,4-Dimethoxy-3-pyridinylmethyl)amino]-5-ethyl-6-methylpvridin-2(1H)-one

Following the procedure of Example 20, reaction of 3-amino-5-ethyl-6-methyl-pyridin-2(1H)-one with the aldehyde of Step A provided 3-[(2,4-dimethoxy-3-pyridinylmethyl)amino]-5-ethyl-6-methylpyridin-2(1H)-one, mp 222-224°C, dec.

Anal. calcd. for $C_{16}H_{21}N_3O_3$ :
C, 63.35; H, 6.98; N, 13.85.
Found: C, 62.99; H, 7.21; N, 13.53.

EXAMPLE 50

3-{[(4,7-Dichlorobenzoxazol-2-yl)methyl]amino}-5-ethyl-6-methylpyridin-2(1H)-thione

Step A: 3-{[(4,7-dichlorobenzoxazol-2-yl)- methyl]amino}-2-tert-butylthio-5-ethyl-6-methylpyridine

Reaction of 2-tert-butylthio-5-ethyl-6-methyl-3-aminopyridine with 4,7-dichloro-2-iodomethyl-benzoxazole by the procedure of Step D of Example 8 afforded 3-{[(4,7-dichlorobenzoxazol-2-yl)methyl]-amino}-2-tert-butylthio-5-ethyl-6-methylpyridine.

Step B: 3-{[(4,7-dichlorobenzoxazol-2-yl)-methyl]amino}5-ethyl-6-methylpyridin-2(1H)-thione

Reaction of 3-{[(4,7)-dichlorobenzoxazol-2-yl)methyl]amino}-2-tert-butylthio-5-ethyl-6-methylpyridine with pyridine HCl by the procedure of Step E of Example 8 afforded 3-{[(4,7-dichlorobenzoxazol-2-yl)methyl]amino}5-ethyl-6-methylpyridin-2(1H)-thione.

Anal. calcd. for $C_{16}H_{15}Cl_2N_3OS$:
C, 52.18; H, 4.11; N, 11.41.
Found: C, 51.47; H, 4.02; N, 11.15.

EXAMPLE 51

3-[N-(2-Methoxy-5-methylbenzyl)amino]-5-ethyl-6-methylpyridin-2(1H)-one

Step A: 2-Methoxy-5-methylbenzaldehyde

4-Methylanisole (5.3 g, 43.5 mmol) was reacted with n-butyllithium (16.4 mL, 2.5 M in hexane) and dimethylformamide (6.7 mL, 86.6 mmol) according to the procedure described for the preparation of 5-ethyl-2-methoxybenzaldehyde (Example 22, Step B). The title compound was isolated as a yellow oil.

Step B: 3-[N-(2-Methoxy-5-methylbenzyl)amino]-5-ethyl-6-methylpyridin-2(1H)-one

2-Methoxy-5-methylbenzaldehyde (0.446 g, 2.97 mmol) was reacted with 3-amino-5-ethyl-6-methylpyridin-2[1H]-one (0.401 g, 2.63 mmol) in the presence of acetic acid (1 drop) and sodium borohydride (0.666 g, 17.6 mmol) according to the procedure described for the preparation of 3-[N-(2,6-dimethoxybenzyl)amino]-5-ethyl-6-methylpyridin-2[1H]-one (Example 21). The crude product was recrystallized from ethanol to give 351 mg of the title compound, mp 167-168°C.

Anal. calcd. for $C_{17}H_{22}N_2O_2$:
C, 71.30; H, 7.74; N, 9.78.
Found: C, 71.00; H, 7.77; N, 9.56.

EXAMPLE 52

3-[N-(2-Methoxy-4-methylbenzyl)amino]-5-ethyl-6-methylpyridin-2[1H]-one

Step A: 2-Methoxy-4-methylbenzaldehyde

3-Methylanisole (10.0 g, 0.081 mol) was reacted with n-butyllithium (32.7 mL, 2.5 M in hexane) and dimethylformamide (12.7 mL, 0.164 mol) according to the procedure described for the preparation of 5-ethyl-2-methoxybenzaldehyde (Example 22, Step B). The title compound was isolated as a yellow oil.

Step B: 3-[N-(2-Methoxy-4-methylbenzyl)amino]-5-ethyl-6-methylpyridin-2(1H)-one

2-Methoxy-4-methylbenzaldehyde (0.301 g, 2.01 mmol) was reacted with 3-amino-5-ethyl-6-methylpyridin-2[1H]-one (0.305 g, 2.01 mmol) in the presence of acetic acid and sodium borohydride (0.455 g, 12.3 mmol) according to the procedure described for the preparation of 3-[N-(2,6-dimethoxybenzyl)amino]-5-ethyl-6-methylpyridin-2[1H]-one (Example 21). The crude product was recrystallized from ethanol to give 143 mg of the title compound, mp 167.5-169°C.

Anal. calcd. for $C_{17}H_{22}N_2O_2$ :
C, 71.30; H, 7.74; N, 9.78.
Found: C, 71.24; H, 7.79; N, 9.71.

EXAMPLE 53

3-[N-(6-Methoxy-5-indanylmethyl)amino]-5-ethyl-6-methylpyridin-2(1H)-one

Step A: 5-Formyl-6-methoxyindane

5-Methoxyindane (5.0 g, 0.033 mol) was reacted with n-butyllithium (13.5 mL, 2.5 M in hexane) and dimethylformamide (5.2 mL, 0.067 mol) according to the procedure described for the preparation of 5-ethyl-2-methoxybenzaldehyde (Example 22, Step B). The title compound (0.602 g) was isolated by chromatography on silica gel with 2.5% ethyl acetate in hexane.

Step B: 3-[N-(6-Methoxy-5-indanylmethyl)amino]-5-ethyl-6-methylpyridin-2(1H)-one

5-Formyl-6-methoxyindane (0.353 g, 2.00 mmol) was reacted with 3-amino-5-ethyl-6-methylpyridin-2[1H]-one (0.305 g, 2.00 mmol) in the presence of acetic acid and sodium borohydride (0.452 g, 12.0 mmol) according to the procedure described for the preparation of 3-[N-(2,6-dimethoxybenzyl)amino]-5-ethyl-6-methylpyridin-2[1H]-one (Example 21). The crude product was recrystallized twice from methanol to give 198 mg of the title compound, mp 188-191.5°C.

Anal. calcd. for $C_{19}H_{24}N_2O_2$ :
C, 73.00; H, 7.74; N, 8.97.
Found: C, 73.00; H, 8.09; N, 8.94.

EXAMPLE 54

3-[N-(2,3-Dimethyl-6-methoxybenzyl)amino]-5-ethyl-6-methylpyridin-2(1H)-one

2,3-Dimethyl-6-methoxybenzaldehyde (from Example 23, Step A) (0.295 g, 1.80 mmol) was reacted with 3-amino-5-ethyl-6-methylpyridin-2[1H]-one (0.274 g, 1.80 mmol) in the presence of acetic acid and sodium borohydride (0.411 g, 10.9 mmol) according to the procedure described for the preparation of 3-[N(2,6-dimethoxybenzyl)amino]-5-ethyl-6-methylpyridin-2[1H]-one (Example 21). The crude product was recrystallized from methanol to give 72 mg of the title compound, mp 225-227°C.

Anal. calcd. for $C_{18}H_{24}N_2O_2$:
C, 71.97; H, 8.05; N, 9.32.
Found: C, 72.04; H, 8.12; N, 9.12.

EXAMPLE 55

3-{N-[(5-Ethyl-2,3-dihydro-7-benzo[b]furanyl)methyl]-amino}-5-ethyl-6-methylpyridin-2(1H)-one

Step A: 5-Acetyl-2,3-dihydrobenzo[b]furan

A solution of acetic acid (3.96 g, 0.065 mol) in trifluoroacetic anhydride (16.1 g, 0.076 mol) was added to 2,3-dihydrobenzo[b]furan (5.00 g, 0.041 mol) at 0°C. After 0.5 hours the reaction was added dropwise to a stirred, saturated solution of sodium bicarbonate (150 mL). The resulting mixture was extracted with chloroform and the organic extract washed with saturated sodium chloride solution and dried over magnesium sulfate. The chloroform solution was filtered and concentrated in vacuo to give the title compound (7.13 g) as a yellow solid

Step B: 5-Ethyl-2,3-dihydrobenzo[b]furan

5-Acetyl-2,3-dihydrobenzo[b]furan (2.00 g, 0.012 mol) dissolved in 5% aqueous tetrahydrofuran (130 mL) and 10% Pd/C (200 mg) was shaken on a Pan hydrogenator under 60 psi of $H_2$ for 72 hours. The catalyst was removed by filtration through diatomaceous earth and the filtrate reduced in volume under vacuum. The residue was dissolved in ether, washed with saturated sodium chloride solution and dried over sodium sulfate. The solution was filtered and concentrated in vacuo to give the title compound (2.01 g) as an oil.

Step C: 5-Ethyl-7-formyl-2,3-dihydrobenzo[b]furan

5-Ethyl-2,3-dihydrobenzo[b]furan (2.00 g, 0.013 mol) was reacted with n-butyllithium (5.39 mL, 2.5 M in hexane) and dimethylformamide (2.1 mL, 0.027 mol) according to the procedure described for the preparation of 5-ethyl-2-methoxybenzaldehyde (Example 22, Step B). The title compound (0.787 g) was isolated by chromatography on silica gel with 5% ethyl acetate in hexane.

Step D: 3-{N-[(5-Ethyl-2,3-dihydro-7-benzo[b]furan-yl)-methyl]amino}-5-ethyl-6-methylpyridin-2(1H)-one

5-Ethyl-7-formyl-2,3-dihydrobenzo[b]furan (0.355 g, 2.02 mmol) was reacted with 3-amino-5-ethyl-6-methylpyridin-2[1H]-one (0.306 g, 2.01 mmol) in the presence of acetic acid and sodium borohydride (0.458 g, 12.1 mmol) according to the procedure described for the preparation of 3-[N-(2,6-dimethoxybenzyl)amino]-5-ethyl-6-methylpyridin-2[1H]-one (Example 21). The crude product was recrystallized twice from methanol to give 266 mg of the title compound, mp 166-167°C.

Anal. calcd. for $C_{19}H_{24}N_2O_2 \cdot 0.25\ H_2O$
C, 72.01; H, 8.43; N, 8.84.
Found: C, 71.91; H, 7.53; N, 8.78.

$^1H$ NMR (300 MHz, $CDCl_3$) δ 11.15 (1H, s), 6.94 (2H, s), 6.26 (1H, s), 5.03 (1H, bs), 4.58 (2H, t, J=8.6 Hz), 4.22 (2H, d, J=5.2 Hz), 3.19 (2H, t, J=8.5 Hz), 2.54 (2H, 1, J=7.6 Hz), 2.33 (2H, q, J=7.6 Hz), 2.18 (3H, s), 1.17 (3H, t, J=7.6 Hz), 1.06 (3H, t, J=7.6 Hz).

EXAMPLE 56

3-[N-(5-Ethyl-2-methoxy-4-methylbenzyl)amino]-5-ethyl-6-methylpyridin-2(1H)-one

Step A: 4-Ethyl-3-methylphenol

4-Acetyl-3-methylphenol (10.0 g, 0.066 mol) in 50% aqueous tetrahydrofuran was hydrogenated with 10% Pd/C under 57 psi of $H_2$ according to the procedure described for the preparation of 5-ethyl-2,3-dihydrobenzofuran. The title compound (10.5 g) was obtained as an oil.

Step B: 4-Ethyl-3-methylanisole

A solution of 4-ethyl-3-methylphenol (5.1 g, 0.037 mol) in dimethylsulfoxide was treated with powdered potassium hydroxide (6.3 g, 0.112 mol) and methyl iodide (15.9 g, 0.112 mol) according to the procedure described for the preparation of 4-ethylanisole (Example 22, Step A). After workup, 5.06 g of the title compound was obtained.

Step C: 5-Ethyl-2-methoxy-4-methylbenzaldehyde

4-Ethyl-3-methylanisole (5.06 g, 0.033 mol) was reacted with n-butyllithium (13.5 mL, 2.5 M) and dimethylformamide (5.2 mL, 0.067 mol) according to the procedure described for the preparation of 5-ethyl-2-methoxybenzaldehyde (Example 22, Step B). The title compound (1.8 g) was isolated by chromatography on silica gel with ethyl acetate in hexane.

Step D: 3-[N-(5-Ethyl-2-methoxy-4-methylbenzyl)amino]-5-ethyl-6-methylpyridin-2(1H)-one

5-Ethyl-2-methoxy-4-methylbenzaldehyde (1.28 g, 8.4 mmol) was reacted with 3-amino-5-ethyl-6-methylpyridin-2[1H]-one (1.5 g, 8.4 mmol) in the presence of acetic acid and sodium borohydride according to the procedure described for the preparation of 3-[N-(2,6-dimethoxybenzyl)amino]-5-ethyl-6-methylpyridin-2[1H]-one (Example 21). The crude product was recrystallized from ethanol to give 1.32 g of the title compound, mp 183-185°C.

Anal. calcd. for $C_{19}H_{26}N_2O_2$:
C, 72.58; H, 8.33; N, 8.91.
Found: C, 72.42; H, 8.43; N, 8.81.

EXAMPLE 57

3-[N-(3,5-Dimethylbenzyl)amino]-5-ethyl-6-methylpyridin-2(1H)-one

3,5-Dimethylbenzaldehyde (200 mg, 1.5 mmol) was reacted with 3-amino-5-ethyl-6-methylpyridin-2[1H]-one (152 mg, 1.0 mmol) in the presence of acetic acid and sodium borohydride according to the procedure described for the preparation of 3-[N-(2,6-dimethoxybenzyl)amino]-5-ethyl-6-methylpyridin-2[1H]-one (Example 21). The crude product was recrystallized from ethanol to give 139 mg of the title compound, mp 221-223°C.

EXAMPLE 58

3-[N-(3,4-Dimethylbenzyl)amino]-5-ethyl-6-methylpyridin-2(1H)-one

3,4-Dimethylbenzaldehyde (134 mg, 1.0 mmol) was reacted with 3-amino-5-ethyl-6-methylpyridin-2[1H]one (152 mg, 1.0 mmol) in the presence of acetic acid and sodium borohydride according to the procedure described for the preparation of 3-[N-(2,6-dimethoxybenzyl)amino]-5-ethyl-6-methylpyridin-2[1H]one (Example 21). The crude product was recrystallized from ethanol to give 350 mg of the title compound, mp 154-155°C.

Anal. Calcd. for $C_{17}H_{22}N_2O$:
C, 75.51; H, 8.21; N, 10.36.
Found: C, 75.28; H, 8.22; N, 10.30.

EXAMPLE 59

3-[N-(2,6-Dimethoxy-4-fluorobenzyl)amino]-5-ethyl-6-methylpyridin-2(1H)-one

Step A: 2,6-Dimethoxy-4-fluorobenzaldehyde

2,6-Dimethoxy-4-fluorobenzene (2.5 g, 0.016 mol) was added to a mixture of phosphorous oxychloride (2.63 g, 0.017 mol) in dimethylformamide (5.0 mL, 0.064 mol). The reaction was stirred at 20°C for 12 hours then heated at 60°C for 24 hours. Water was added and the reaction was stirred overnight. The precipitated product was collected by filtration and air dried to give 1.6 g of the title compound.

Step B: 3-[N-(2,6-Dimethoxy-4-fluorobenzyl)amino]-5-ethyl-6-methylpyridin-2(1H)-one

2,6-Dimethoxy-4-fluorobenzaldehyde (0.184 g, 1.00 mmol) was reacted with 3-amino-5-ethyl-6-methylpyridin-2[1H]-one (0.152 g, 1.00 mmol) in the presence of acetic acid and sodium borohydride according to the procedure described for the preparation of 3-[N-(2,6-dimethoxybenzyl)amino]5-ethyl-6-methylpyridin-2[1H]-one (Example 21). The crude product was recrystallized from ethanol to give 186 mg of the title compound, mp 190-192°C.

Anal. Calcd. for $C_{17}H_{21}N_2O_3$:
C, 63.73; H, 6.61; N, 8.75.
Found: C, 63.54; H, 6.61; N, 9.00.

$^1H$ NMR (300 MHz, DMSO-$d_6$) δ 11.15 (1H, s), 6.41 (2H, m), 6.27 (1H, s), 4.81 (1H, bs), 4.09 (2H, s), 3.80 (3H, s), 3.71 (3H, s), 2.24 (2H, q, J=7.6 Hz), 1.99 (3H, s), 0.99 (3H, t, J=7.6 Hz).

EXAMPLE 60

3-[2-(Benzoxazol-2-yl)ethyl]-5-cyclopropyl-6-methylpyridin-2(1H)-one

Step A: Preparation of 5-Cyclopropyl-6-methyl-3-cyanopyridin-2(1H)-one

Potassium hydride (4.3 g of a 26.2% mineral oil dispersion; 28.0 mmol) was washed two times with hexane and the solid residue suspended in 20 mL of dry ethylene glycol dimethylether (DME). The suspension was stirred at -20°C as a solution of cyclopropylacetone (2.5 g, 25.5 mmol) in 5 mL of DME was added dropwise over 15 minutes. The cooling bath was removed and the reaction allowed to stir at ambient temperature for 3 hours, as the reaction temperature rose to 19°C. The yellow solution was cooled to -50°C and a solution containing 2.58 mL of ethyl formate (31.8 mmol) and 0.5 mL absolute ethanol in 4 mL of DME was added over 10 minutes during which the reaction temperature rose to -35°. When the addition was complete the cooling bath was removed and stirring was continued at ambient temperature for 18 hours. The resulting orange suspension was then chilled to -5° and treated with 0.57 mL (10 mmol) acetic acid and 1.0 mL (10 mmol) pyridine, followed by a solution of 2.35 g (28.0 mmol) of cyanoacetamide in 15 mL DME. The mixture was stirred at reflux for 4 hours, then cooled to 50-60° before adding 2 equivalents (2.9 mL) of acetic acid. After stirring at room temperature for 4 hours, the DME was removed on a rotary evaporator and the residue was partitioned between 60 mL each of water and $CHCl_3$. The organic layer was separated and the aqueous extracted once more with $CHCl_3$. The combined organic phases were washed once with 5% citric acid solution, saturated $NaHCO_3$ and brine before drying over $MgSO_4$. The solvent was removed on a rotary evaporator, leaving 2.9 g of an oily solid which proton NMR analysis showed to be a 2.5:1 mixture of the title compound and the undesired regioisomer, 6-cyclopropylmethyl-3-cyanopyridin-2(1H)-one, respectively. The latter could be removed by trituration with an ether/acetonitrile mixture, leaving 790 mg (18%) of 5-cyclopropyl-6-methyl-3-cyanopridin-2(1H)-one, mp 260-262°, dec.

$^1H$ NMR ($CDCl_3$) δ 0.56 (dd, 2H), 0.98 (dd, 2H), 1.61 (s, 1H), 1.69 (m, 1H), 2.58 (s, 3H), 7.65 (s, 1H).

The 6-cyclopropylmethyl isomer could also be removed via silica gel chromatography, eluting with a gradient of 100% ethyl acetate to 10% methanol in ethyl acetate. This undesired, less polar isomer is removed first with ethyl acetate prior to elution with the more polar solvent mixtures.

Step B through G:

Following the procedures of Example 5, Steps B through G, but substituting at each step the 5-ethyl pyridinone intermediate with the corresponding 5-cyclopropyl pyridinone intermediate, the title compound is

obtained, mp 159.5-160°C.
$^1H$ NMR (300 MHz, $CDCl_3$) δ 0.61 (m, 2H), 0.98 (m, 2H), 1.85 (m, 1H), 2.60 (s, 3H), 5.56 (s, 2H), 7.22-7.55 (m, 10H), 7.69-7.72 (m, 1H), 7.91 (d, 1H, J=16 Hz).

| | |
|---|---|
| Anal. | Calcd. for $C_{18}H_{18}N_2O_2$: |
| | C, 73.45; H, 6.16; N, 9.52. |
| Found: | C, 73.26; H, 6.07; N, 9.42. |

EXAMPLE 61

3-[(4,7-Dichlorobenzoxazol-2-yl)methyl]sulfinyl-5-ethyl-6-methylpyridin-2(1H)-one.

To a mixture of 3-[(4,7-dichlorobenzoxazol-2-yl)methyl]thio-5-ethyl-6-methylpyridin-2(1H)-one (Example 25) (0.019g, 0.00005 mol) in methylene chloride (2 mL) cooled in an ice-acetone bath was added a solution of 3-chloroperbenzoic acid (80%) (0.011g, 0.00005 mol) in methylene chloride (3 mL), and the mixture stirred overnight. The solvent was evaporated and the solid residue washed three times with diethyl ether. After drying under vacuum, the crude product was digested with hot acetonitrile. After cooling to room temperature there was obtained on filtration the desired product, mp 220-224°C.
$^1H$ NMR (DMSO-$d_6$) δ 0.72 (t, 3H, J=7.5 Hz), 2.25 (s, 3H), 2.26 (q, 2H, J=7.5 Hz), 4.70 (d, 1H, J=13.5 Hz), 4.90 (d, 1H, J=13.5 Hz), 7.20 (s, 1H), 7.48 (d, 1H, J=8.5 Hz), 7.52 (d, 1H, J=8.5).

EXAMPLE 62

3-[(4,7-Dichlorobenzoxazol-2-yl)methyl]sulfonyl-5-ethyl-6-methylpyridin-2(1H)-one.

To a mixture of 3-[(4,7-dichlorobenzoxazol-2-yl)methyl]thio-5-ethyl-6-methylpyridin-2(1H)-one (Example 25) in methylene chloride (2 mL) cooled in an ice-acetone bath was added 3-chloroperbenzoic acid (0.028g, 0.0001 mol) in methylene chloride (3 mL). The mixture was allowed to warm to room temperature and stirred overnight. After evaporation of solvent the residue was washed three times with excess diethyl ether. The solid remainder (0.015g) was dissolved in excess hot acetonitrile and filtered. Evaporation to a small volume gave the desired sulfone (0.012g), mp 312-313°C. $^1H$ NMR (DMSO-$d_6$) δ 0.94 (t, 3H, J=7.2 Hz), 2.33 (s, 3H), 2.38 (q, 2H, J=7.2 Hz), 5.36 (s, 2H) 7.54 (d, 1H, J=8.7 Hz), 7.59 (d, 1H, J=8.7 Hz).

EXAMPLE 63

Preparation of 3-[2-(7-Fluorobenzoxazol-2-yl)ethyl]-5-ethyl-6-methylpvridin-2(1H)-one

Step A: 3-[2-(7-fluorobenzoxozol-2-yl)-ethenyl]-2-benzyloxy-5-ethyl-6-methylpyridine

The title compound is prepared by Wittig condensation of 2-benzyloxy-5-ethyl-6-methylnicotinaldehyde and [(7-fluorobenzoxazol-2-yl)methyl]triphenylphosphonium chloride.

Step B: 3-[2-(7-Fluorobenzoxazol-2-yl)ethyl]-5-ethyl-6-methylpyridin-2(1H)-one

A solution of the olefin of Step A (150 mg, 0.40mmol) in absolute EtOH (10mL) and THF (6mL) was hydrogenated over a 5% Pd/C catalyst (25mg) at room temperature and atmospheric pressure for 18 hours. After filtering solvents were removed and the residue flash chromatographed on silica gel. Elution with 5% MeOH-95% $CHCl_3$ gave 108 mg of product. An analytical sample, mp 160-161°, was obtained upon recrystallization from MeOH.

| | |
|---|---|
| Anal. | Calcd for $C_{17}H_{17}FN_2O_2$: |
| | C, 67.99; H, 5.71; N, 9.33 |
| Found: | C, 67.84; H, 5.78; N, 9.33 |

EXAMPLE 64

Preparation of 3-[2-(3-Methoxybenzo[b]furan-2-yl)-ethyl]-5-ethyl-6-methylpyridin-2(1H)one.

Step A: Preparation of 3-[2-(3-Methoxybenzo[b]furan-2-yl)ethenyl]-5-ethyl-6-methylpyridin-2(1H)-one.

A mixture of 3-chloromethyl-5-ethyl-6-methylpyridin-2(1H)one (0.50g, 2.7mmol) and triphenylphosphine HBr (0.92g, 2.7mmol) in THF (8mL) was stirred at reflux under argon for 2 hr. The phosphonium salt was removed by filtration, washed with THF and $Et_2O$ and dried.

Sodium hydride (56mg, 2.3mmol) was added to a stirred slurry of this phosphonium salt (0.47g, 0.96mmol) in THF (8mL) under argon. After stirring at room temperature for 30 min, a solution of 3-methoxybenzo[b]furan-2-carboxaldehyde (0.17g, 0.96mmol) [Example 46, Step D] in THF (3mL) was added and the reaction stirred at 60° for 5 hours. At that point, additional NaH (28mg) was added and after heating for 16 hr more, the reaction was quenched with MeOH (2mL). Solvents were removed under reduced presure and the residue flash chromatographed over silica gel. Elution with 5% MeOH-95% EtOAc gave 77mg of product.

Step B: Preparation of 3-[2-(3-Methoxybenzo[b]furan-2-yl)ethyl]-5-ethyl-6-methylpyridin-2(1H)-one.

A solution of the olefin from Step A (75mg, 0.24 mmol) in MeOH (10mL) and THF (10mL) was hydrogenated over a 5% Pd/C catalyst (22mg) at room temperature and atmospheric pressure for 17 hr. After filtering, solvents were removed under reduced pressure and the residue flash chromatographed over silica gel. Elution with 5% MeOH-95% $CHCl_3$ gave 22mg of product, mp 128-30°

Anal. Calcd for $C_{19}H_{21}NO_3$;
C, 73.29; H, 6.80; N, 4.50
Found: C, 73.26; H, 6.88; N, 4.12

EXAMPLE 65

Preparation of 3-[2-(4,5,6,7-Tetrahydrobenzoxazol-2-yl)ethyl]-5-ethyl-6-methylpyridin-2(1H)one.

Step A: Preparation of 2-Choloromethyl-4,5,6,7-tetrahydrobenzoxazole.

A solution of triethylamine (4.65mL, 33.4 , mmol) in $CHCl_3$ (13mL) was added over 30 min to an ice bath cooled solution of chloroacetylchloride (2.83g, 25.1mmol) and 2-aminocyclohexanone HCl (2.5g, l6.7mmol) in $CHCl_3$. After stirring at room temperature overnight, the reaction mixture was washed with water, dried ($Na_2SO_4$), filtered and concentrated. Recrystallization from EtOAc-hexane afforded 1.23g of the chloroacetamide.

A solution of this amide (1.23g, 6.49mmol) in $POCl_3$ (21.6mL) was stirred at 100-105° for 7 hr. After removing excess $POCl_3$ under reduced pressure, ice was added and product extracted into $Et_2O$. The organic extract was washed with satd $NaHCO_3$, dried ($Na_2SO_4$), filtered and concentrated to give 1.07g of product.

Step B: Preparation of 2-Benzyloxy-3-[2-(4,5,6,7-tetrahydrobenzoxazol-2-yl)ethenyl]-5-ethyl-6-methylpyridine.

2-Chloromethyl-4,5,6,7-tetrahydrobenzoxazole was reacted with triphenylphosphine by the procedure of Example 6, Step A to give the corresponding phosphonium salt. Subsequent condensation with 2-benzyloxy-5-ethyl-6-methylnicotinaldehyde by the procedure of Example 6, Step A gave the title compound.

Step C: Preparation of 3-[2-(4,5,6,7-Tetrahydrobenzoxazol-2-yl)ethyl]-5-ethyl-6-methylpyridin-2(1H)one.

Catalytic reduction of the product of Step B by the procedure of Example 6, Step B gave 3-[2-(4,5,6,7-Tetrahydrobenzoxazol-2-yl)ethyl]-5-ethyl-6-methylpyridin-2(1H)one, mp 114-116°.

Anal. Calcd for $C_{17}H_{22}N_2O_2$:
C, 71.3; H, 7.74; N, 9.78
Found: C, 69.8; H, 7.75; N, 9.44

EXAMPLE 66

Preparation of 3-[2-(Benzoxazol-2-yl)ethyl]-5-isopropyl-6-methylpyridin-2(1H)one.

Step A: Preparation of 3-Cyano-5-acetyl-6-methylpyridin-2(1H)one.

A mixture of pentan-2,4-dione (25.7mL, 250 mmol) and DMF dimethylacetal (70.0mL, 5.23mol) was stirred at room temperature overnight. Upon standing, the reaction solidified. The solid was washed with $Et_2O$ and dried to give 29.9g of product

This product, dissolved in EtOH (200mL), was added to a solution of cyanoacetamide (16.8g. 200mmol) in EtOH (150mL) containing Na (4.6g, 200mmol). After stirring at room temperature overnight, the insoluble solid was removed by filtration and dissolved in water. Acidification with 6N HCl precipitated a yellow solid which was recrystallized from 95% EtOH to give 20g of product.

Step B: Preparation of 5-Acetyl-2-benzyloxy-3-cyano-6-methylpyridine.

A mixture of 3-cyano-5-acetyl-6-methylpyridin-2(1H)one (18g, 102mmol) from Step A, benzylbromide (12.1mL, 102mmol), silver carbonate (28.1g, 102mmol) and benzene (500mL) was protected from light and stirred under argon overnight. After filtering, solvent was removed under reduced pressure and the residue flash chromatographed over silica gel. Elution with $CHCl_3$ gave 25g of product.

Step C: Preparation of 2-Benzyloxy-3-cyano-5-isopropenyl-6-methylpyridine.

The acetyl derivative of Step B (3.0g) was added to a solution of Wittig reagent prepared from triphenylmethyl phosphonium iodide (9.1g, 22.5mmol) and n-butyl lithium (14mL of a 1.6M solution in hexane, 22.5mmol) in THF (60mL) and the reaction stirred at room temperature overnight. After concentration, the residue was flash chromatographed over silica gel. Product, 300mg, was eluted with $CHCl_3$.

Step D: Preparation of 2-benzyloxy-5-isopropenyl-6-methylnicotinaldehyde

The nitrile of Step C (800mg, 1.70mmol) was reduced with diisobutylaluminumhydride by the procedure of Example 41, Step C to give 270mg of the corresponding aldehyde.

Step E: Preparation of 3-[2-(Benzoxazo1-2-yl)-ethenyl]-2-benzyloxy-5-isopropenyl-6-methylpyridine.

The aldehyde of Step D (267mg, 1.0 mmol) was condensed with 2-benzoxazolylmethyl triphenylphosphonium chloride by the procedure of Example 6, Step A to give 200mg of product.

Step F: Preparation of 3-[2-(Benzoxazol-2-yl)ethyl]-5-isopropyl-6-methylpyridin-2(1H)one.

Catalytic reduction of the product of Step E (200mg) by the procedure of Example 6, Step B gave 49 mg of 3-[2-(Benzoxazol-2-yl)ethyl]-5-isopropyl-6-methylpyridin-2(1H)one.

| | |
|---|---|
| Anal. | Calcd for $C_{18}H_{20}N_2O$ |
| | C, 71.41; H, 7.05; N, 9.04. |
| Found: | C, 71.42; H, 6.83; N, 9.09. |

EXAMPLE 67

Preparation of 3-{[(7-Chlorobenzoxazol-2-yl)methyl]-amino}-5-ethyl-6-methylpyridin-2(1H)one.

Step A: Preparation of 2-Chloromethyl-7-chlorobenzoxazole

Following the prodedure of Example 7, Step A, reaction of 2-amino-6-chlorophenol with ethyl 2-chloroiminoacetate HCl provided 2-chloromethyl-7-chlorobenzoxazole.

Step B: Preparation of 3-{[(7-Chlorobenzoxazol-2-yl)methyl]amino}-5-ethyl-6-methylpyridin-2(1H)one.

Following the procedure described in Example 7, Step B, reaction of 3-amino-5-ethyl-6-methylpyridin-2(1H)-one with 2-chloromethyl-7-chlorobenzoxazole gave 3-{[(7-chlorobenzoxazol-2-yl)methyl]-amino}-5-ethyl-6-methylpyridin-2(1H)one, mp 219-220° (EtOH-$CHCl_3$).

| | |
|---|---|
| Anal. | Calcd for $C_{16}H_{16}ClN_3O_2$: |
| | C, 60.48; H, 5.08; N, 13.22 |
| Found: | C, 60.22; H, 4.94; N, 13.04 |

EXAMPLE 68

Preparation of 3-{[(4-Fluorobenzoxazol-2-yl)methyl] amino)-5-ethyl-6-methylpyridin-2(1H)one.

Step A: Preparation of 2-Chloromethyl-4-fluorobenzoxazole

Following the procedure of Example 7, Step A, reaction of 2-amino-3-fluorophenol with ethyl 2-chloroiminoacetate HCl provided 2-chloromethyl-4-fluorobenzoxazole.

Step B: Preparation of 3-{[(4-Fluorobenzoxazol-2-yl) methyl]amino}-5-ethyl-6-methylpyridin-2(1H)one.

Following the procedure described in Example 7, Step B, reaction of 3-amino-5-ethyl-6-methylpyridin-2(1H)one with 2-chloromethyl-4-fluorobenzoxazole gave 3-{[(4-fluorobenzoxazol-2-yl)methyl]-amino}-5-ethyl-6-methylpyridin-2(1H)one, mp 205-206° (EtOH-$CHCl_3$).

| | |
|---|---|
| Anal | Calcd. for $C_{16}H_{16}FN_3O_2$; |
| | C, 63.78; H, 5.35; N, 13.95 |
| Found | C, 63.88; H, 5.55; N, 13.98 |

EXAMPLE 69

Preparation of 3-[(5,6-Dimethyl-3-methoxy-2-pyridyl-methyl)amino]-5-ethy-6-methylpyridin-2(1H)one.

Step A: Preparation of 5,6-Dimethyl-3-nitropyridin-2(1H)one

Following the procedure of Example 1, Step A, 5,6-dimethyl-3-nitropyridin-2(1H)-one was prepared.

Step B: Preparation of 2-Chloro-5,6-dimethyl-3-nitropyridine

A solution of 3-nitro-5,6-dimethylpyridin-2(1H)one (16.8g, 0.1mol) in phosphorus oxychloride (100mL) was heated at 130°C for 6 hours. Excess $POCl_3$ was distilled off under reduced pressure. The residue was poured into vigorously stirred ice-water The off white solid precipitated was filtered and dissolved in methylene chloride. The resultant solution was washed with saturated aq. sodium bicarbonate, dried over anhydrous sodium sulfate, and passed through a plug of silica gel. Removal of solvent gave 17.2g (92%) of 2-chloro-5,6-dimethyl-3-nitropyridine.

Step C: Preparation of 3-amino-5,6-dimethylpyridine

A mixture of 2-chloro-5,6-dimethyl-3-nitropyridine (5g, 26.8mmol), triethylamine (10mL), and 5% palladium on charcoal (0.4g) in methanol (200 mL) was shaken under an atmosphere of hydrogen (45-40 psi) for 16 h. The resultant mixture was filtered through a plug of diatomaceous earth and concentrated under reduced pressure to yield 3.2g (100%) of 3-amino-5,6-dimethylpyridine.

Step D: Preparation of 5,6-dimethyl-3-hydroxypyridine

To a cold (0°C) solution of 3-amino-5,6-dimethylpyridine (4g, 32.8 mmol) in 5% aq. sulfuric acid (100mL), a solution of sodium nitrite (2.5g, 36mmol) in water (20mL) was added dropwise. The resultant solution was stirred at 0°C for 30 min, transferred into an addition funnel maintained at 0°C with external cooling, and added dropwise into boiling 5% aq. sulfuric acid (70 mL) over a period of 30 minutes. The resultant solution was refluxed for additional 15 minutes, cooled to 0°C, neutralized with 40% aq sodium hydroxide, saturated with the addition of solid sodium chloride, and the product extracted into methylene chloride. The organic extract was dried over anhydrous sodium sulfate, filtered, and concentrated under reduced pressure to yield 3.5 g (86%) of crude 3-hydroxy-5,6-dimethylpyridine.

Step E: Preparation of 5,6-Dimethyl-2-hydroxymethyl-3-hydroxypyridine

To a solution of 3-hydroxy-5,6-dimethylpyridine (3.5 g, 28.4 mmol) and sodium hydroxide (1.2 g, 28.4 mmol) in water (12 mL) at 90°C, 38% aq formaldehyde was added in four 2.5 mL aliquots in 90 minute intervals. The resultant solution was heated at 90°C for an additional 90 minutes, neutralized with acetic acid, and concentrated under reduced pressure. The residue was triturated with 10% methanol in chloroform saturated with ammonia, and filtered through a plug of Celite. The filtrate wes concentrated and the residue subjected to column chromatography on silica gel eluted with 10% methanol in chloroform. Collection and concentration of appropriate fractions gave 2.1 g (48%) of 2-hydroxymethyl-3-hydroxy-5,6-dimethylpyridine.

Step F: Preparation of 2-hydromethyl-5,6-dimethyl-3-methoxypyridine

To a solution of 2-hydroxymethyl-3-hydroxy-5,6-dimethylpyridine (1.16 g, 7.6 mmol) and sodium hydride (0.18 g, 7.6 mmol) in dimethylformamide (20 mL) at 0°C, methyl iodide (1.08 g, 7.6 mmol) was added and stirred at 0°C for 30 minutes, and then at room temperature for 1 hour. The resultant solution was concentrated under reduced pressure, and the residue subjected to column chromatography on silica gel eluted with 2% methanol in chloroform. Collection and concentration of appropriate fractions gave 0.7 g (55%) of 2-hydroxymethyl-3-methoxy-5,6-dimethylpyridine.

Step G: Preparation of 5,6-dimethyl-3-methoxy picolinaldehyde

A slurry of 2-hydroxymethyl-3-methoxy-5,6-dimethylpyridine (0.7 g, 4.2 mmol) and activated manganese (IV) oxide (8 g) in methylene chloride (40 mL) was stirred at room temperature overnight. The resultant mixture was filtered through a plug of diatomaceous earth and the filtrate concentrated to give 0.4 g (58%) of 5,6-dimethyl-3-methoxy-2-picolinaldehyde.

Step H: Preparation of 3-[(5,6-dimethyl-3-methoxy-2-pyridylmethyl)amino]-5-ethy-6-methylpyridine-2(1H)-one

A solution of 3-amino-5-ethyl-6-methylpyridin-2(1H)-one (184 mg, 1.21 mmol), 5,6-dimethyl-3-methoxypicolinaldehyde (200 mg, 1.21 mmol), and acetic acid (1 drop) in methanol (5 mL) was stirred at room temperature for 2 hours. The yellow precipitate was filtered and dissolved in a mixture of methanol and chloroform. To the resultant yellow solution, sodium borohydride was added until the solution became colorless. The product solution was then concentrated, and the residue subjected to column chromatography on silica gel eluted with 8% methanol in chloroform. Collection and concentration of appropriate fractions yielded 3-[(5,6-dimethyl-3-methoxy-2-pyridylmethyl)amino]-5-ethy-6-methylpyridin-2(1H)-one; mp 234-236°C ($CHCl_3$-EtOH);
$^1$H-NMR (300 MHz, DMSO) δ 7.25 (s, 1H), 6.22 (s, 1H), 5.83 (t, 1H, J=5.2 Hz), 4.13 (d, 2H, J=5.2 Hz), 3.82 s, 3H), 2.38 s, 3H), 2.30 (q, 2H, J=7.5 Hz), 2.26 (s, 3H), 2.05 (s, 3H), 1.05 (t, 3H, J=7.5 Hz).

| | |
|---|---|
| Anal. | Calcd for $C_{17}H_{23}N_3O_2$: |
| | C, 67.75; H, 7.69; N, 13.94. |
| Found: | C, 67.56; H, 7.58; N, 13.65. |

EXAMPLE 70

Preparation of 3-{N-[(7-Methylbenzoxazol-2-yl)-methyl]amino}-5-ethyl-6-methylpyridin-2(1H)-one

Step A: Preparation of 2-chloromethyl-7-methylbenzoxazole

2-Methyl-6-aminophenol was allowed to react with 2-chloroiminoacetate HCl by the procedure of Example 7, Step A to give 2-chloromethyl-7-methylbenzoxazole, mp 72-75°C.

Step B: Preparation of 3-{N-[(7-Methylbenzoxazol-2-yl)methyl]amino}-5-ethyl-6-methylpyridinpyridin-2(1H)-one

Reaction of the 2-chloromethyl-7-methylbenzoxazole of Step A with 3-amino-5-ethyl-6-methylpyridin-2(1H)-one by the procedure of Step B, Example 7 gave the title product.

| | |
|---|---|
| Anal | Calcd for $C_{17}H_{19}N_3O$: |
| | C, 68.67; H, 6.44; N, 14.13. |

Found: C, 68.71; H, 6.72; N, 14.13.

EXAMPLE 71

Preparation of 3-[(5-Ethyl-2-methoxy-6-methyl-3-pyridinylmethyl)amino]-5-cyclopropyl-6-methylpyridin-2(1H)-one.

Step A: Preparation of 5-Cyclopropyl-6-methyl-3-nitropyridin-2(1H)-one.

A solution of cyclopropylacetone (1.87 g, 19.05 mmol) in dimethoxyethane (10 mL) was added slowly over 10 min to a stirred slurry of KH (0.841 g, 20.96 mmol) in dimethoxyethane (18 mL) cooled to -20 to -35° under Ar. After addition was complete, the mixture was stirred at room temperature for 1 hr and then cooled to -45°. A solution of ethyl formate (1.76 g, 23.82 mmol) in dimethoxyethane (5 mL) was added over 1 hr and the reaction allowed to stir at room temperature overnight. After filtering and adding ethyl ether (20mL), the precipitated solids were combined by dissolving in MeOH and concentrating to 4.02 g of product.

This product was combined with ammonium nitroacetamide (2.96 g, 24.5 mmol) in water (55 mL) and HOAc (2.80 mL, 49 mmol) and stirred at room temperature overnight. The yellow solid was removed by filtration and recrystallized from CH3CN to give 5-cyclopropyl-6-methyl-3-nitropyridin-2(1H)-one.

Step B: Preparation of 5-Cyclopropyl-6-methyl-3-aminopyridin-2(1H)-one.

Catalytic reduction of 5-cyclopropyl-6-methyl-3-nitropyridin-2(1H)-one in THF- MeOH over a Pt/C catalyst by the procedure of Example 1, Step B, gave 5-cyclopropyl-6-methyl-3-aminopyridin-2(1H)-one.

Step C: Preparation of 3-[(5-Ethyl-2-methoxy-6-methyl-3-pyridinylmethyl)amino]-5-cyclopropyl-6-methylpyridin-2(1H)-one.

Reaction of 5-cyclopropyl-6-methyl-3-aminopyridin-2(1H)-one with 2-methoxy-5-ethyl-6-methylnicotinaldehyde by the procedure of Example 20 gave 3-[(5-ethyl-2-methoxy-6-methyl-3-pyridinylmethyl) amino]-5-cyclopropyl-6-methylpyridin-2(1H)-one.

Anal. Calcd for $C_{19}H_{25}N_3O_2$:
C, 69.70; H, 7.70; N, 12.83.
Found: C, 70.09; H, 7.50; N, 12.67.

EXAMPLE 72

3-[2-(Benzoxazol-2-yl)ethyl]-5-cyano-6-methylpyridin-2(1H)-one

Step A: Ethyl 3-cyano-6-methylpyridin-2(1H)-on-5-ylcarboxylate

To a solution made from ethanol (30 mL) and sodium (0.96 g, 0.042 mol) was added cyanoacetamide (3.54g, 0.042 mol) in ethanol (40 mL). To this mixture was added 2-[(dimethylamino)methylene]-3-oxobutanoic acid ethyl ester (6.88 g, 0.40 mol) in ethanol (20 mL). After stirring overnight at room temperature, the solids were filtered and washed with diethyl ether. After drying, the crude sodium salt was slurried in water and acidified with conc. hydrochloric acid to pH 2. Filtration gave the title compound (56% yield) mp 207-211°C.

Step B: Ethyl 6-benzyloxy-5-cyano-2-methylpyridine-3-carboxylate

A mixture of ethyl 3-cyano-6-methylpyridin-2(1H)-on-5-ylcarboxylate (6.18 g, 0.03 mol), benzyl bromide (6.16 g, 0.036 mol) and silver carbonate (9.09 g, .033 mol) in benzene (100 mL) was stirred overnight at room temperature in the dark. Filtration, followed by evaporation, gave a solid residue which was slurried with hexane and filtered to give the desired product (7.67 g, 88.2% yield), mp 130-133°C.

Step C: 6-Benzyloxy-5-cyano-2-methylpyridine-3-carboxamide

A mixture of ethyl 6-benzyloxy-5-cyano-2-methylpyridine-3-carboxylate (5.92 g, 0.02 mol), 1.0 N sodium hydroxide (21 mL, 0.021 mol) in ethanol (125 mL) was stirred under argon overnight. The clear solution was evaporated to dryness under vacuum to give intermediate sodium salt (5.97 g).

To a mixture of the sodium salt (5.97 g, 0.021 mol) in tetrahydrofuran, cooled in an ice-acetone bath, was added dropwise isobutyl chloroformate (3.09 g, 0.023 mol). The mixture was stirred overnight at room temperature and then added rapidly dropwise to an ice cold solution of tetrahydrofuran (80 mL) which had been saturated with ammonia gas. The mixture was allowed to warm to room temperature and then stirred for an additional two hours. Evaporation afforded a solid residue which was partitioned between chloroform and water. After washing the chloroform solution with water and then brine, it was evaporated to give the title product (3.81 g, 69.3% yield); mp 160-162°C

Step D: 6-Benzyloxy-5-formyl-2-methylpyridine-3-carboxamide.

To a mixture of 6-benzyloxy-5-cyano-2-methylpyridine-3-carboxamide (90.92 g, 0.0034 mol) in toluene (50 mL), cooled in an ice-acetone bath, was added dropwise 1.5 M diisobutylaluminum hydride in toluene (5.0 mL, 0.0075 mol). After stirring for 2 hours the reaction mixture was quenched into a mixture of ice and excess 1N hydrochloric acid. The product was extracted into ethyl acetate which was then washed well with water, saturated aqueous sodium bicarbonate and brine. After drying, evaporation of solvent gave solid aldehyde (0.64 g, 69.7% yield) which was not purified further.

Step E: 6-Benzyloxy-5-[2-(benzoxazol-2-yl)ethenyl]-2-methylpyridine-3-carboxamide

Following the procedure of Example 6, Step A, the Wittig reaction is carried out by reacting 6-benzyloxy-5-formyl-2-methyl pyridine-3-carboxamide with [2-(benzoxazo-2-yl)methyl]triphenylphosphonium chloride to give the title compound.

Step F: 6-Benzyloxy-5-[2-(benzoxazol-2-yl)ethenyl]-2-methylpyridine-3-carbonitrile

To a solution of 6-benzyloxy-5-[2-(benzoxazol-2-yl)ethenyl]-2-methylpyridine-3-carboxamide (0.53 g, 0.0014 mol) in dry tetrahydrofuran (50 mL) was added in portions over 2 hours methoxycarbonylsulfamoyltrimethylammonium hydroxide inner salt (Burgess reagent) (0.88 g, 0.0037 mol). After stirring overnight chloroform was added and the organic phase washed well with water, and then brine. Evaporation gave crude product (0.64 g). Purification by flash chromatography on 230-400 mesh silica gel, using 20% ethyl acetate-methylene chloride for development, gave pure product (0.50 g, 97% yield).

Step G: 3-[2-(Benzoxazol-2-yl)ethyl]-5-cyano-6-methylpyridin-2(1H)-one

A mixture of 6-benzyloxy-5-[2-(benzoxazol-2-yl)ethenyl]-2-methylpyridine-3-carbonitrile (0.10 g, 0.00028 mol), and 10% palladium on carbon in 50-50 ethanol-tetrahydrofuran (40 mL) was stirred at room temperature overnight under a balloon of hydrogen gas. After filtration through a bed of filter-aid, evaporation, refiltration in chloroform solution and re-evaporation, there was obtained single spot product. Recrystallization from boiling acetonitrile gave the title compound (0.046 g, 58.8% yield), mp 220-225°C.
$^1$H NMR (300 MHz, $CDCl_3$) δ 2.51 (s, 3H), 3.10 (t, 2H, J=7.2 Hz), 3.27 (t, 2H, J=7.2 Hz), 7.3 (m, 2H), 7.40 (s, 1H), 7.48 (m, 1H), 7.68 (m, 1H).

Anal. Calcd for $C_{16}H_{13}N_3O_2$:
C, 68.81; H, 4.69; N, 15.04;
Found: C, 68.95; H, 4.63; N, 14.65.

EXAMPLE 73

3-[2-(Benzoxazol-2-yl)ethyl]-6-methylpyridin-2(1H)-on-5-ylcarboxamide

A mixture of 6-benzyloxy-5-[2-(benzoxazol-2-yl)ethenyl]-2-methylpyridine-3-carboxamide (0.05 g, 0.00013 mol), 10% palladium on carbon (0.04 g) in a 50-50 mixture of ethanol-tetrahydrofuran (60 mL) was stirred under a balloon of hydrogen gas overnight. The mixture was filtered through a bed of filter-aid. After evaporation the residue was dissolved in a mixture of chloroform and ethanol (with warming) and refiltered. The residue obtained on evaporation (0.041 g) was recrystallized from hot acetonitrile to give product (0.032 g, 82.9% yield) mp 264-270°C. Slurrying in methanol improved the melting point to 273-275°C.
$^1$H NMR (300 MHz, DMSO-$d_6$) δ 2.38 (s, 3H), 2.9 (t, 2H), 3.2 (t, 2H), 7.15 (s, NH), 7.35 (m, 2H), 7.5 (s, NH), 7.56 (s, 1H), 7.64 (m, 2H).

Anal. Calcd for $C_{16}H_{15}N_3O_3$:

C, 63.67; H, 5.17; N, 13.92;
Found: C, 63.75; H, 4.87; N, 13.73.

EXAMPLE 74

3-[2-(Benzoxazol-2-yl)ethyl]-4,6-dimethyl-5-ethylpyridin-2(1H)-one

Step A: 4,6-Dimethyl-5-ethylpyridin-2(1H)-one

A mixture of 3-cyano-4,6-dimethyl-5-ethyl pyridin-2(1H)-one (1.24 g, 0.0007 mol) and conc. hydrochloric acid was heated overnight at 150°C in a sealed tube. After cooling, the solution was evaporated to dryness. The residue was dissolved in water and the solution made basic with conc. ammonium hydroxide. The solids after filtering and drying had mp 193-199°C (0.99 g, 93.6% yield).

Step B: 4,6-Dimethyl-5-ethyl-3-nitropyridin-2(1H)-one

To a mixture of 4,6-dimethyl-5-ethylpyridin-2(1H)-one (0.72 g, 0.0044 mol) in conc. sulfuric acid (95.1 mL) cooled in an ice bath was added 70% nitric acid (0.44 mL, 0.0049 mol). The reaction mixture was stirred at ice temperature for 1 hour and then warmed to room temperature for 1 hour. The reaction was poured onto ice, and the solids which formed filtered, washed well with water, and dried (0.39 g, 45.2% yield), mp 233-238°C.

Step C: 3-Amino-4,6-dimethyl-5-ethylpyridin-2(1H)-one

A mixture of 4,6-dimethyl-5-ethyl-3-nitro-pyridin-2(1H)-one (0.37 g, 0.0019 mole) and 10% palladium on carbon (0.06 g) in 50-50 ethanoltetrahydrofuran (50 mL) was stirred under a balloon of hydrogen for 4 hours. Filtration followed by evaporation gave the title product (0.32 g).

Step D: 3-[(Benzoxazol-2-yl)methyl]amino-4,6-dimethyl-5-ethylpyridin-2(1H)-one

A mixture of 3-amino-4,6-dimethyl-5-ethylpyridin-2(1H)-one (0.32 g, 0.0019 mol), 2-chloromethylbenzoxazole (0.36 g, 0.0021 mol), 1,8-bis(dimethylamino)naphthalene (0.45 g, 0.0021 mol) in acetonitrile (10 mL) was heated at reflux for 24 hours. After cooling, the solids were filtered, washed with acetonitrile and dried (0.33 g). Chromatography on 230-400 mesh silica gel, employing 5% 2-propanol-methylene chloride and then 10% 2-propanol-methylene chloride for development, permitted separation of the desired product from contaminating dialkylated byproduct. The title compound had mp 214-216°C

Anal. Calcd for $C_{17}H_{19}N_3O_2$:
C, 68.67; H, 6.44; N, 14.13
Found: C, 68.53; H, 6.49; N, 14.13

EXAMPLE 75

3-[(4,7-Dichlorobenzoxazo1-2-yl)methyl]amino-5-methoxy-6-methylpyridin-2(1H)-one

Step A: 5-Methoxy-6-methyl-3-nitropyridin-2(1H)-one

A mixture of 1-methoxy-2-propanone (4.41 g, 0.05 mol) and ethyl formate (4.07 g, 0.055 mol) was added dropwise to a solution of sodium ethoxide in ethanol (1.15 g of sodium dissolved in 60 mL of ethanol) cooled in an ice-acetone bath. After stirring for 1 hour, the bath was removed and the mixture allowed to warm to room temperature. Some colorless solids were removed by filtration and the filtrate allowed to stir at room temperature overnight. The dark colored reaction mixture was evaporated and the solid residue pumped to constant weight.

A portion (4.9 g) of the crude 1-hydroxymethylene-1-methoxy-2-propanone sodium salt and 2-nitroacetamide ammonium salt (5.08 g, 0.043 mol) were dissolved in water (50 mL). To this mixture was added 3.4 M piperidinium acetate (10.6 mL) and acetic acid (2.52 g, 0.042 mol). After being heated at 50 C for 4.5 hours, the aqueous mixture was extracted with chloroform. Evaporation of the chloroform gave a very crude product which was chromatographed on a column of 230-400 mesh silica gel. The product was eluted with 2% 2-propanolmethylene chloride, followed by 3% 2-propanolmethylene chloride. The title compond had mp 204-207°C (dec), (0.31 g).

Anal. Calcd for $C_7H_8N_2O_4$:

C, 45.66; H, 4.38; N, 15.21.
Found: C, 45.66; H, 4.03; N, 15.02.

Step B: 3-[(4,7-Dichlorobenzoxazol-2-yl)methyl]amino-5-methoxy-6-methylpyridin-2(1H)-one

A mixture of 5-methoxy-6-methyl-3-nitropyridin-2(1H)-one (0.072 g, 0.0064 mol) and 10% palladium on carbon (0.03 g) in ethanol (20 mL) was stirred under a balloon of hydrogen gas for 1.5 hours. After filtration through a bed of filter-aid and evaporation of solvent, the residue (0.066 g) was carried directly into the next reaction. Acetonitrile (5 mL), 2-chloromethyl-4,7-dichlorobenzoxazole (0.12 g, 0.0005 mol) and 1,8-bis-(dimethylamino)naphthalene (0.11 g, 0.0005 mol) were added and the mixture heated at reflux for 31 hours. After cooling, the solids were removed by filtration (0.033 g). After dissolving in chloroform, filtering and evaporation, the product was crystallized by dissolving in hot acetonitrile and partially evaporating. The title compound (0.02 g) had mp 215°C (dec).
$^1$H NMR (300 MHz, $CD_3OD$) δ 2.11 (s, 3H), 3.69 (s, 3H), 4.76 (s, 2H), 6.73 (s, 1H), 7.39 (s, 2H).
Anal. Calcd for $C_{15}H_{13}Cl_2N_3O_3$:
C, 50.87; H, 3.70; N, 11.86.
Found: C, 50.22; H, 3.56; N, 11.65.

EXAMPLES 76 & 77

1-Methyl-4-[2-(benzoxazol-2-yl)ethyl]-2,5,6,7-tetrahydro-3H-2-pyrinden-3-one and 4-methyl-3-[2-(benzoxazol-2-yl)ethyl]-1,5,6,7-tetrahydro-2H-1-pyrinden-2-one

Step A: 1-Methyl-4-cyano-2,5,6,7-tetrahydro-3H-2-pyrinden-3-one and 4-methyl-3-cyano-1,5,6,7-tetrahydro-2H-1-pyrinden-2-one

2-Acetylcyclopentanone (5.05 g, 0.04 mol), cyanoacetamide (3.36 g, 0.04 mol), and diethylamine (1.6 mL) in ethanol (80 mL) were heated together overnight in a bath maintained at 60°C. After cooling the solid product was obtained as a nearly 1:1 mixture of regioisomers (5.50 g, 80% yield).

Step B: 1-Methyl-3-benzyloxy-4-cyano-6,7-dihydro-5H-2-pyrindene and 2-benzyloxy-3-cyano-4-methyl-6,7-dihydro-5H-1-pyrindene

The product mixture from Step A (4.34 g, 0.025 mol), benzyl bromide (4.48 g, 0.026 mol) and silver carbonate (7.55 g, 0.026 mol) in benzene (300 mL) were stirred together for 24 hours at room temperature in the dark. Following filtration and evaporation the two components were separated by flash chromatography on an 80mm (i.d.) column of 230-400 mesh silica gel. Elution with 50-50 methylene chloride - hexane gave 1-methyl-3-benzyloxy-4-cyano-6,7-dihydro-5H-2-pyrindene (1.39 g) as the first product off; mp 63-67°C.
Anal. Calcd for $C_{17}H_{16}N_2O$:
C, 77.24; H, 6.10; N, 10.60
Found: C, 77.28; H, 6.19; N, 10.61
Following the collection of mixed fractions (3.40 g), pure 2-benzyloxy-3-cyano-4-methyl-6,7-dihydro-5H-1-pyrindene (1.98 g) was obtained, mp 100-102°C.
Anal. Calcd for $C_{17}H_{16}N_2O$:
C, 77.24; H, 6.10; N, 10.60
Found: C, 77.06; H, 5.98; N, 10.65

Step C: 1-Methyl-3-benzyloxy-6,7-dihydro-5H-2-pyrindene-4-carboxaldehyde

To 1-methyl-3-benzyloxy-4-cyano-6,7-dihydro-5H-2-pyrindene (0.53 g, 0.002 mol) in toluene (20 mL) cooled in an ice-acetone bath was added dropwise 1.5M diisobutylaluminum hydride in toluene (1.47 mL, 0.0022 mol). After stirring for 4 hours, the reaction was quenched into a mixture of ice and 1N hydrochloric acid (40 mL). Extraction into ethyl acetate, followed by washing with water, saturated aqueous sodium bicarbonate, and brine, drying (magnesium sulfate) and evaporation gave the title product as a solid (0.344 g, 64.4% yield).

Step D: 1-Methyl-3-benzyloxy-4-[2-(benzoxazol-2-yl)-ethenyl]-6,7-dihydro-5H-2-pyrindene

Following the procedure of Example 6, Step A, the Wittig reaction is carried out by reacting 1-methyl-3-benzyloxy-6,7-dihydro-5H-2-pyrindene-4- carboxaldehyde with [2-(benzoxazo-2-yl)methyl]triphenylphosphonium chloride to give the title compound.

Step E: 1-Methyl-4-[2-(benzoxazol-2-yl)ethyl]-2,5,6,7-tetrahydro-3H-2-pyrinden-3-one

A mixture of 1-methyl-3-benzyloxy-4-[2-(benzoxazol-2-yl)ethenyl]-6,7-dihydro-5H-2-pyrindene (0.23 g, 0.0006 mol), 10% palladium on carbon (0.04 g) in 50-50 ethanol-tetrahydrofuran (90 mL) was stirred under a balloon of hydrogen overnight. Filtration through a pad of filter-aid, followed by evaporation of solvent gave a white solid (0.18 g). Recrystallization from boiling acetonitrile gave an analytical sample, mp 224-227°C.

Anal. Calcd for $C_{18}H_{18}N_2O_2$:
C, 73.45; H, 6.10; N, 9.52
Found: C, 73.21; H, 5.97; N, 9.92

Starting with 2-benzyloxy-3-cyano-4-methyl-6,7-dihydro-5H-1-pyrindene (from Step B) and carrying out the procedures of Steps C,D and E, there was obtained 4-methyl-3-[2-(benzoxazol-2-yl)ethyl]-1,5,6,7-tetrahydro-2H-1-pyrinden-2-one, mp 227-230°C.

Anal. Calcd for $C_{18}H_{18}N_2O_2$:
C, 73.45; H, 6.10; N, 9.52.
Found: C, 73.53; H, 6.10; N, 9.58.

EXAMPLE 78

3-[2-(Benzoxazol-2-yl)ethyl-5-vinyl-6-methylpyridin-2(1H)-one

Step A: 2-Benzyloxy-5-acetyl-6-methylpyridin-3-carbonitrile

A mixture of 3-cyano-5-acetyl-6-methylpyridin-2(1H)-one (1.76g, 0.01 mol) (L. Mosti et al, J. Het. Chem. 22, 1503 (1985)), benzyl bromide (2.12g, 0.012 mol), silver carbonate (3.06g, 0.011 mol) in benzene was stirred at room temperature under a nitrogen atmosphere overnight, while protected from light. The following day the mixture was filtered, and the solids washed well with benzene. Evaporation of the filtrate gave the desired intermediate (2.47g, 92.8% yield), mp 94-99°C.

Anal. Calcd for $C_{16}H_{14}N_2O_2$
C, 72.17; H, 5.30; N, 10.52;
Found: C, 71.97; H, 5.30; N, 10.19.

Step B: 2-Benzyloxy-5-(1-hydroxyethyl)-6-methylpyridin-3-carbonitrile

Sodium borohydride (0.35 g, 0.0092 mol) was added at room temperature to 2-benzyloxy-3-cyano-5-acetyl-6-methylpyridin-2(1H)-one (2.44g, 0.0092 mol) in ethanol (60mL). After 2 hours chloroform was added plus a few drops of acetic acid. After washing with water and brine, the chloroform solution was evaporated to give product (2.85g) without further purification.

Step C: 2-Benzyloxy-5-(1-hydroxyethyl)-6-methylpyridin-3-carboxaldehyde

To a mixture of 2-benzyloxy-5-(1-hydroxyethyl)-6-methylpyridin-3-carbonitrile (1.00g, 0.0037 mol) in toluene (25 mL), cooled in an ice-acetone bath, was added dropwise diisobutylaluminum hydride (1.5 M in toluene) (5.22 mL, 0.0078 mol). After stirring for one hour under nitrogen the mixture was worked up by quenching into a mixture of excess 1N hydrochloric acid and ice. Extraction with chloroform followed by washing with water, saturated sodium bicarbonate solution, and then brine, and evaporation, gave the desired product (0.091g, 90% yield).

Step D: (±)-3-[2-(Benzoxazol-2-yl)ethyl]-5-(1-Hydroxyethyl)-6-methyl-2(1H)-pyridinone

The product of step C is then condensed with [(benzoxazol-2-yl)methyl]triphenylphosphonium chloride via the Wittig reaction, then treated with base, in accordance with the procedure and protocols of Example 6, Step A, yielding the title compound(s).

To a solution of 3-[2-(benzoxazol-2-yl) ethyl]-5-(1-hydroxyethyl)-6-methylpyridin-2(1H)-one (0.05 g, 0.00018 mol) in tetrahydrofuran (20 mL) was added in portions over 1 hour methoxycarbonylsulfamoyltriethylammonium hydroxide inner salt (Burgess reagent). The mixture was stirred overnight at room temperature. For work-up the reaction was diluted with chloroform and the solution washed well with 1N hydrochloric acid. After separation the chloroform layer was washed with saturated aqueous sodium bicarbonate solution and brine. Evaporation gave a very crude residue which was dissolved in ethyl acetate and the solution stirred with 1N hydrochloric acid for 2 hours. After separation the ethyl acetate phase was washed with aqueous saturated sodium bicarbonate solution, and brine. Following drying over magnesium sulfate and evaporating, the residue was purified further by chromatography on a silica gel plate (20 cm by 20 cm, 2 mm thick), with 10% methanol - methylene chloride for development. The major UV visible band was scraped off and extracted with 10% methanol-chloroform to obtain product which showed peaks in the $^1H$ NMR spectrum ($CDCl_3$) for olefin protons - 6 5.2 (d, 1H, J=11 Hz), 5.4 (d, 1H, J=17 Hz), 6.6 (q, 1H, J=11, 17 Hz).

EXAMPLE 79

3-[2-(Benzoxazol-2-yl)ethyl]-5-ethyl-6-methoxymethylpyridin-2(1H)-one

Step A: Preparation of 2-benzyloxy-5-ethyl-3-[2-(benzoxazol-2-yl)ethyl]-6-methoxyethylpyridine

To a solution of 2-benzyloxy-3-[2-(benzoxazol-2-yl)ethyl]-5-ethyl-6-hydroxymethylpyridine (See Example 34, 324 mg, 0.84 mmol) in distilled tetrahydrofuran (4 mL) was added 80% sodium hydride/ mineral oil (35 mg, 1.1 mmol). After stirring for 0.5 hours at ambient temperature, methyl iodide (.12 mL, 20 mmol) was added and the reaction was stirred for 23 hours. The reaction was neutralized with dilute HCl, extracted with chloroform and the chloroform extract dried ($Na_2SO_4$) and evaporated to give 323 mg (96% yield) of title compound as an oil.

Step B: Preparation of 3-[2-(benzoxazol-2-yl)ethyl]-5-ethyl-6-methoxymethylpyridin-2(1H)-one

A solution of 2-benzyloxy-5-ethyl-3-[2-(benzoxazol-2-yl)ethyl]-6-methoxymethylpyridine (231 mg, 0.57 mmol) in glacial acetic acid (5 mL) containing potassium iodide (177 mg, 1.07 mmol) was warmed at 60°C for 1.5 hours. The solvent was removed under vacuum and residue dissolved in methylene chloride. This solution was washed with $NaHCO_3$ solution, sodium bisulfite, dried ($Na_2SO_4$) and evaporated. The residue was triturated with diethyl ether to give 101 mg (56% yield) of the title compound. An analytical sample was obtained by two crystallizations from diethyl ether, m.p. 134-136°C.
$^1H$ NMR ($CDCl_3$, 300 mHz) δ 7.64-7.67 (1H, m), 7.45-7.48 (1H, m), 7.27-7.30 (2H, m), 7.16 (1H, s), 4.35 (2H, s), 3.46 (3H, s), 3.30 (2H, t, J=7.3 Hz), 3.11 (2H, t, J=7.3 Hz), 2.26 (2H, ABq, J=7.6 Hz), 1.00 (3H, t, J=7.6 Hz).

| | |
|---|---|
| Anal. | calcd. for $C_{18}H_{20}N_2O_3$: |
| | C, 69.21; H, 6.45; N, 8.97 |
| Found: | C, 69.49; H, 6.19; N, 8.85 |

EXAMPLE 80

3-[2-(Benzoxazol-2-yl)ethyl]-5-(1,1-difluoroethyl)-6- methylpyridin-2(1H)-one

Step A: Preparation of 3-cyano-5-[1-(1,3-dithiolan-2-yl)ethyl]-6-methylpyridin-2(1H)-one

A suspension of 3-cyano-5-acetyl-6-methylpyridin-2(1H)-one (1.0 g, 5.68 mmol) in 1,2-ethanedithiol (1.0 mL) was treated with boron trifluoride etherate (1 mL), followed by glacial acetic acid (5.0 mL). The resulting suspension was stirred at room temperature under an argon atmosphere for seven hours. Additional quantites of boron trifluoride etherate (1.0 mL) and 1,2-ethanedithiol (1.0 mL) were added and the mixture was stirred for 15 hours. The thick reaction mixture was diluted with n-butyl chloride/hexanes and filtered. The yellow solid was triturated with warm diethyl ether and filtered to give 1.10 g (77% yield) of the title compound as a pale orange solid, mp 130-132°C;
$^1H$ NMR ($CDCl_3$3, 300 MHz) δ 2.11 (3H, s), 2.80 (3H, s), 3.29-3.40 (2H, m), 3.44-3.55 (2H, m), 8.53 (1H, s).

Step B: Preparation of 3-cyano-5-(1,1-difluoroethyl)-6-methylpyridin-2(1H)-one

Pyridine hydrofluoride (3.19 mL, 13.9 mmol) was added to a suspension of 1,3-dibromo-5,5-dimethylhydantoin (1.80 g, 6.3 mmol) in methylene chloride (10 mL) cooled to -78°C. This was followed by a rapid portionwise addition of 3-cyano-5-[1-(1,3-dithiolan-2-yl)ethyl]-6-methylpyridin-2(1H)-one (1.0 g, 3.96 mmol). The suspension was stirred at -78°C for 30 minutes. The cold reaction was treated with dilute aqueous $NaHCO_3$

until the pH was adjusted to 8. The layers were separated and the organic layer was washed with dilute aq. $NaHCO_3$, water and brine. The dried ($Na_2SO_4$) methylene chloride solution was evaporated to give .87 g of orange solid which was purified by flash chromatography over silica gel eluting with 4% methanol/chloroform. The resultant inseparable 3:1 mixture (.50 g) of title compound and 3-cyano-5-(1,1-difluoro-2-bromoethyl)-6-methylpyridin-2(1H)-one was dissolved in tetrahydrofuran (5 mL) and treated with azobisisobutyronitrile (8 mg) and tributyltin hydride (.28 g, 0.95 mmol). The resulting solution was refluxed for one hour and then concentrated in vacuo to give a clear oil which was flash chromatographed on silica gel eluting with 5% methanol/chloroform to give a white solid. The solid was recrystallized from hot isopropanol to give .25 g (32% yield) of title compound, mp 203-2,05°C (dec.);

$^1$H NMR (DMSO-$d_6$, 300 mHz) δ 1.95 (3H, t, J=17 Hz), 2.42 (3H, s), 8.17 (1H, s).

| | | |
|---|---|---|
| Anal | calcd for $C_9H_8F_2N_2O$: | |
| | C, 54.54; H, 4.07; N, 14.14 | |
| Found: | C, 54.56; H, 3.95; N, 13.99 | |

Step C: Preparation of 2-benzyloxy-3-cyano-5-(1,1-difluoro)ethyl-6-methylpyridine

To a suspension of 3-cyano-5-(1,1-difluoroethyl)-6-methylpyridine (750 mg, 3.78 mmol) and benzyl bromide (778 mg, 4.54 mmol) in benzene (10 mL) was added silver carbonate (1.17 g, 4.54 mmol). This suspension was covered in aluminum foil to exclude light and stirred for 24 hours under an argon atmosphere. The reaction was filtered through a Celite pad which was rinsed with chloroform. The filtrate was concentrated in vacuo to give 1.29g (100% yield) of title compound as a white solid containing a small amount of benzyl bromide. The product was used as is:

$^1$H NMR ($CDCl_3$, 300 mHz) δ 1.90 (3H, t, J=17 Hz), 2.63 (3H, s), 5.50 (2H, s), 7.25-7.40 (3H, m). 7.43-7.52 (2H, m), 7.89 (1H, s).

Step D: Preparation of 2-benzyloxy-5-(1,1-difluoroethyl)-6-methylnicotinaldehyde

To a solution of 2-benzyloxy-3-cyano-5-(1,1-difluoroethyl)-6-methylpyridine (.84 g, 2.91 mmol) in dry toluene (10 mL) cooled to -10°C was added dropwise 1.5 M diisobutylaluminum hydride/toluene (2.13 mL, 3.2 mmol). The reaction was stirred for one hour and then additional diisobutylaluminum hydride/toluene (.30 mL) was added. After 0.5 hour, the reaction was quenched by addition of 1N HCl (20 mL). The resulting mixture was stirred at room temperature for two hours. The layers were separated and the organic layer was concentrated in vacuo to give a pale yellow oil. This material was flash chromatographed on silica gel by eluting with 10:1 hexane/diethyl ether to give 510 mg (60% yield) of title compound as a clear oil, $^1$H NMR ($CDCl_3$, 300 MHz) δ 1.95 (3H, t, J=17 Hz), 2.67 (3H, s), 5.55 (2H, s), 7.30-7.41 (3H, m), 7.47 (2H, dd, J=5 Hz), 8.19 (1H, s), 10.37 (1H, s).

Step E: Preparation of 2-benzyloxy-3-[2-(benzoxazol-2-yl)ethenyl]-5-(1,1-difluoroethyl)-6-methylpyridine

Following the procedure of Example 6, Step A, the Wittig reaction is carried out by reacting 2-benzyloxy-5-(1,1-difluoroethyl)-6-methylnicotinaldehyde with [2-(benzoxazo-2-yl)methyl]triphenylphosphonium chloride to give the title compound.

Step F: Preparation of 3-[2-(benzoxazol-2-yl)ethyl]-5-(1,1-difluoroethyl)-6-methylpyridin-2(1H)-one

A solution of 2-benzyloxy-3-[2-(benzoxazol-2-yl)ethenyl]-5-(1,1-difluoroethyl)-6-methylpyridine (425 mg, 1.08 mmol) in tetrahydrofuran (30 mL) and methanol (30 mL) containing 5% palladium/carbon (100 mg) was hydrogenated at atmospheric pressure for four hours. The mixture was filtered through a Celite pad and the filtrate concentrated to give an off-white oily solid, which was flash chromatographed on silica gel with 4% methanol/chloroform to give a white solid. Recrystallization from hot hexanes/butyl chloride gave 210 mg (64% yield) of pure title compound as white needles, mp 141.5-143°C, $^1$H NMR ($CDCl_3$, 300 MHz) δ 1.77 (3H, t, J=17 Hz), 2.45 (3H, s) 3.12 (2H, t, J=4 Hz), 3.29 (2H, t, J=4 Hz), 7.25-7.33 (2H, m), 7.41 (1H, s), 7.43-7.51 (1H, m), 7.64-7.70 (1H, m).

| | |
|---|---|
| Analysis | calcd. for $C_{17}H_{16}F_2N_2O_2$: |
| | C, 64.00; H, 5.07; N, 8.80 |
| Found: | C, 64.14; H, 4.91; N, 9.03 |

EXAMPLE 81

3-[2-(4,7-Dichlorobenzoxazol-2-yl)ethyl]-5-ethyl-6-methylpyridin-2(1H)-one

Step A: Preparation of 2-methoxy-3-[2-(4,7-dichlorobenzoxazol-2-yl)ethenyl]-5-ethyl-6-methyl pyridine

Sodium hydride (60% in mineral oil, 47 mg, 1.15 mmol) was added to a suspension of [(4,7-dichlorobenzoxazol-2-yl)methyl]triphenylphosphonium chloride (521 mg, 1.06 mmol) [prepared by heating 2-chloromethyl-4,7-dichlorobenzoxazole with an equimolar amount of triphenylphosphine in refluxing toluene for 15-25 hours] in dry tetrahydrofuran (8 mL) under a nitrogen atmosphere at 25°C. After 30 minutes, solid 2-methoxy-5-ethyl-6-methyl nictotinaldehyde (197 mg, 1.15 mmol) was added to the yellow suspension. This reaction mixture was heated at reflux for 6 hours. Upon cooling, this reaction was diluted with chloroform, washed with water, dried ($Na_2SO_4$), filtered and the solvent evaporated. This residue was flash chromatographed on silica gel and the product eluted with chloroform to yield 302 mg (83%) of a cis/trans mixture of product as an oil.

Step B: Preparation of 2-methoxy-3-[2-(4,7-dichlorobenzoxazol-2-yl)ethyl]-5-ethyl-6-methyl pyridine

A solution of crude cis/trans 2-methoxy-3-[2-(4,7-dichlorobenzoxazol-2-yl)ethenyl]-5-ethyl-6-methylpyridine (288 mg, 0.79 mmol) in methanol (6 mL) and tetrahydrofuran (6 mL) containing 5% palladium on charcoal (63 mg) was hydrogenated at atmospheric pressure for 5 hours. The catalyst was filtered off and the solution evaporated to yield 260 mg of crude product as an oil. This material was chromatographed on silcia gel and eluted with chloroform to give 103 mg (36%) of title compound as an oil.

Step C: Preparation of 3-[2-(4,7-dichlorobenzoxazol-2-yl)ethyl]-5-ethyl-6-methylpyridin-2(1H)-one

A mixture of crude 2-methoxy-3-[2-(4,7-dichlorobenzoxazol-2-yl)ethyl]-5-ethyl-6-methylpyridine (103 mg, .28 mmol) and pyridine hydrochloride (334 mg, 2.89 mmol) was warmed in a pre-heated oil bath at 140°C for 10 minutes. The cooled residue was diluted with water and the product extracted into chloroform. This extract was dried ($Na_2SO_4$), filtered and the solvent evaporated. This residue was chromatographed on silica gel and eluted with a 0.5 to 1.5% methanol/chloroform gradient to yield 78 mg of pure product. The product was crystallized from diethyl ethermethylene chloride to give 59 mg, m.p. 184-185°C.

Anal. Calcd. for $C_{17}H_{16}Cl_2N_2O_2$
C, 58.13; H, 4.59; N, 7.98.
Found: C, 58.14; H, 4.42; N, 7.92.

EXAMPLE 82

3-[2-(4,7-Dichlorobenzoxazol-2-yl)ethyl]-5-ethyl-6-methylpyridin-2(1H)-thione

A mixture of 3-[2-(4,7-dichlorobenzoxazol-2-yl)ethyl]-5-ethyl-6-methylpyridin-2(1H)-one (493 mg, 1.4 mmol) and 2,4-bis(4-methoxyphenyl)-1,3-dithia-2,4-diphosphetane-2,4-disulfide (Lawesson's Reagent) (664 mg, 1.64 mmol) in dry toluene (10 ml) was refluxed, under a nitrogen atmosphere for 5 hours. Some methanol was added and the solvents were evaporated. The residue was chromatographed on silica gel and eluted with a 0-1.25% methanol/ chloroform gradient to give 458 mg (89%) of title compound. Several recrystallizations from diethyl ether/methylene chloride gave 378 mg of analytically pure compound, mp 243-245°C.

Anal. Calcd. for $C_{17}H_{16}Cl_2N_2OS$
C, 55.59; H, 4.39; N, 7.63.
Found: C, 55.64; H, 4.26; N, 7.63.

EXAMPLE 83

Preparation of 3-[2-(4,7-Difluorobenzoxazol-2-yl)-ethyl]-5-ethyl-6-methylpyridin-2(1H)-one

Following the procedure of Example 63 but substituting 2-chloromethyl-4,7-difluorobenzoxazole [prepared by the procedure of Example 40] for 2-chloromethyl-7-fluorobenzoxazole, one obtains 3-[2-(4,7-difluorobenzoxazol-2-yl)ethyl]-5-ethyl-6-methylpyridin-2(1H)-one, mp 187.5-89° (MeOH).

Anal. Calcd. for $C_{17}H_{16}F_2N_2O_2$:
C, 64.15; H, 5.07; N, 8.80.

Found: C, 63.96; H, 4.93; N, 8.71.

EXAMPLE 84

Preparation of 3-[(6-Ethyl-5-methyl-3-methoxy-2-pyridinylmethyl)amino]-5-ethyl-6-methylpyridin-2(1H)-one

Following procedured described in Example 69 and starting with 3-pentanone, 3-[(6-ethyl-5-methyl-3-methoxy-2-pyridinylmethyl)amino]-5-ethyl-6-methyl-pyridin-2(1H)-one, mp 215-217°C ($CHCl_3$-EtOH), was prepared.
$^1$H NMR ($CDCl_3$) $\delta$ = 6.94 (1H, s), 6.44 (1H,s) 4.36 (2H, br s), 3.85 (3H, s,), 2.78 (2H, q, J=7.5 Hz), 2.36 (2H, q, J=7.6 Hz), 2.30 (3H, s), 2.17 (3H, s), 1.29 (3H, t, J=7.5 Hz), 1.12 (3H, t, J=7.6 Hz).
Anal. Calcd. for $C_{18}H_{25}N_3O_2$:
C, 68.54; H, 7.99; N, 13.32.
Found: C, 68.56; H, 7.93; N, 13.27.

EXAMPLE 85

Preparation of 3-[(3-Methoxy-5,6,7,8-tetrahydro-2-quinolinylmethyl)amino]-5-ethyl-6-methylpyridin-2(1H)-one

Following procedures described in Example 69 and starting with cyclohexanone, 3-[(3-methoxy-5,6,7,8-tetrahydro-2-quinolinylmethyl)amino]-5-ethyl-6-methylpyridin-2(1H)-one, mp 250-53°C ($CHCl_3$-EtOH); was prepared.
$^1$H NMR ($CDCl_3$) $\delta$ = 7.10 (1H, s), 6.61 (1H, s), 4.60 (2H, br s), 3.92 (3H, s), 3.05 (2H, m), 2.78 (2H, m,), 2.38 (2H, q, J = 7.3 Hz), 2.20 (3H, s), 1.85 (4H, m), 1.10 (3H, t, J = 7.3 Hz).
Anal. Calcd. for $C_{19}H_{25}N_3O_2$:
C, 69.70; H, 7.70; N, 12.83.
Found: C, 69.35; H, 7.83; N, 12.70.

EXAMPLE 86

Preparation of3-{[5-(1-Hydroxy-1-ethyl)-6-methyl-2-methoxy-3-pyridinylmethyl]amino}-5-ethyl -6-methylpyridin-2(1H)-one

Step A: Preparation of 5-Acetyl-3-cyano-2-methoxy-6-methylpyridine.

A mixture of 3-cyano-5-acetyl-6-methylpyridin-2(1H)-one (1.76 g, 10 mmol), methyl bromide (1.14 g, 12 mmol) and silver carbonate (3.06 g, 11 mmol) in benzene is protected from light and stirred at room temperature under Ar overnight. Solids are removed by filtration and washed with benzene. The organic solutions are combined and concentrated to give 5-acetyl-3-cyano-2-methoxy-6-methylpyridine.

Step B: Preparation of 3-cyano-5-(1-hydroxy-1-ethyl)-2-methoxy-6-methylpyridine

Sodium borohydride (0.35 g, 9.2 mmol) is added at room temperature to 5-acetyl-3-cyano-2-methoxy-6-methylpyridine (1.64 g, 9.2 mmol) in EtOH (60 mL). After 2 hours, $CHCl_3$, containing a few drops of HOAc, is added. The mixture is washed with brine, dried ($Na_2SO_4$), filtered and concentrated to give the alcohol.

Step C: Preparation of 5-(1-Hydroxy-1-ethyl)-2-methoxy-6-methylnicotinaldehyde

Diisobutylaluminumhydride (5.22 mL of a 1.5 M solution in toluene, 7.8 mmol) is added dropwise to a stirred mixture of 3-cyano-5-(1-hydroxy-1-ethyl)-2-methoxy-6-methylpyridine (0.67 g, 3.7 mmol) in toluene (25 mL) cooled in an ice-acetone bath under Ar. After stirring for 1 hour, the reaction is quenched by pouring into a mixture of 1N HCl and ice. Extraction with $CHCl_3$ followed by washing with saturated $NaHCO_3$ solution and brine and evaporation gives the aldehyde

Step D: Preparation of 3{[5-(1-Hydroxy-1-ethyl)-6-methyl-2-methoxy-3-pyridiny1methyl]amino}-5-ethyl-6-methylpyridin-2(1H)-one

A solution of 3-amino-5-ethyl-6-methylpyridin-2(1H)-one (140 mg, 0.92 mmol) and the aldehyde of Step C

(180 mg, 0.92 mmol) in MeOH (5.0 mL) is treated with 1 drop of glacial HOAc. After stirring at room temperature for 1 hour, the yellow solid is removed by filtration and suspended in a mixture of MeOH (40 mL) and CHCl3 (15 mL). Sodium borohydride (60 mg) is added and after stirring for 30 minutes the mixtured is filtered. The filtrate is concentrated and the residue flash chromatographed over silica gel. Elution with 87 MeOH-927 $CHCl_3$ gives pure 3{[5-(1-Hydroxy-1-ethyl)-6-methyl-2-methoxy-3-pyridinylmethyl]amino}-5-ethyl-6-methylpyridin-2(1H)-one.

EXAMPLE 87

Preparation of 6-Aminomethyl-3-[((4,7-dichlorobenzoxazol-2-yl)methyl)aminol-5-ethylpyridin-2(1H)-one

Step A: Preparation of 6-Azidomethyl-3-[((4,7-dichlorobenzoxazol-2-yl)methyl)amino]-5-ethylpyridin-2(1H)-one

To a solution of 3-[((4,7-dichlorobenzoxazol-2-yl)methyl)amino]-5-ethyl-6-hydroxymethylpyridin-2(1H)-one (368 mg, 1.0 mmol) in THF (5 mL) is added 1.1 mL of a 1M solution of hydrazoic acid in benzene followed by a solution of diisopropyl azodicarboxylate (222 mg, 1.1 mmol) in THF (5 mL). To this mixture is added a solution of triphenylphosphine (288 mg, 1.1 mmol) in THF (3 mL) with stirring. After 1 hour at room temperature, the mixture is heated at 50° for 3 hours. Solvents are removed under reduced pressure and the residue partitioned between $CH_2Cl_2$ and water. The organic layer is dried ($Na_2SO_4$), filtered and concentrated. Flash chromatography of the residue over silica gel gives pure 6-azidomethyl product.

Step B: Preparation of 6-Aminomethyl-3-[((4,7-dichlorobenzoxazol-2-yl)methyl)amino]-5-ethylpyridin-2(1H)-one

A mixture of 6-azidomethyl-3-[((4,7-dichlorobenzoxazol-2-yl)methyl)amino]-5-ethylpyridin-2(1H)-one (197 mg, 0.50 mmol) and triethylammonium trisphenylthiostannite (0.75 mmol) (prepared from tin (II) phenylmercaptide, thiophenol and triethylamine) in benzene (5 mL) is stirred at 15° for 30 minutes. Methylene chloride (20 mL) is added and the diluted reaction mixture washed with 0.1N NaOH. After drying the organic extract ($Na_2SO_4$), filtering and concentrating, the residue is flash chromatograhed over silica gel to give 6-aminomethyl-3-[((4,7-dichlorobenzoxazol-2-yl)methyl)amino]-5-ethylpyridin-2(1H)-one.

EXAMPLE 88

Preparation of 6-Aminomethyl-3-[((4,7-dimethylbenzoxazol-2-yl)methyl)amino]-5-ethylpyridin-2(1H)-one, 6-Aminomethyl-3-[4,5-dimethyl-2-methoxybenzylamino]-5-ethylpyridin-2(1H)-one, 6-Aminomethyl-3-[5-ethyl-2-methoxybenzylamino]-5-ethylpyridin-2(1H)-one, 6-Aminomethyl-3-[(5-ethyl-6-methyl-2-methoxy-3-pyridinylmethyl)amino]-5-ethylpyridin-2(1H)-one and 6-Aminomethyl-3-[(5,6-dimethyl-3-methoxy-2-pyridinylmethyl)-aminol-5-ethylpyridin-2(1H)-one

Substitution of 6-hydroxymethyl-3-[((4,7-dimethylbenzoxazol-2-yl)methyl)amino]-5-ethylpyridin-2(1H)-one, 6-hydroxymethyl-3-[4,5-dimethyl-2-methoxybenzylamino]-5-ethylpyridin-2(1H)-one, 6-hydroxymethyl-3-[5-ethyl-2-methoxybenzylamino]-5-ethylpyridin-2(1H)-one, 6-hydroxymethyl-3-[(5-ethyl-6-methyl-2-methoxy-3-pyridiny1methyl)amino)]-5-ethylpyridin-2(1H)-one and 6-hydroxymethy1-3-[(5,6-dimethyl-3-methoxy-2-pyridinylmethyl)amino]-5-ethylpyridin-2(1H)-one for 3-[((4,7-Dichlorobenzoxazol2-yl)methyl)amino]-5-ethyl-6-hydroxyethylpyridin2(1H)-one in Example 87 and following the procedure of this example yields the title compounds.

EXAMPLE 89

Preparation of 3-[N-(5,6-dimethyl-2-methoxy-3-pyridylmethyl)aminol-5-ethyl-6-methyl-2(1H)-pyridinone

By the procedure set forth in Example 20, the title compound is obtained.

EXAMPLE 90

Preparation of 3-[(4-methylbenzoxazol-2-yl)ethyl]-5-ethyl-6-methylpyridin-2(1H)-one

By the procedure set forth in Example 6, the title compound is obtained.

EXAMPLE 91

Preparation of 3-[(4-fluorobenzoxazol-2-yl)ethyl]-5-ethyl]-6-methylpyridin-2(1H)one

The title compound, mp 155.5-56.6°, was prepared from 2-chloromethyl-4-fluorobenzoxazole by the procedure of Example 6.

Anal. Calcd. for $C_{17}H_{17}FN_2O_2$:
C, 67.99; H, 5.71; N, 9.33
Found: C, 67.84; H, 5.62; N, 9.32

EXAMPLE 92

Preparation of 3-[(4-chlorobenzoxazo1-2-yl)ethyl]-5-ethyl]-6-methylpyridin-2(1H)one

The title compound is prepared from 2-chloromethyl-4-chlorobenzoxazole by the procedure of Example 6.

REVERSE TRANSCRIPTASE ASSAY

The assay measures the incorporation of tritiated deoxyguanosine monophosphate by recombinant HIV reverse transcriptase (HIV RTR) (or other RT) into acid-precipitable cDNA at the Km values of dGTP and poly r(C)•oligo $d(G)_{12-18}$. The inhibitors of the present invention inhibit this incorporation.

Thirty uL of a reaction mixture containing equal volumes of: 500 mM Tris•HCl (pH 8.2), 300 mM $MgCl_2$, 1200 mM KCl, 10 mM DTT, 400 μg/mL poly r(c)•oligo d(G) [prepared by dissolving 1.5 mg (25 U) poly r(C)•oligo d(G) in 1.5 ml sterile distilled $H_2O$ and diluting to 400 μg/ml], 0.1 μCi/μl [$^3$H] dGTP, 160 μM dGTP, was added to 10 μl sterile distilled $H_2O$, 2.5 μl of potential inhibitor and 10 μL of 5 nM purified HIV $RT_R$ in tubes. The mixture was incubated at 37°C for 45 minutes.

After incubation is complete, the tubes were cooled in ice for 5 minutes. Ice-cold 13% TCA containing 10 mM $NaPP_i$ (200 μl) are added and the mixture incubated on ice for 30 minutes. The precipitated cDNA is removed by filtration using presoaked glass filters [TCA, $NaPP_i$]. The precipitate is then washed with 1N HCl, 10 mM $NaPP_i$.

The filter discs are then counted in a scintillation counter.

Under these conditions [dGTP] and poly r(C)•oligo $d(G)_{12-18}$ each are approximately equal to the appropriate Km value. Approximately 5-6,000 cpm of [$^3$H] GMP are incorporated into acid-precipitable material. The RT reaction is concentration- and time-dependent. DMSO (up to 5%) does not affect enzyme activity. Calculated $IC_{50}$ values for the tested compounds of this invention vary from about 10 nM to more than 300 μM. The $IC_{50}$ values of the most preferred compounds range from about 7 nM to about 30 nM

INHIBITION OF VIRUS SPREAD

A. Preparation of HIV-infected MT-4 Cell Suspension.

MT cells were infected at Day 0 at a concentration of 250,000 per ml with a 1:2000 dilution of HIV-1 strain IIIb stock (final 125 pg p24/ml; sufficient to yield ≦ 1% infected cells on day 1 and 25-100% on day 4). Cells were infected and grown in the following medium: RPMI 1640 (Whittaker BioProducts), 10% inactivated fetal bovine serum, 4 mM glutamine (Gibco Labs) and 1:100 Penicillin-Streptomycin (Gibco Labs).

The mixture was incubated overnight at 37°C in 5% $CO_2$ atmosphere.

B. Treatment with Inhibitors

Serial two-fold dilutions of compound were prepared in cell culture medium. At Day 1, aliquots of 125 μl of compound were added to equal volumes of HIV-infected MT-4 cells (50,000 per well) in a 96-well microtiter

cell culture plate. Incubation was continued for 3 days at 37°C in 5% $CO_2$ atmosphere.

C. Measurement of Virus Spread

Using a multichannel pipettor, the settled cells were resuspended and a 125 µl harvested into a separate microtiter plate. After the settling of the cells, the plates were frozen for subsequent assay of the supernatant for HIV p24 antigen.

The concentration of HIV p24 antigen was measured by an enzyme immunoassay, described as follows. Aliquots of p24 antigen to be measured were added to microwells coated with a monoclonal antibody specific for HIV core antigen. The microwells were washed at this point, and at other appropriate steps that follow. Biotinylated HIV-specific antibody was then added, followed by conjugated streptavidinhorseradish peroxidase. A color reaction occurs from the added hydrogen peroxide and tetramethylbenzidine substrate. Color intensity is proportional to the concentration of HIV p24 antigen.

The cell culture inhibitory concentration ($CIC_{95}$) for each compound is defined as that concentration which inhibited by greater than 95% the spread of infection, as assessed by a greater than 95% reduction in p24 antigen production relative to untreated controls. The tested compounds of the present invention were found to have $CIC_{95}$ values ranging from about 6 nM to about 100 nM for preferred species, and up to about 40 µM for others.

While the foregoing specification teaches the principles of the present invention, with examples provided for the purpose of illustration, it will be understood that the practice of the invention encompasses all of the usual variations, adaptations, and modifications, as come within the scope of the following claims and its equivalents.

## Claims

1. A compound of the formula

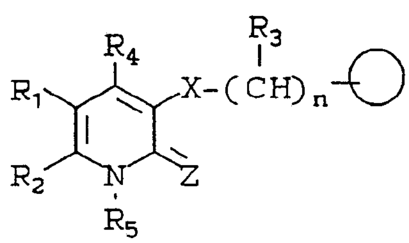

I

wherein
X is

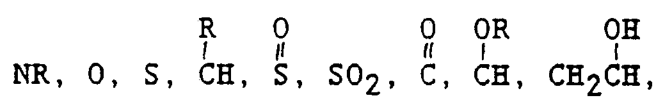

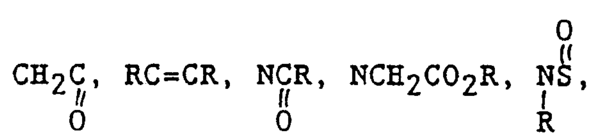

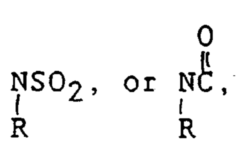 where R is H, $C_{1-8}$ alkyl,

where R is H, $C_{1-8}$ alkyl, $C_{1-8}$ alkenyl or $C_{3-8}$ cycloalkyl;

Z is 0, S or $NR_x$ is H or $C_{1-8}$ alkyl
n is 0-4;
$R_1$, $R_2$ and $R_4$ are the same or different and are independently
(i) H;
(ii) $C_{1-8}$ alkyl, $C_{1-8}$ alkenyl, $C_{3-8}$ cycloalkyl, any of which is unsubstituted or substituted with one or two of $C_{1-3}$ alkoxy, $C_{1-4}$ alkylamino, di($C_{1-4}$ alkyl)amino, $C_{1-3}$ alkylthio, hydroxy, amino, carbonyl, aminocarbonyl, or oximido, or one to five of halo;
(iii) $C_{1-5}$ alkylthio;
(iv) $C_{1-5}$ alkylsulfinyl;
(v) $C_{1-5}$ alkylsulfonyl;
(vi) $C_{1-5}$ alkoxy;
(vii) $C_{1-5}$ alkoxycarbonyl;
(viii) cyano;
(ix) halo; or
(x) aryl;
or $R_1$ and $R_4$ may together form a cycloalkyl ring containing 5-7 members;
or $R_1$ and $R_2$ may together form a cycloalkyl ring containing 5-7 members;
and $R_3$ or $R_5$ are the same or different and are independently
(i) H;
(ii) $C_{1-8}$ alkyl;
(iii) $C_{1-8}$ alkenyl;
(iv) $C_{3-8}$ cycloalkyl;

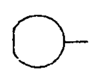

is aryl or heterocycle each unsubstituted or substituted with one or more of
(i) $C_{1-6}$ alkyl unsubstituted or substituted with one or more of A, wherein A is halo, hydroxy, hydroxy-$C_{1-4}$ alkyl, amino, $C_{1-6}$ alkylamino, di($C_{1-6}$ alkyl)amino, $C_{1-6}$ alkoxy or aryl; (ii) $C_{1-6}$ alkenyl unsubstituted or substituted with one or more of A; (iii) C3,6 cycloalkyl unsubstituted or substituted with one or more of A; (iv) $C_{1-6}$ alkoxy unsubstituted or substituted with one or more of A; (v) aryl; (vi) amino; (vii) $C_{1-6}$ alkylamino; (viii) di($C_{1-8}$ alkyl)amino; (ix) amino-$C_{1-8}$ alkyl; (x) $C_{1-8}$ alkyl-amino-$C_{1-8}$ alkyl; (xi) di-($C_{1-6}$ alkyl)amino $C_{1-8}$ alkyl; (xii) $C_{1-6}$ alkoxycarbonyl; (xiii) aminocarbonyl; (xiv) $C_{1-6}$ alkyl aminocarbonyl; (xv) di($C_{1-6}$ alkyl)aminocarbonyl; (xvi) $C_{1-6}$ alkylthio; (xvii) $C_{1-6}$ alkylsulfinyl; (xviii) $C_{1-6}$ alkylsulfonyl; (xix) hydroxy; (xx) halo; (xxi) CN, or (xxii) $NO_2$,
with the provisos that
I. $R_1$ or $R_2$ or both are not substituted with OH; and
II. heterocycle is not phthalimide;
or pharmaceutically acceptable salt, hydrate or ester thereof.

2. A compound of Claim 1, of the formula

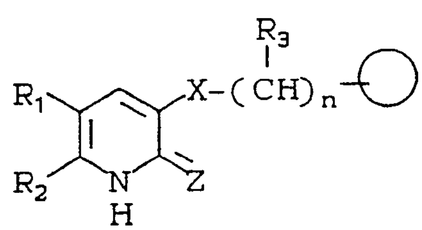

II

wherein
X is NH, O, S, or $CH_2$;
Z is O or S;
n is 1-4;

$R_1$ is

(i) $C_{1-8}$ alkyl, unsubstituted or substituted with one or two of $C_{1-3}$alkoxy, halo, amino, $C_{1-4}$alkylamino, $C_{1-4}$-di-(alkyl)amino, or
$C_{1-3}$ alkylthio;
(ii) $C_{1-3}$ alkylthio;
(iii) $C_{1-3}$ alkoxy; or
(iv) halo;

$R_2$ is H, methyl or ethyl, unsubstituted or substituted with one or two of methoxy, amino, methylamino, dimethylamino or methylthio;

$R_3$ is H or $C_{1-8}$ alkyl;

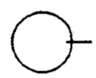

is aryl or heterocycle, each unsubstituted or substituted with one or more of

(a) $C_{1-6}$ alkyl,
(b) $C_{1-6}$ alkoxy unsubstituted or substituted with hydroxy-$C_{1-4}$ alkyl,
(c) amino,
(d) $C_{1-6}$ alkyl amino,
(e) di($C_{1-6}$ alkyl)amino,
(f) amino- $C_{1-8}$ alkyl,
(g) $C_{1-8}$ alkyl-amino-$C_{1-8}$ alkyl,
(h) di-($C_{1-6}$ alkyl)amino $C_{1-8}$ alkyl,
(i) hydroxyl,
(j) halogen,
(k) CN, or
(l) $NO_2$.

**3.** A compound of Claim 2, wherein
$R_1$ is $C_{1-4}$ alkyl;
$R_2$ is H or $CH_3$;
aryl is

(a) naphthyl, unsubstituted or substituted with one or more of $C_{1-6}$ alkyl, halogen or hydroxyl;
(b) phenyl, unsubstituted or substituted with one or more of $C_{1-6}$ alkyl, $C_{1-6}$ alkoxy, halogen or hydroxyl; or
(c) biphenyl.

**4.** A compound of Claim 2, wherein
aryl is

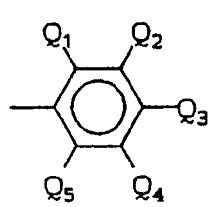

wherein
$Q_1$ is $C_{1-2}$alkoxy;
$Q_2$, $Q_3$, $Q_4$ and $Q_5$ are independently selected from
H, $C_{1-3}$alkyl, $C_{1-2}$alkoxy, halogen, CN or $NO_2$.

**5.** A compound of Claim 3, wherein aryl is 2-naphthyl, 2-naphthyl substituted with methyl, 2-naphthyl substituted with chloro; phenyl; 2-hydroxyphenyl, 2-hydroxyphenyl substituted with methyl, 2-hydroxyphenyl

substituted with chloro, 2-methoxy-5-ethylphenyl, 2-methoxy-4,5-dimethylphenyl, or 2,6-dimethoxyphenyl.

6. A compound of Claim 3, wherein aryl is 2-naphthyl, phenyl or 2-hydroxyphenyl.

7. A compound of Claim 5 wherein aryl is 2-methoxy-5-ethylphenyl, 2-methoxy-4,5-dimethylphenyl, or 2,6-dimethoxyphenyl.

8. A compound as in any of Claims 1-2 wherein heterocycle is thienyl, oxazolyl, thiazolyl, triazolyl, pyridyl unsubstituted or substituted with one or more of $C_{1-2}$alkoxy or $C_{1-3}$alkyl or hydroxyalkyl, pyridinonyl, pyrazinyl, benzothienyl, quinolinyl, 5,6,7,8-tetrahydroquinolinyl, isoquinolinyl, benzimidazolyl, benzofuranyl, benzothiazolyl, benzoxazolyl unsubstituted or substituted with one or more of $C_{1-6}$ alkyl or halogen or hydroxyl, 4,5,6,7-tetrahydrobenzoxazolyl, naphth [3,2-d] oxazolyl, oxazolo [4,5-b] pyridyl, chromone, or benzopyrimidinone.

9. A compound of Claim 8 wherein heterocycle is 2-oxazolyl, 2-pyridyl, 3-pyridyl unsubstituted or substituted with one or more of $C_{1-2}$alkoxy or C1-3lalkyl, 2-benzothienyl, 2-quinolinyl, 2-benzimidazolyl, 2-benzofuranyl, 2-benzothiazolyl, 2-oxazolo [4,5-b] pyridyl, or 2-benzoxazolyl unsubstituted or substituted with one or more of methyl, halogen or hydroxyl.

10. A compound of Claim 9 wherein heterocycle is 2-methoxy-5,6-dimethyl-3-pyridyl, 2-methoxy-5-ethyl-6-methyl-3-pyridyl, 3-methoxy-5,6-dimethyl-2-pyridyl, 2-benzoxazolyl, 7-methyl-2-benzoxazolyl, 4,7-dimethyl-2-benzoxazolyl, 7-chloro-2-benzoxazolyl, 4,7-dichloro-2-benzoxazolyl, 4,7-difluoro-2-benzoxazolyl, 4-fluoro-2-benzoxazolyl, or 4-chloro-2-benzoxazolyl.

11. The Compound,
3-{[(4,7-dichlorobenzoxazol-2-yl)methyl]amino}-5-ethyl-6-methyl-2-(1H)-pyridinone,
3-{[(4,7-dimethylbenzoxazol-2-yl)methyl]amino}-5-ethyl-6-methyl-2-(1H)-pyridinone,
3-{[(7-chlorobenzoxazol-2-yl)methyl]amino}-5-ethyl-6-methyl-2-(1H)-pyridinone,
3-{[(7-methylbenzoxazol-2-yl)methyl]amino}-5-ethyl-6-methyl-2-(1H)-pyridinone,
3-{[(4-fluorobenzoxazol-2-yl)methyl]amino}-5-ethyl-6-methyl-2-(1H)-pyridinone,
3-{[(7-fluorobenzoxazol-2-yl)methyl]amino}-5-ethyl-6-methyl-2-(1H)-pyridinone, 3-{[(benzoxazol-2-yl)methyl]amino}-5-ethyl-6-methyl-2-(1H)-pyridinone,
3-{[(4-chlorobenzoxazol-2-yl)methyl]amino}-5-ethyl-6-methyl-2-(1H)-pyridinone,
3-{[(4-fluoro-7-chlorobenzoxazol-2-yl)methyl]amino}-5-ethyl-6-methyl-2-(1H)-pyridinone,
3-[2-(4,7-Dichlorobenzoxazol-2-yl)ethyl]-5-ethyl-6-methyl-2-(1H)-pyridinone,
3-[2-(4,7-Dichlorobenzoxazol-2-yl)ethyl]-5-ethyl-6-methyl-2-(1H)-pyridinone,
3-[2-(benzoxazol-2-yl)ethyl]-5-ethyl-6-methyl-2-(1H)pyridinthione,
3-[2-(benzoxazol-2-yl)ethyl]-5-ethyl-6-methyl-2-(1H)-pyridinone,
3-[2-(4,7-dimethylbenzoxazol-2-yl)ethyl]-5-ethyl-6-methyl-2-(1H)-pyridinone,
3-[2-(4-methylbenzoxazol-2-yl)ethyl]-5-ethyl-6-methyl-2-(1H)-pyridinone,
3-[2-(7-methylbenzoxazol-2-yl)ethyl]-5-ethyl-6-methyl-2-(1H)-pyridinone,
3-[N-(5-Ethyl-2-methoxy-6-methyl-3-pyridylmethyl)-amino]-5-ethyl-6-methyl-2(1H)-pyridinone,
3-[N-(5-(2-Hydroxyethyl)-2-methoxy-6-methyl-3-pyridylmethyl)amino]-5-ethyl-6-methyl-2(1H)-pyridinone,
3-[N-(5-(1-Hydroxyethyl)-2-methoxy-6-methyl-3-pyridylmethyl)amino]-5-ethyl-6-methyl-2(1H)-pyridinone,
3-[N-(5,6-Dimethyl-2-methoxy-3-pyridylmethyl)amino]-5-ethyl-6-methyl-2(1H)-pyridinone,
3-[N-(5-Ethyl-2-methoxybenzyl)amino]-5-ethyl-6-methyl-2(1H)-pyridinone,
3-[N-(2-Methoxy-4,5-dimethylbenzyl)amino]-5-ethyl-6-methyl-2(1H)-pyridinone,
3-[N-(2,6-dimethoxybenzyl)amino]-5-ethyl-6-methyl-2(1H)-pyridinone,
3-{[(4,7-dichlorobenzoxazol-2-yl)methyl]amino}-5-methylthio-6-methyl-2(1H)-pyridinone,
3-{[(4,7-dichlorobenzoxazol-2-yl)methyl]thio}-5-ethyl-6-methyl-2(1H)-pyridinone,
3-[N-(2-methoxy-5-methylbenzyl)amino]-5-ethyl-6-methyl-2-(1H)-pyridinone,
3-[(5-ethyl-2-methoxy-6-methyl-3-pyridinylmethyl)-amino]-S-cyclopropyl-6-methyl-2(1H)-pyridinone,
3-[N-(2-methoxy-4-methylbenzyl)amino]-5-ethyl-6-methyl-2-(1H)-pyridinone,
3-{N-[(4,7-dichlorobenzoxazol-2-yl)methyl]-N-methyl-amino}-5-ethyl-6-methyl-2-(1H)-pyridinone,
3-[2-(4,7-dichlorobenzoxazol-2-yl)ethyl]-5-propyl-6-methyl-2-(1H)-pyridinone,
3-[2-(4,7-dichlorobenzoxazol-2-yl)ethyl]-5-ethyl-6-methyl-pyridin-2(1H)-thione, or

3-[2-(7-fluorobenzoxazol-2-yl)ethyl]-5-ethyl-6-methyl-pyridin-2(1H)-one, or pharamceutically acceptable ester thereof.

12. A compound, which is
3-{[(4,7-dichlorobenzoxazol-2-yl)methyl]amino}-5-ethyl-6-methyl-2(1H)-pyridinone,
3-{[(4,7-dimethylbenzoxazol-2-yl)methyl]amino}-5-ethyl-6-methyl-2(1H)-pyridinone,
3-{[(7-chlorobenzoxazol-2-yl)methyl]amino}-5-ethyl-6-methyl-2(1H)-pyridinone,
3-{[(7-methylbenzoxazol-2-yl)methyl]amino}-5-ethyl-6-methyl-2(1H)-pyridinone,
3-[2-(4,7-Dichlorobenzoxazol-2-yl)ethyl]-5-ethyl-6-methyl-2(1H)-pyridinone,
3-[2-(4,7-Difluorobenzoxazol-2-yl)ethyl]-5-ethyl-6-methyl-2(1H)-pyridinone,
3-[2-(4-fluorobenzoxazol-2-yl)ethyl]-5-ethyl-6-methyl-2(1H)-pyridinone,
3-[2-(4-chlorobenzoxazol-2-yl)ethyl]-5-ethyl-6-methyl-2(1H)-pyridinone,
3-[2-(benzoxazol-2-yl)ethyl]-5-ethyl-6-methyl-2(1H)-pyridinone,
3-[2-(benzoxazol-2-yl)ethyl]-5-ethyl-6-methyl-2(1H)-pyridinthione,
3-[N-(5-Ethyl-2-methoxy-6-methyl-3-pyridylmethyl)-amino]-5-ethyl-6-methyl-2(1H)-pyridinone,
3-[N-(5,6-Dimethyl-2-methoxy-3-pyridylmethyl)-amino]-5-ethyl-6-methyl-2(1H)-pyridinone,
3-[N-(5-Ethyl-2-methoxybenzyl)amino]-5-ethyl-6-methyl-2(1H)-pyridinone,
3-[N-(2-Methoxy-4,5-dimethylbenzyl)amino]-5-ethyl-6-methyl-2(1H)-pyridinone, or
3-[N-(2,6-Dimethoxybenzyl)amino]-5-ethyl-6-methyl-2(1H)-pyridinone,
or pharmaceutically acceptable esters thereof.

13. A compound, which is
3-[N-(5-Ethyl-2-methoxybenzyl)amino]-5-ethyl-6-methyl-2(1H)-pyridinone,
3-[N-(2-Methoxy-4,5-dimethylbenzyl)amino]-5-ethyl-6-methyl-2(1H)-pyridinone,
3-[N-(5,6-Dimethyl-2-methoxy-3-pyridylmethyl)amino]-5-ethyl-6-methyl-2(1H)-pyridinone,
3-[2-(4,7-Dichlorobenzoxazol-2-yl)ethyl]-5-ethyl-6-methyl-2(1H)-pyridinone,
3-{[(4,7-dichlorobenzoxazol-2-yl)methyl]thio}-5-ethyl-6-methyl-2(1H)-pyridinone,
3-[2-(benzoxazol-2-yl)ethyl]-5-ethyl-6-methyl-2(1H)-pyridinthione,
3-[2-(4,7-Difluorobenzoxazol-2-yl)ethyl]-5-ethyl-6-methyl-2-(1H)-pyridinone,
3-[N-(5-Ethyl-2-methoxy-6-methyl-3-pyridylmethyl)-amino]-5-ethyl-6-methyl-2(1H)-pyridinone,
3-[2-(4,7-Dichlorobenzoxazol-2-yl)ethyl]-5-ethyl-6-methyl-pyridin-2(1H)thione,
3-[2-(4-methylbenzoxazol-2-yl)ethyl]-5-ethyl-6-methyl-2-(1H)-pyridinone,
3-{[(4,7-dimethylbenzoxazol-2-yl)methyl]amino}-5-ethyl-6-methyl-2-(1H)-pyridinone,
3-{[(4,7-dichlorobenzoxazol-2-yl)methyl]amino}-5-ethyl-6-methyl-2-(1H)-pyridinone,
3-[2-(7-fluorobenzoxazol-2-yl)ethyl]-5-ethyl-6-methylpyridin-2-(1H)-one,
3-[2-(benzoxazol-2-yl)ethyl]-5-ethyl-6-methyl-2-(1H)-pyridinone,
3-[N-(2-methoxy-5-methylbenzyl)amino]-5-ethyl-6-methyl-2-(1H)-pyridinone,
3-[2-(4,7-dimethylbenzoxazol-2-yl)ethyl]-5-ethyl-6-methyl-2-(1H)-pyridinone,
3-[(5-ethyl-2-methoxy-6-methyl-3-pyridinylmethyl)-amino]-5-cyclopropyl-6-methyl-2-(1H)-pyridinone,
3-{[(4,7-dichlorobenzoxazol-2-yl)methyl]amino}-5-methylthio-6-methyl-2(1H)-pyridinone,
3-[N-(2-methoxy-4-methylbenzyl)amino]-5-ethyl-6-methyl-2-(1H)-pyridinone,
3-[N-(2,6-dimethoxybenzyl)amino]-5-ethyl-6-methyl-2(1H)-pyridinone,
3-{N-[(4,7-dichlorobenzoxazol-2-yl)methyl]-N-methyl-amino}-5-ethyl-6-methyl-2(1H)-pyridinone, or
3-[2-(4,7-dichlorobenzoxazol-2-yl)ethyl]-5-propyl-6-methyl-2(1H)-pyridinone.
or pharmaceutically acceptable esters thereof.

14. The use of a compound as claimed in any of Claims 1 to 13 for the manufacture of a medicament for inhibiting HIV reverse transcriptase.

15. The use of a compound as claimed in any of Claims 1 to 13 for the manufacture of a medicament for the treatment of infection by HIV, or the treatment of AIDS or ARC.

16. A pharmaceutical composition useful for inhibiting HIV reverse transcriptase, comprising an effective amount of a compound as in any of Claims 1-13, and a pharmaceutically acceptable carrier.

17. A pharmaceutical composition useful for preventing or treating infection of HIV or for treating AIDS or ARC, comprising an effective amount of a compound a$ in any of Claims 1-13, and a pharmaceutically acceptable carrier.

**18.** Process for synthesizing a compound of formula III,

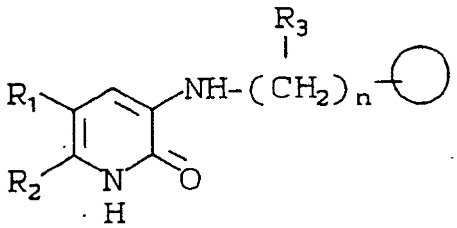

III

wherein
n is 1-4;
$R_1$ is
(i) $C_{1-8}$ alkyl, unsubstituted or substituted with one or two of $C_{1-3}$alkoxy, halo, amino, $C_{1-4}$alkylamino, $C_{1-4}$-di-(alkyl)amino, or $C_{1-3}$ alkylthio;
(ii) $C_{1-3}$ alkylthio;
(iii) $C_{1-3}$ alkoxy; or
(iv) halo;
$R_2$ is H, methyl or ethyl, unsubstituted or substituted with one or two of methoxy, amino, methylamino, dimethylamino or methylthio;
$R_3$ is H or $C_{1-8}$ alkyl;

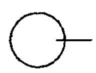

is aryl or heterocycle, each unsubstituted or substituted with one or more of
(a) $C_{1-6}$ alkyl,
(b) $C_{1-8}$ alkoxy unsubstituted or substituted with hydroxy-$C_{1-4}$ alkyl,
(c) amino,
(d) $C_{1-6}$ alkyl amino,
(e) di($C_{1-6}$ alkyl)amino,
(f) amino- $C_{1-8}$ alkyl,
(g) $C_{1-8}$ alkyl-amino-$C_{1-8}$ alkyl,
(h) di-($C_{1-6}$ alkyl)amino $C_{1-8}$ alkyl,
(i) hydroxyl,
(j) halogen,
(k) CN, or
(l) $NO_2$,
with the proviso that heterocycle is not phthalimide;
comprising the step of condensing

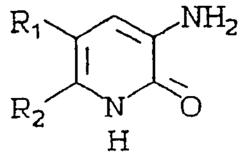

1

and

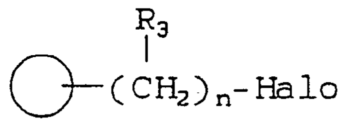

2

by alkylation to yield a compound of Formula III.

**19.** A process for synthesizing a compound of Formula III,

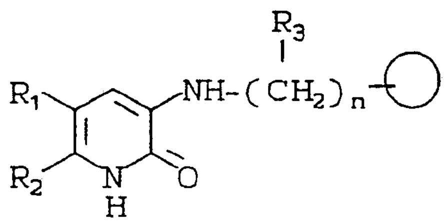

III

wherein
n is 1-4;
$R_1$ is
(i) $C_{1-8}$ alkyl, unsubstituted or substituted with one or two of $C_{1-3}$alkoxy, halo, amino, $C_{1-4}$alkylamino, $C_{1-4}$-di-(alkyl)amino, or
$C_{1-3}$ alkylthio;
(ii) $C_{1-3}$ alkylthio;
(iii) $C_{1-3}$ alkoxy; or
(iv) halo;
$R_2$ is H, methyl or ethyl, unsubstituted or
substituted with one or two of methoxy, amino, methylamino, dimethylamino or methylthio;
$R_3$ is H or $C_{1-8}$ alkyl;

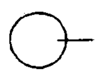

is aryl or heterocycle, each unsubstituted or substituted with one or more of
(a) $C_{1-6}$ alkyl,
(b) $C_{1-6}$ alkoxy unsubstituted or
substituted with hydroxy-$C_{1-4}$ alkyl,
(c) amino,
(d) $C_{1-6}$ alkyl amino,
(e) di($C_{1-6}$ alkyl)amino,
(f) amino- $C_{1-8}$ alkyl,
(g) $C_{1-8}$ alkyl-amino-$C_{1-8}$ alkyl,
(h) di-($C_{1-6}$ alkyl)amino $C_{1-8}$ alkyl,
(i) hydroxyl,
(j) halogen,
(k) CN, or
(l) $NO_2$.
with the proviso that heterocycle is not phthalimide;
comprising the step of condensing

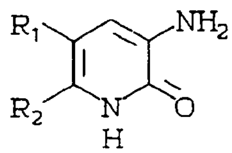

1

and

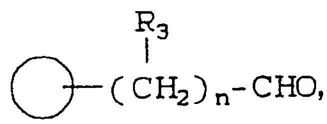

3

by reductive alkylation to give a compound of Formula III.

**20.** A process for synthesizing a compound of Formula IV,

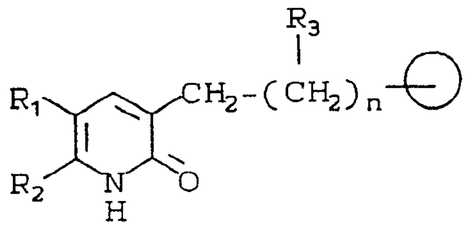

IV

wherein
n is 1-4;
$R_1$ is
(i) $C_{1-8}$ alkyl, unsubstituted or substituted with one or two of $C_{1-3}$alkoxy, halo, amino, $C_{1-4}$alkylamino, $C_{1-4}$-di-(alkyl)amino, or $C_{1-3}$ alkylthio;
(ii) $C_{1-3}$ alkylthio;
(iii) $C_{1-3}$ alkoxy; or
(iv) halo;
$R_2$ is H, methyl or ethyl, unsubstituted or substituted with one or two of methoxy, amino, methylamino, dimethylamino or methylthio;
$R_3$ is H or $C_{1-8}$ alkyl;

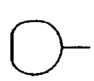

is aryl or heterocycle, each unsubstituted or substituted with one or more of
(a) $C_{1-6}$ alkyl,
(b) $C_{1-6}$ alkoxy unsubstituted or substituted with hydroxy-$C_{1-4}$ alkyl,
(c) amino,
(d) $C_{1-6}$ alkyl amino,
(e) di($C_{1-8}$ alkyl)amino,
(f) amino- $C_{1-8}$ alkyl,
(g) $C_{1-8}$ alkyl-amino-$C_{1-8}$ alkyl,
(h) di-($C_{1-6}$ alkyl)amino $C_{1-8}$ alkyl,
(i) hydroxyl,
(j) halogen,

(k) CN, or
(l) $NO_2$ .

with the proviso that heterocycle is not phthalimide;
comprising conversion of the intermediate

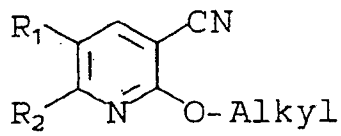

to the intermediate

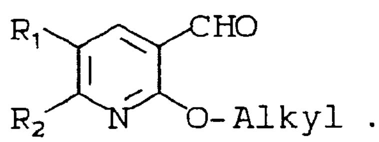

**21.** A process for synthesizing a compound of Formula IV,

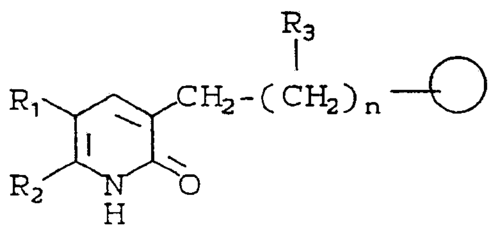

IV

wherein
n is 1-4;
$R_1$ is

(i) $C_{1-8}$ alkyl, unsubstituted or substituted with one or two of $C_{1-3}$alkoxy, halo, amino, $C_{1-4}$alkylamino, $C_{1-4}$-di-(alkyl)amino, or $C_{1-3}$ alkylthio;
(ii) $C_{1-3}$ alkylthio;
(iii) $C_{1-3}$ alkoxy; or
(iv) halo;

$R_2$ is H, methyl or ethyl, unsubstituted or substituted with one or two of methoxy, amino, methylamino, dimethylamino or methylthio;
$R_3$ is H or $C_{1-8}$ alkyl;

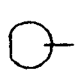

is aryl or heterocycle, each unsubstituted or substituted with one or more of

(a) $C_{1-8}$alkyl,
(b) $C_{1-8}$ alkoxy unsubstituted or substituted with hydroxy-$C_{1-4}$ alkyl,
(c) amino,
(d) $C_{1-6}$ alkyl amino,
(e) di($C_{1-6}$ alkyl)amino,
(f) amino- $C_{1-8}$ alkyl,
(g) $C_{1-8}$ alkyl-amino-$C_{1-8}$ alkyl,
(h) di-($C_{1-6}$ alkyl)amino $C_{1-8}$ alkyl,
(i) hydroxyl,

(j) halogen,
(k) CN, or,
(l) $NO_2$.

with the proviso that heterocycle is not phthalimide;
comprising conversion of the intermediate

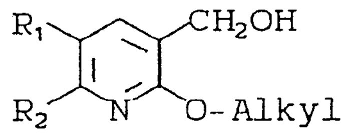

to the intermediate

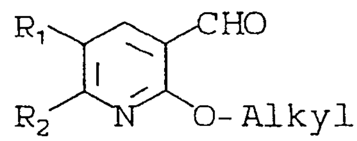
.

**22.** A compound of the formula

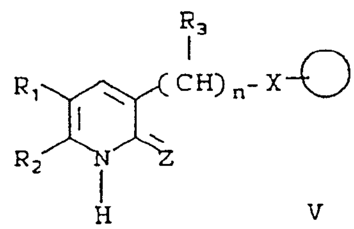

V

wherein
X is NH, O, S;
Z is O or S;
n is 1-4;.
$R_1$ is

(i) $C_{1-8}$ alkyl, unsubstituted or substituted with one or two of $C_{1-3}$alkoxy, halo, $C_{1-4}$alkylamino, amino, $C_{1-4}$-di-(alkyl)amino,
or $C_{1-3}$ alkylthio;
(ii) $C_{1-3}$ alkylthio;
(iii) $C_{1-3}$ alkoxy; or
(iv) halo;

$R_2$ is H, methyl or ethyl, unsubstituted or substituted with one or two of methoxy, amino, methylamino, dimethylamino or methylthio;
$R_3$ is H or $C_{1-8}$ alkyl;

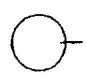

is aryl or heterocycle, each unsubstituted or substituted with one or more of

(a) $C_{1-8}$ alkyl,
(b) $C_{1-8}$ alkoxy unsubstituted or substituted with hydroxy-$C_{1-4}$ alkyl,
(c) amino,
(d) $C_{1-8}$ alkyl amino,
(e) di($C_{1-8}$ alkyl)amino,
(f) amino- $C_{1-8}$ alkyl,

(g) $C_{1-8}$ alkyl-amino-$C_{1-8}$ alkyl,
(h) di-($C_{1-6}$ alkyl)amino $C_{1-8}$ alkyl,
(i) hydroxyl, or
(j) halogen.
with the proviso that heterocycle is not phthalimide.

**23.** The compounds of Claim 22, wherein
$R_1$ is $C_{1-4}$ alkyl;
$R_1$ is H or $CH_3$; aryl is
(a) naphthyl, unsubstituted or substituted with one or more of $C_{1-6}$ alkyl, halogen or hydroxyl;
(b) phenyl, unsubstituted or substituted with one or more of $C_{1-6}$ alkyl, halogen or hydroxyl; or
(c) biphenyl.

**24.** The compounds of Claim 23, wherein
aryl is 2-naphthyl, 2-naphthyl substituted with methyl, 2-naphthyl substituted with chloro; phenyl; 2-hydroxyphenyl, 2-hydroxyphenyl substituted with methyl, or 2-hydroxyphenyl substituted with chloro.

**25.** The compounds of Claim 24, wherein
aryl is 2-naphthyl, phenyl or 2-hydroxyphenyl.

**26.** The compounds of Claim 22 wherein
Heterocycle is thienyl, oxazolyl, thiazolyl, triazolyl, pyridyl, pyrazinyl, benzothienyl, quinolinyl, isoquinolinyl, benzimidazolyl, benzofuranyl, benzothiazolyl, benzoxazolyl unsubstituted or substituted with one or more of $C_{1-6}$ alkyl or halogen or hydroxyl, naphth [3,2-d] oxazolyl, oxazolo [4,5-b] pyridyl, chromone, or benzopyrimidinone.

**27.** The compounds of Claim 26 wherein
Heterocycle is 2-oxazolyl, 2-pyridyl, 2-benzothienyl, 2-quinolinyl, 2-benzimidazolyl, 2-benzofuranyl, 2-benzothiazolyl, 2-benzoxazolyl methyl substituted 2-benzoxazolyl, halo substituted 2-benzoxazolyl, hydroxyl substituted 2-benzoxazolyl, or 2-oxazolo [4,5-b] pyridyl.

**28.** The compounds of Claim 27 wherein
Heterocycle is 2-benzoxazolyl, 7-methyl-2-benzoxazolyl, 4,7-dimethyl-2-benzoxazolyl, 7-chloro-2-benzoxazolyl, or 4,7-dichloro-2-benzoxazolyl.

**29.** The use of a compound as claimed in any of Claims 22 to 28 for the manufacture of a medicament for inhibiting HIV reverse transcriptase.

**30.** The use of a compound as claimed in any of Claims 22 to 28 for the manufacture of a medicament for the treatment of infection by HIV, or the treatment of AIDS or ARC.

**31.** A pharmaceutical composition useful for inhibiting HIV transcriptase, comprising an effective amount of a compound as claimed in any of Claims 22-28, and a pharmaceutically acceptable carrier.

**32.** A pharmaceutical composition useful for preventing or treating infection by HIV or for treating AIDS or ARC, comprising an effective amount of a compound as claimed in any of Claims 22-28, and a pharmaceutically acceptable carrier.

**33.** A compound as claimed in Claim 22 wherein Heterocycle is benzoxazolyl unsubstituted or substituted with one or more of $C_{1-6}$ alkyl or halogen or hydroxyl.

**34.** A compound of Claim 33 wherein benzoxazolyl is 2-benzoxazolyl, unsubstituted or substituted with one or more of methyl, halogen or hydroxyl.

**35.** A compound of Claim 34 wherein benzoxazolyl is 2-benzoxazolyl, 7-methyl-2-benzoxazolyl, 4,7-dimethyl-2-benzoxazolyl, 7-chloro-2-benzoxazolyl, or 4,7-dichloro-2-benzoxazolyl.

**36.** The use of a compound as claimed in any of Claims 22, 33, 34 or 35 for the manufacture of a medicament for inhibiting HIV reverse transcriptase.

**37.** The use of a compound as claimed in any of Claims 22, 33, 34 or 35 for the manufacture of a medicament for the treatment of infection by HIV, or the treatment of AIDS or ARC.

**38.** A pharmaceutical composition useful for inhibiting HIV reverse transcriptase, comprising an effective amount of a compound as claimed in any of Claims 22, 33-35, and a pharmaceutically acceptable carrier.

**39.** A pharmaceutical composition useful for preventing or treating infection of HIV or for treating AIDS or ARC, comprising an effective amount of a compound as claimed in any of Claims 22, 33-35, and a pharmaceutically acceptable carrier.